# EUROPEAN PATENT APPLICATION

(11) **EP 1 302 463 A1**
(43) Date of publication of application: **16.04.2003**
(21) Application number: 01948028.4
(22) Date of filing: 16.07.2001
(51) Int. Cl.: C07D 213/85, C07D 401/04, C07D 401/10, C07D 401/12, C07D 405/04, C07D 405/12, C07D 417/04, C07D 409/04, A61K 31/44, A61K 31/4409, A61K 31/443, A61K 31/4436, A61K 31/4439, A61K 31/4709, A61K 31/196, A61K 31/506, A61K 31/5377

(54) **MEDICINE COMPRISING DICYANOPYRIDINE DERIVATIVE**

(30) Priority: 18.07.2000 JP 2000216982
(71) Applicant: YAMANOUCHI PHARMACEUTICAL CO. LTD., Tokyo 103-8411 (JP)
(72) Inventor: HARADA, Hironori, Yamanouchi Pharmaceutical Co Ltd, Tsukuba-shi, Ibaraki 305-8585 (JP); WATANUKI, Susumu, Yamanouchi Pharmaceutical Co Ltd, Tsukuba-shi, Ibaraki 305-8585 (JP); TAKUWA, Tomofumi, Yamanouchi Pharmaceutical Co Ltd, Tsukuba-shi, Ibaraki 305-8585 (JP); KAWAGUCHI, Kenichi, Tsukuba-shi, Ibaraki 305-8585 (JP); OKAZAKI, Toshio, Yamanouchi Pharmaceutical Co. Ltd, Tsukuba-shi, Ibaraki 305-8585 (JP); HIRANO, Yuusuke, Yamanouchi Pharmaceutical Co. Ltd, Tsukuba-shi, Ibaraki 305-8585 (JP); SAITOH, Chikashi, Yamanouchi Pharmaceutical Co Ltd, Tsukuba-shi, Ibaraki 305-8585 (JP)
(74) Representative: HOFFMANN - EITLE
(86) International application number: JP0106136
(87) International publication number: WO02006237

(57) **Abstract**

Compounds having a high conductance-type of calcium-activated K channel opening effect and a smooth muscle relaxant effect for bladder based on the K-channel opening effect, which can be used in treating pollakiuria and urinary incontinence, are provided. 3,5-Dicyanopyridine derivatives or their salts.

## Description

### Technical Field

The present invention relates to pharmaceutical compositions comprising 3,5-dicyanopyridine derivatives or their pharmaceutically acceptable salts as effective components, a high conductance-type of calcium-activated K channel opening agents, smooth muscle relaxants for bladder and agents for treating pollakiuria and urinary incontinence, as well as novel 3,5-dicyanopyridine derivatives or their pharmaceutically acceptable salts.

### Background Art

It is known that the K channel plays an important role in generation of resting membrane potential or action potential in cells and the opening of the K channel induces hyperpolarizaiton of the cell membrane to suppress excitability of the cells and exhibit the effect of smooth muscle relaxation (J. Urol., 154, 1914-20, 1995).

The high conductance-type of calcium-activated K channel (also referred to as maxi-K channel or BK channel) is one of calcium-activated K channels that open when an increase in Ca level in the cells and depolarization of membrane is detected, and which are widely distributed in the living body to have an important function as an excitable negative feedback system (Am. J. Physiol., 291, C9-C34, 1996). Thus, the drugs of opening the maxi-K channel are expected to have the effects for protecting or improving the function of a variety of organs by exhibiting relaxation in the smooth muscle or suppression of the hyper excitation in the neurocytes.

Particularly, among them, it is known that the smooth muscle of the bladder is highly sensitive to maxi-K channel inhibitors, charybdotoxin and iberiotoxin (J. Pharmacol. Exp. Ther., 259 (1), 439-443, 1991), and accordingly the drugs of opening the maxi-K channel are expected to be highly bladder selective agents for treating pollakiuria or urinary incontinence.

The compounds of the invention exhibit the effect of opening the maxi-K channel to hyper polarize the membrane potential in the cells, and they, acting through their smooth muscle relaxant effect or effect for suppressing nerve excitation, are useful, for example, in prophylaxis and/or treatment of hypertension, asthma, premature birth, irritable bowel syndrome, chronic heart failure, angina pectoris, myocardial infarction, cerebral infarction, subarachnoid hemorrhage, cerebrovascular spasm, cerebral hypoxia, peripheral vascular diseases, anxiety, male baldness, erectile insufficiency, diabetes mellitus, diabetic peripheral neuropathy, other diabetic complication, infertility, urinary calculus and its accompanying pain (relief), particularly in treatment of instability of urinary bladder, e.g., pollakiuria, urinary incontinence, nocturnal enuresis.

It has been reported concerning the maxi-K channel opening drug that the pyrrole derivative NS-8 of the following structure exhibits a relaxant effect for the murine removed bladder smooth muscle, and charybdotoxin exhibits an inhibitory effect to the relaxant action and further makes rhythmic vesical contraction subsided in an anesthetized rat to increase the bladder volume without having any influence on the maximum contraction pressure of the bladder (Nippon Hinyouki-ka Gakkai Zasshi (J. Jap. Urological Association), 89 (2), 138, 1998).

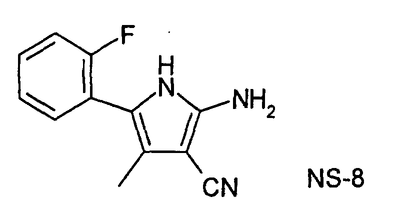

In JP8-67670, the 4-phenyl-6-aminonicotinic acid derivatives as shown below have been disclosed as maxi K channel regulators, which are useful in treatment of brain diseases. 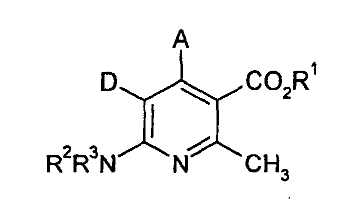 (wherein D represents a nitro or cyano. Other symbols are defined in the specification of JP8-67670)

Other derivatives disclosed as the maxi-K channel opening agents include benzimidazole derivatives in EP477819 and EP617023, pyridine derivatives in WO94/22807 and WO96/06610, thiopyranopyridine deivatives in WO96/2547, cyclohexadiene derivatives in EP698597, pyran derivatives in EP758649, nitrogen-containing 5-membered ring derivatives in WO98/04135, indole derivatives in WO98/16222, quinoline derivatives in WO98/23273 and WO99/09983, and anthranilic acid derivatives in WO99/07669 and WO99/07670. However, there is no report on 3,5-dicyanopyridine derivatives.

On the other hand, as for the 3,5-dicyanopyridine derivatives, 2-amino-3,5-dicyano-4-aryl-6-sulfanylpyridine derivatives have been disclosed in WO01/25210 as ligands for adenosine receptors, which are described as useful in prophylaxis and/or treatment of cardiovascular diseases, urogenital diseases, respiratory diseases, inflammation and inflammation in nervous system, diabetes mellitus, particularly diabetes mellitus in pancreas, neural degenerative diseases, pain, hepatic fibrosis, and liver cirrhosis.

In Japanese Patent Publication No. 48-24726/1973, the 3,5-dicyanopyridine derivatives of the following structure have been described, which can be used as antifungals, insecticides, herbicides, miticides, nematocides, and antimicrobials, particularly as bactericides.

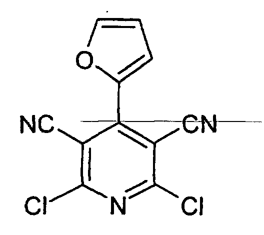

In addition, a process for synthesizing 3,5-dicyanopyridine derivatives or the use of the 3,5-dicyanopyridine derivatives as intermediates in synthesis have been described in J. Chin. Chem. Soc. (Taipei)(2000), 47(2), 347-350; Eur. J. Med. Chem. (1998), 33(11), 887-897; Reel. Trav. Chim. Pays-Bas (1994), 113(1), 35-9; Eur. J. Med. Chem. Chim. Ther. (1984), 19(6), 555-7.

In addition, the 3,5-dicyanopyridine derivatives have also been reported in Phosphorus, Sulfur Silicon Relat. Elem. (2000), 163, 29-40; Chem. Commun. (Cambridge)(2000), (18), 1775-1776; Mendeleev Commun. (2000), (3), 114-115; Russ. Chem. Bull. (2000), 49(2), 348-354; Proc. Natl. Acad. Sci. U.S.A. (2000), 97(11), 6073-6078; Russ. J. Org. Chem. (1999), 35(9), 1377-1384; Chin. Pharm. J. (Taipei)(1999), 51(5), 313-318; Mendeleev Commun. (2000), (1), 7-9; J. Am. Chem. Soc. (2000), 122(8), 1572-1579; J. Am. Chem. Soc. (2000), 122(8), 1580-1588; Z. Naturforsch., B: Chem. Sci. (1999), 54(9), 1205-1209., Mendeleev Commun. (1999), (4), 166-167., Heterocycl. Commun. (1999), 5(2), 179-182., J. Heterocycl. Chem. (1999), 36(2), 481-483., J. Serb. Chem. Soc. (1999), 64(1), 9-18., AIP Conf. Proc. (1998), 450(SCIFI 97: Conference on Scintillating Fiber Detectors), 14-24., J. Prakt. Chem./Chem.-Ztg. (1998), 340(7), 676-678., Chem. Heterocycl. Compd. (N. Y.) (1998), 34(2), 188-194., Chem. Heterocycl. Compd. (N. Y.) (1998), 34(1), 96-101., Chem. Heterocycl. Compd. (N. Y.) (1998), Volume Date 1997, 33(12), 1430-1437., Rev. Roum. Chim. (1998), 43(2), 163-170., Chem. Heterocycl. Compd. (N. Y.) (1998), Volume Date 1997, 33(11),., Russ. J. Org. Chem. (1997), 33(7), 1014-1017., Chem. Heterocycl. Compd. (N. Y.) (1998), Volume Date 1997, 33(7), 793-798., Chem. Heterocycl. Compd. (N. Y.) (1997), 33(5), 587-595., Russ. Chem. Bull. (1997), 46(11), 1909-1911., Chem. Heterocycl. Compd. (N. Y.) (1998), Volume Date 1997, 33(7), 871-872., J. Chem. Soc., Perkin Trans. 1 (1997), (21), 3285-3290., J. Chem. Res., Synop. (1997), (9), 312-313., Bioorg. Med. Chem. (1997), 5(8), 1543-1553., Heterocycl. Commun. (1997), 3(4), 371-380., Tetrahedron (1997), 53(23), 7911-7916., Monatsh. Chem. (1997), 128(1), 29-35., Ukr. Khim. Zh. (Russ. Ed.) (1996), 62(11-12), 61-66., Dokl. Akad. Nauk (1997), 352(5), 636-640., Heteroat. Chem. (1997), 8(1), 1-6., Khim. Geterotsikl. Soedin. (1996), (8), 1099-1103., Khim. Geterotsikl. Soedin. (1996), (8), 1094-1098., Tetrahedron (1996), 52(31), 10497-10506., Khim. Geterotsikl. Soedin. (1996), (1), 67-73., Izv. Akad. Nauk, Ser. Khim. (1996), (4), 938-942., Tetrahedron (1996), 52(3), 1011-26., Bull. Soc. Chim. Fr. (1995), 132(9), 920-4., Natl. Acad. Sci. Lett. (India) (1995), Volume Date 1995, 18(1&2), 15-16., Monatsh. Chem. (1995), 126(6/7), 663-71., Tetrahedron (1995), 51(2), 635-48., Zh. Org. Khim. (1994), 30(4), 581-7., Aswan Sci. Technol. Bull. (1994), 15 108-19., Tetrahedron (1994), 50(22), 6705-14., Heterocycles (1994), 38(6), 1299-305., J. Heterocycl. Chem. (1994), 31(1), 49-52., Arch. Pharm. (Weinheim, Ger.) (1993), 326(12), 959-61., Synth. Commun. (1993), 23(18), 2605-9., Corros. Sci. (1993), 34(5), 779-84., J. Heterocycl. Chem. (1992), 29(7), 1693-702., Gazz. Chim. Ital. (1992), 122(8), 299-303., Z. Naturforsch., B: Chem. Sci. (1992), 47(10), 1438-40., EP476607, Z. Naturforsch., B: Chem. Sci. (1992), 47(4), 572-8., Bull. Fac. Sci., Assiut Univ. (1991), 20(2), 43-53., Bull. Fac. Sci., Assiut Univ. (1991), 20(2), 135-40., Monatsh. Chem. (1991), 122(12), 1035-45., Izv. Akad. Nauk SSSR, Ser. Khim. (1991), (7), 1643-6., J. Chem. Res., Synop. (1991), (7), 178-9., Phosphorus, Sulfur Silicon Relat. Elem. (1991), 55(1-4), 175-83., J. Chem. Res., Synop. (1991), (5), 116-17., Zh. Obshch. Khim. (1990), 60(12), 2750-5., Arch. Pharmacal Res. (1990), 13(3), 274-7., DE3905238, Orient. J. Chem. (1989), 5(4), 273-80., J. Chem. Res., Synop. (1990), (6), 186-7., DD275688, Phosphorus, Sulfur Silicon Relat. Elem. (1990), 48(1-4), 281-4., Indian J. Chem., Sect. B (1990), 29B(4), 322-5., J. Chem. Res., Synop. (1990), (5), 136-7., Ukr. Khim. Zh. (Russ. Ed.) (1990), 56(1), 65-9., Zh. Org. Khim. (1989), 25(6), 1323-30., Zh. Org. Khim. (1989), 25(3), 622-8., J. Prakt. Chem. (1988), 330(5), 817-13., Zh. Org. Khim. (1988), 24(4), 854-61., Acta Pol. Pharm. (1987), 44(1), 32-41., J. Chem. Eng. Data (1988), 33(2), 218-19., Gazz. Chim. Ital. (1987), 117(7), 385-9., Zh. Obshch. Khim. (1987), 57(4), 959-61., Z. Naturforsch., B: Chem. Sci. (1987), 42(1), 107-11., Z. Naturforsch., B: Anorg. Chem., Org. Chem. (1986), 41B(6), 781-3., Izv. Akad. Nauk SSSR, Ser. Khim. (1986), (1), 153-9., Heterocycles (1985), 23(12), 3107-10., Liebigs Ann. Chem. (1986), (9), 1639-44., Heterocycles (1985), 23(8), 2013-17., Drug Chem. Toxicol. (1977) (1985), 8(3), 171-82., Chem. Ber. (1985), 118(6), 2198-207., An. Quim., Ser. C (1984), 80(3), 268-72., Heterocycles (1985), 23(1), 93-8., Synthesis (1984), (8), 679-81., DD210262, An. Quim., Ser. C (1983), 79(3), 368-72., Zh. Obshch. Khim. (1983), 53(5), 1187-8., J. Heterocycl. Chem. (1982), 19(5), 1021-4., Zh. Org. Khim. (1982), 18(11), 2421-5., DD150894, Synthesis (1982), (4), 320-2., Deposited Doc. (1981), VINITI 1509-81, 12 pp. Avail.: VINITI., Monatsh. Chem. (1981), 112(8-9), 973-85., Monatsh. Chem. (1981), 112(11), 1271-7., Synthesis (1981), (7), 529-30., Synthesis (1981), (7), 531-3., Bull. Chem. Soc. Jpn. (1981), 54(3), 787-9., An. Quim., Ser. C (1980), 76(1), 68-9., Indian J. Chem., Sect. B (1980), 19B(2), 151-2., Pol. J. Chem. (1979), 53(10), 2121-7., Arch. Pharm. (Weinheim, Ger.) (1979), 312(6), 478-86., An. Quim. (1978), 74(4), 648-50., Synthesis (1978), (9), 681-2., J. Chem. Soc., Perkin Trans. 1 (1978), (6), 549-53., An. Quim. (1977), 73(11), 1359-62., Afinidad (1978), 35(354), 138-40., Proc. R. Ir. Acad., Sect. B (1977), 77B(19-47), 533-8; Yakugaku Zasshi (1977), 97(9), 1022-33; J. Org. Chem. (1977), 42(21), 3410-13; An. Quim. (1976), 72(11-12), 926-30; PL72750; An. Quim. (1974), 70(12), 951-8; Acta Pol. Pharm. (1974), 31(3), 271-8; J. Org. Chem. (1974), 39(25), 3735-8; J. Org. Chem. (1974), 39(12), 1685-8; Indian J. Chem. (1973), 11(12), 1315-16; Aust. J. Chem. (1973), 26(11), 2567-9; DE2206506; Act Pol. Pharm. (1972), 29(6), 545-53; J. Chem. Soc., Perkin Trans. 1 (1972), (23), 2946-50; Chem. Parm Bull. (1972), 20(7), 1544-50; Ind. Chim. Belge (1968), 33(5), 449-54; J. Chem. Soc. C (1968), (10), 1252-8; J. Chem. Soc. C (1968), (8), 960-6; An. R. Soc. Esp. Fis. Quim., Ser. B (1967), 63(6), 691-702; Rev. R. Acad. Cienc. Exactas, Fis. Nat. Madrid (1966), 60(1), 35-144.

However, there is no report on the relation to a "high conductance-type of calcium-activated K channel opening agents", "smooth muscle relaxants for bladder" and "agents for treating pollakiuria and urinary incontinence" at all.

Though the compounds as descried in the above-mentioned patent specifications are known as the maxi-K channel opening agents, it is a therapeutically important problem to create a much better maxi-K channel opening agent as well as a therapeutic agent for treating pollakiuria and urinal incontinence based on the above-mentioned effect.

### Disclosure of Invention

The present inventors worked assiduously to study maxi-K channel opening compounds and found that 3,5-dicyanopyridine derivatives exhibit an excellent effect to open the maxi-K channel. The invention was completed based on this finding.

According to the invention, there are provided a high conductance-type of calcium-activated K channel (maxi-K channel) opening agents, smooth muscle relaxants for bladder and agents for treating pollakiuria and urinary incontinence, comprising any one of 3,5-dicyanopyridine derivatives of the general formula (I) or pharmaceutically acceptable salts thereof as effective components. 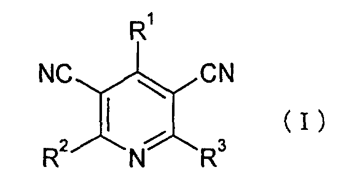 Wherein
R¹ represents H, an optionally substituted lower alkyl, optionally substituted cycloalkyl, optionally substituted aryl, optionally substituted heteroaryl, or optionally substituted 5- or 6-membered saturated heterocycle;
R² and R³ are the same or different, each representing -O-R⁴, -S(O)ₙ-R⁴, -N(-R⁴)-R⁵, -NHCO-R⁵, -NHS(O)ₙ-R⁵, -NHCON(-R⁴)-R⁵, -N(CO-R⁵)₂, halogen atom or optionally substituted heteroaryl;
R⁴ represents H, an optionally substituted lower alkyl, optionally substituted lower alkenyl, optionally substituted alkynyl, optionally substituted aryl, optionally substituted heteroaryl, or optionally substituted 5- or 6-membered saturated heterocycle;
R⁵ represents H, an optionally substituted lower alkyl, cycloalkyl, -lower alkyl-O-lower alkyl, -lower alkyl-O-aryl, -lower alkyl-aryl, optionally substituted aryl, or optionally substituted heteroaryl;
or alternatively R⁴ and R⁵ taken with the adjacent N atom may form a 5- or 6-membered saturated heterocycle or a heteroaryl;
n represents 0, 1 or 2.

The 3,5-dicyanopyridine derivatives are characterized in the structure that they are substituted by cyano groups at the 3 and 5 positions of the pyridine ring and in the pharmacological properties that they exhibit an opening effect for the maxi-K channel.

In addition, according to the invention, there are provided 3,5-dicyanopyridine derivatives of the general formula (II) or pharmaceutically acceptable salts thereof. 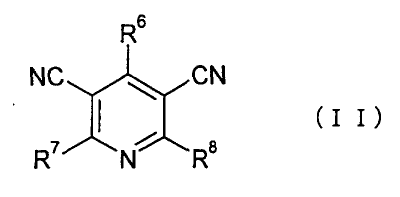 Wherein
R⁶ represents phenyl, 2-fluorophenyl, 2,5-difluorophenyl, 2,6-difluorophenyl, 4-aminophenyl, 2,3-dihydro-1H-indol-6-yl, quinolin-7-yl, 3,4,5,6-tetrahydro-2H-pyran-2-yl, cyclohexylmethyl, benzyl, thiophen-2-yl or thiophen-3-yl;
R⁷ and R⁸ are the same or different, each representing -O-R⁹, -S(O)ₘ-R⁹, -N(-R⁹)-R¹⁰, -NHCO-R¹⁰, -NHS(O)ₘ-R¹⁰, -NHCON(-R⁹)-R¹⁰, -N(CO-R¹⁰)₂, halogen atom or optionally substituted heteroaryl;
R⁹ represents H, an optionally substituted lower alkyl, optionally substituted lower alkenyl, optionally substituted alkynyl, optionally substituted aryl, optionally substituted heteroaryl, or optionally substituted 5- or 6-membered saturated heterocycle;
R¹⁰ represents H, an optionally substituted lower alkyl, cycloalkyl, -lower alkyl-O-lower alkyl, -lower alkyl-O-aryl, -lower alkyl-aryl, optionally substituted aryl, or optionally substituted heteroaryl;
or alternatively R⁹ and R¹⁰ taken with the adjacent N atom may form a 5- or 6-membered saturated heterocycle or a heteroaryl;
m represents 0, 1 or 2;
provided that
when R⁶ is phenyl, then
R⁷ is methoxy, 2-(2-amino-3-phenylpropionyloxy)ethoxy, 2-hydroxyethoxy, 2-aminomethylphenoxy or pyridin-3-ylmethyloxy; when R⁵ is phenyl and R⁷ is methoxy, then R⁸ is 2-hydroxyethylamino or methoxycarbonylmethylamino;
when R⁶ is phenyl, 2-fluorophenyl, 2,5-difluorophenyl, 2,6-difluorophenyl or 4-aminophenyl, R⁷ is -S-R⁹, and R⁹ is not N-oxidopyridinylmethyl, then
R⁸ excludes NH₂;
when R⁶ is benzyl, then
2-amino-4-benzyl-6-ethoxypyridine-3,5-dicarbonitrile is excluded;
when R⁶ is thiophen-2-yl, then
R⁷ is methoxy or 2-hydroxyethylsulfanyl;
when R⁶ is thiophen-3-yl, then
2-amino-6-sulfanyl-4-(thiophen-2-yl)pyridine-3,5-dicarbonitrile is excluded.

Among the compounds represented by the general formula (II) or their pharmaceutically acceptable salts, the followings are preferred:
2-amino-4-(2-fluorophenyl)-6-methoxypyridine-3,5-dicarbonitrile;
2-amino-6-methoxy-4-(tetrahydro-2H-pyran-2-yl)pyridine-3,5-dicarbonitrile;
2-[(6-amino-3,5-dicyano-4-phenylpyridin-2-yl)oxy]ethyl (S)-2-amino-3-phenyl propanoate;
2-amino-6-(2,2-difluoroethoxy)-4-(2-fluorophenyl)pyridine-3,5-dicarbonitrile;
2-amino-4-(2-fluorophenyl)-6-(prop-2-yn-1-yloxy)pyridine-3,5-dicarbonitrile;
N-[3,5-dicyano-4-(2-fluorophenyl)-6-methoxypyridin-2-yl]acetamide;
2-amino-4-(2,3-dihydro-1H-indol-6-yl)-6-methoxypyridine-3,5-dicarbonitrile;
N-[3,5-dicyano-4-(2-fluorophenyl)-6-methoxypyridin-2-yl]-2-methoxyacetamide;
N-[3,5-dicyano-4-(2,6-difluorophenyl)-6-methoxypyridin-2-yl]-2-methoxyacetamide;
N-[3,5-dicyano-4-(2,6-difluorophenyl)-6-methoxypyridin-2-yl]acetamide;
N-[3,5-dicyano-6-methoxy-4-(tetrahydropyran-2-yl)pyridin-2-yl]-2-methoxyacetamide;
N-[3,5-dicyano-6-(2,2-difluoroethoxy)-4-(2-fluorophenyl)pyridin-2-yl]-2-methoxyacetamide;
2-amino-6-methoxy-4-thiophen-2-ylpyridine-3,5-dicarbonitrile;
2-amino-6-methylsulfanyl-4-thiophen-3-ylpyridine-3,5-dicarbonitrile;
2-amino-6-(2-hydroxyethoxy)-4-phenylpyridine-3,5-dicarbonitrile;
2-amino-6-[(2-hydroxyethyl)sulfanyl]-4-thiophen-2-ylpyridine-3,5-dicarbonitrile;
2-amino-4-(4-aminophenyl)-6-methoxypyridine-3,5-dicarbonitrile;
N-(3,5-dicyano-6-methoxy-4-thiophen-2-ylpyridin-2-yl)acetamide;
2-amino-4-(2,5-difluorophenyl)-6-methoxypyridine-3,5-dicarbonitrile;
2-[(2-hydroxyethyl)amino]-6-methoxy-4-phenylpyridine-3,5-dicarbonitrile;
methyl [(3,5-dicyano-6-methoxy-4-phenylpyridin-2-yl)amino]acetate;
2-amino-4-(2,6-difluorophenyl)-6-methoxypyridine-3,5-dicarbonitrile;
2-amino-4-(2-fluorophenyl)-6-(2-hydroxyethoxy)pyridine-3,5-dicarbonitrile;
2-amino-4-(2-fluorophenyl)-6-isopropoxypyridine-3,5-dicarbonitrile;
2-amino-4-benzyl-6-methoxypyridine-3,5-dicarbonitrile;
2-amino-4-cyclohextlmethyl-6-methoxypyridine-3,5-dicarbonitrile;
2-amino-6-(3-fluorophenoxy)-4-(2-fluorophenyl)pyridine-3,5-dicarbonitrile;
2-amino-6-(2-aminomethylphenoxy)-4-phenylpyridine-3,5-dicarbonitrile;
2-allyloxy-6-amino-4-(2-fluorophenyl)pyridine-3,5-dicarbonitrile;
2-amino-4-(2-fluorophenyl)-6-(pyridin-3-ylmethoxy)pyridine-3,5-dicarbonitrile ;
2-amino-4-benzyl-6-[(pyridin-3-ylmethyl)sulfanyl]pyridine-3,5-dicarbonitrile;
2-amino-4-benzyl-6-(pyridin-3-ylmethoxy)pyridine-3,5-dicarbonitrile;
2-amino-4-(2,6-difluorophenyl)-6-(pyridin-3-ylmethoxy)pyridine-3,5-dicarbonitrile;
2-amino-4-phenyl-6-(pyridin-3-ylmethoxy)pyridine-3,5-dicarbonitrile;
2-amino-4-(2-fluorophenyl)-6-{[(1-oxidopyridin-3-yl)methyl]sulfanyl}pyridine-3,5-dicarbonitrile;
2-amino-4-(2-fluorophenyl)-6-(pyridin-2-ylmethoxy)pyridine-3,5-dicarbonitrile ;
2-amino-4-(2-fluorophenyl)-6-(pyridin-4-ylmethoxy)pyridine-3,5-dicarbonitrile ;
2-amino-6-benzylsulfanyl-4-(tetrahydro-2H-pyran-2-yl)pyridine-3,5-dicarbonitrile;
2-amino-4-(2-fluorophenyl)-6-[(1-oxidopyridin-3-yl)methoxy]pyridine-3,5-dicarbonitrile;
2-amino-6-(but-3-en-1-yloxy)-4-(2-fluorophenyl)pyridine-3,5-dicarbonitrile;
2-diacetylamino-4-(2-fluorophenyl)-6-methoxypyridine-3,5-dicarbonitrile;
N-[3,5-dicyano-4-(2-fluorophenyl)-6-methoxypyridin-2-yl]propionamide;
N-[3,5-dicyano-4-(2-fluorophenyl)-6-methoxypyridin-2-yl]-2,2,2-trifluoroacetamide;
N-[3,5-dicyano-4-(2-fluorophenyl)-6-methoxypyridin-2-yl]isobutyramide;
N-[3,5-dicyano-4-(2-fluorophenyl)-6-methoxypyridin-2-yl]-3-phenylpropionamide;
N-[3,5-dicyano-4-(2-fluorophenyl)-6-methoxypyridin-2-yl]-2-phenoxyacetamide;
N-[3,5-dicyano-4-(2-fluorophenyl)-6-methoxypyridin-2-yl]-2-phenylacetamide;
1-[3,5-dicyano-4-(2-fluorophenyl)-6-methoxypyridin-2-yl]-3-(2-hydroxyethyl)urea;
N-[3,5-dicyano-4-(2-fluorophenyl)-6-methoxypyridin-2-yl]-2,2-dimethylpropionamide;
N-[3,5-dicyano-4-(2-fluorophenyl)-6-methoxypyridin-2-yl]hexanamide;
N-[3,5-dicyano-4-(2-fluorophenyl)-6-methoxypyridin-2-yl]thiophene-2-carboxamide;
methyl N-[3,5-dicyano-4-(2-fluorophenyl)-6-methoxy-pyridin-2-yl]oxamate;
N-[3,5-dicyano-4-(2-fluorophenyl)-6-methoxypyridin-2-yl]pyridine-2-carboxamide;
2-amino-6-methoxy-4-quinolin-7-ylpyridine-3,5-dicarbonitrile;
N-[3,5-dicyano-4-(2-fluorophenyl)-6-methoxypyridin-2-yl]naphthalene-2-carboxamide;
N-[3,5-dicyano-4-(2-fluorophenyl)-6-methoxypyridin-2-yl]furan-2-carboxamide ;
[3,5-dicyano-4-(2-fluorophenyl)-6-methoxypyridin-2-ylcarbamoyl]methylacetate;
2-benzyloxy-N-[3,5-dicyano-4-(2-fluorophenyl)-6-methoxypyridin-2-yl]acetamide;
N-[3,5-dicyano-4-(2-fluorophenyl)-6-methoxypyridin-2-yl]-3-methoxypropionamide;
N-[3,5-dicyano-4-(2-fluorophenyl)-6-methoxypyridin-2-yl]-2-dimethylaminoacetamide;
N-[3,5-dicyano-4-(2-fluorophenyl)-6-methoxypyridin-2-yl]-3-pyridin-3-ylpropionamide; or
(R)-N-[3,5-dicyano-4-(2-fluorophenyl)-6-methoxypyridin-2-yl]-2-phenylpropionamide; or
pharmaceutically acceptable salts thereof. More preferred are:
2-amino-4-(2-fluorophenyl)-6-methoxypyridine-3,5-dicarbonitrile;
2-amino-6-methoxy-4-(tetrahydro-2H-pyran-2-yl)pyridine-3,5-dicarbonitrile;
2-[(6-amino-3,5-dicyano-4-phenylpyridin-2-yl)oxy]ethyl (S)-2-amino-3-phenylpropanoate;
2-amino-6-(2,2-difluoroethoxy)-4-(2-fluorophenyl)pyridine-3,5-dicarbonitrile;
2-amino-4-(2-fluorophenyl)-6-(prop-2-yn-1-yloxy)pyridine-3,5-dicarbonitrile;
N-[3,5-dicyano-4-(2-fluorophenyl)-6-methoxypyridin-2-yl]acetamide;
2-amino-4-(2,3-dihydro-1H-indol-6-yl)-6-methoxypyridine-3,5-dicarbonitrile;
N-[3,5-dicyano-4-(2-fluorophenyl)-6-methoxypyridin-2-yl]-2-methoxyacetamide;
N-[3,5-dicyano-4-(2,6-diffuorophenyl)-6-methoxypyridin-2-yl]-2-methoxyacetamide;
N-[3,5-dicyano-4-(2,6-difluorophenyl)-6-methoxypyridin-2-yl]acetamide;
N-[3,5-dicyano-6-methoxy-4-(tetrahydropyran-2-yl)pyridin-2-yl]-2-methoxyacetamide; or
N-[3,5-dicyano-6-(2,2-difluoroethoxy)-4-(2-fluorophenyl)pyridin-2-yl]-2-methoxyacetamide; or
pharmaceutically acceptable salts thereof.

According to the invention, there are provided pharmaceutical compositions, a high conductance-type of calcium-activated K channel opening agents, smooth muscle relaxants for bladder and agents for treating pollakiuria and urinary incontinence, comprising any one of the following compounds represented by the general formula (II) or their pharmaceutically acceptable salts as effective components. Preferred are:
2-amino-4-(2-fluorophenyl)-6-methoxypyridine-3,5-dicarbonitrile;
2-amino-6-methoxy-4-(tetrahydro-2H-pyran-2-yl)pyridine-3,5-dicarbonitrile;
2-[(6-amino-3,5-dicyano-4-phenylpyridin-2-yl)oxy]ethyl (S)-2-amino-3-phenylpropanoate;
2-amino-6-(2,2-difluoroethoxy)-4-(2-fluorophenyl)pyridine-3,5-dicarbonitrile;
2-amino-4-(2-fluorophenyl)-6-(prop-2-yn-1-yloxy)pyridine-3,5-dicarbonitrile;
N-[3,5-dicyano-4-(2-fluorophenyl)-6-methoxypyridin-2-yl]acetamide;
2-amino-4-(2,3-dihydro-1H-indol-6-yl)-6-methoxypyridine-3,5-dicarbonitrile;
N-[3,5-dicyano-4-(2-fluorophenyl)-6-methoxypyridin-2-yl]-2-methoxyacetamide;
N-[3,5-dicyano-4-(2,6-difluorophenyl)-6-methoxypyridin-2-yl]-2-methoxyacetamide;
N-[3,5-dicyano-4-(2,6-difluorophenyl)-6-methoxypyridin-2-yl]acetamide;
N-[3,5-dicyano-6-methoxy-4-(tetrahydropyran-2-yl)pyridin-2-yl]-2-methoxyacetamide;
N-[3,5-dicyano-6-(2,2-difluoroethoxy)-4-(2-fluorophenyl)pyridin-2-yl]-2-methoxyacetamide;
2-amino-6-methoxy-4-thiophen-2-ylpyridine-3,5-dicarbonitrile;
2-amino-6-methylsulfanyl-4-thiophen-3-ylpyridine-3,5-dicarbonitrile;
2-amino-6-(2-hydroxyethoxy)-4-phenylpyridine-3,5-dicarbonitrile;
2-amino-6-[(2-hydroxyethyl)sulfanyl]-4-thiophen-2-ylpyridine-3,5-dicarbonitrile;
2-amino-4-(4-aminophenyl)-6-methoxypyridine-3,5-dicarbonitrile;
N-(3,5-dicyano-6-methoxy-4-thiophen-2-ylpyridin-2-yl)acetamdie;
2-amino-4-(2,5-difluorophenyl)-6-methoxypyridine-3,5-dicarbonitrile;
2-[(2-hydroxyethyl)amino]-6-methoxy-4-phenylpyridine-3,5-dicarbonitrile;
methyl [(3,5-dicyano-6-methoxy-4-phenylpyridin-2-yl)amino]acetate;
2-amino-4-(2,6-difluorophenyl)-6-methoxypyridine-3,5-dicarbonitrile;
2-amino-4-(2-fluorophenyl)-6-(2-hydroxyethoxy)pyridine-3,5-dicarbonitrile;
2-amino-4-(2-fluorophenyl)-6-isopropoxypyridine-3,5-dicarbonitrile;
2-amino-4-benzyl-6-methoxypyridine-3,5-dicarbonitrile;
2-amino-4-cyclohexylmethyl-6-methoxypyridine-3,5-dicarbonitrile;
2-amino-6-(3-fluorophenoxy)-4-(2-fluorophenyl)pyridine-3,5-dicarbonitrile;
2-amino-6-(2-aminomethylphenoxy)-4-phenylpyridine-3,5-dicarbonitrile;
2-allyloxy-6-amino-4-(2-fluorophenyl)pyridine-3,5-dicarbonitrile;
2-amino-4-(2-fluorophenyl)-6-(pyridin-3-ylmethoxy)pyridine-3,5-dicarbonitrile ;
2-amino-4-benzyl-6-[(piridin-3-ylmethyl)sulfanyl]pyridine-3,5-dicarbonitrile;
2-amino-4-benzyl-6-(pyridin-3-ylmethoxy)pyridine-3,5-dicarbonitrile;
2-amino-4-(2,6-difluorophenyl)-6-(pyridin-3-ylmethoxy)pyridine-3,5-dicarbonitrile;
2-amino-4-phenyl-6-(pyridin-3-ylmethoxy)pyridine-3,5-dicarbonitrile;
2-amino-4-(2-fluorophenyl)-6-{[(1-oxidopyridin-3-yl)methyl]sulfanyl)pyridine-3,5-dicarbonitrile;
2-amino-4-(2-fluorophenyl)-6-(pyridin-2-ylmethoxy)pyridine-3,5-dicarbonitrile ;
2-amino-4-(2-fluorophenyl)-6-(pyridin-4-ylmethoxy)pyridine-3,5-dicarbonitrile ;
2-amino-6-benzylsulfanyl-4-(tetrahydro-2H-pyran-2-yl)pyridine-3,5-dicarbonitrile;
2-amino-4-(2-fluorophenyl)-6-[(1-oxidopyridin-3-yl)methoxy]pyridine-3,5-dicarbonitrile;
2-amino-6-(but-3-en-1-yloxy)-4-(2-fluorophenyl)pyridine-3,5-dicarbonitrile;
2-diacetylamino-4-(2-fluorophenyl)-6-methoxypyridine-3,5-dicarbonitrile;
N-[3,5-dicyano-4-(2-fluorophenyl)-6-methoxypyridin-2-yl]propionamide;
N-[3,5-dicyano-4-(2-fluorophenyl)-6-methoxypyridin-2-yl]-2,2,2-trifluoroacetamide;
N-[3,5-dicyano-4-(2-fluorophenyl)-6-methoxypyridin-2-yl]isobutyramide;
N-[3,5-dicyano-4-(2-fluorophenyl)-6-methoxypyridin-2-yl]-3-phenylpropionamide;
N-[3,5-dicyano-4-(2-fluorophenyl)-6-methoxypyridin-2-yl]-2-phenoxyacetamide;
N-[3,5-dicyano-4-(2-fluorophenyl)-6-methoxypyridin-2-yl]-2-phenylacetamide;
1-[3,5-dicyano-4-(2-fluorophenyl)-6-methoxypyridin-2-yl]-3-(2-hydroxyethyl)urea;
N-[3,5-dicyano-4-(2-fluorophenyl)-6-methoxypyridin-2-yl]-2,2-dimethylpropionamide;
N-[3,5-dicyano-4-(2-fluorophenyl)-6-methoxypyridin-2-yl]hexanamide;
N-[3,5-dicyano-4-(2-fluorophenyl)-6-methoxypyridin-2-yl]thiophene-2-carboxamide;
methyl N-[3,5-dicyano-4-(2-fluorophenyl)-6-methoxy-pyridin-2-yl]oxamate;
N-[3,5-dicyano-4-(2-fluorophenyl)-6-methoxypyridin-2-yl]pyridine-2-carboxamide;
2-amino-6-methoxy-4-quinolin-7-ylpyridine-3,5-dicarbonitrile;
N-[3,5-dicyano-4-(2-fluorophenyl)-6-methoxypyridin-2-yl]naphthalene-2-carboxamide;
N-[3,5-dicyano-4-(2-fluorophenyl)-6-methoxypyridin-2-yl]furan-2-carboxamide ;
[3,5-dicyano-4-(2-fluorophenyl)-6-methoxypyridin-2-ylcarbamoyl]methyl acetate;
2-benzyloxy-N-[3,5-dicyano-4-(2-fluorophenyl)-6-methoxypyridin-2-yl]acetamide;
N-[3,5-dicyano-4-(2-fluorophenyl)-6-methoxypyridin-2-yl]-3-methoxypropionamide;
N-[3,5-dicyano-4-(2-fluorophenyl)-6-methoxypyridin-2-yl]-2-dimethylaminoacetamide;
N-[3,5-dicyano-4-(2-fluorophenyl)-6-methoxypyridin-2-yl]-3-pyridin-3-ylpropionamide; or
(R)-N-[3,5-dicyano-4-(2-fluorophenyl)-6-methoxypyridin-2-yl]-2-phenylpropionamide; or
pharmaceutically acceptable salts thereof. More preferred are:
2-amino-4-(2-fluorophenyl)-6-methoxypyridine-3,5-dicarbonitrile;
2-amino-6-methoxy-4-(tetrahydro-2H-pyran-2-yl)pyridine-3,5-dicarbonitrile;
2-[(6-amino-3,5-dicyano-4-phenylpyridin-2-yl)oxy]ethyl (S)-2-amino-3-phenylpropanoate;
2-amino-6-(2,2-difluoroethoxy)-4-(2-fluorophenyl)pyridine-3,5-dicarbonitrile;
2-amino-4-(2-fluorophenyl)-6-(prop-2-yn-1-yloxy)pyridine-3,5-dicarbonitrile;
N-[3,5-dicyano-4-(2-fluorophenyl)-6-methoxypyridin-2-yl]acetamide;
2-amino-4-(2,3-dihydro-1H-indol-6-yl)-6-methoxypyridine-3,5-dicarbonitrile;
N-[3,5-dicyano-4-(2-fluorophenyl)-6-methoxypyridin-2-yl]-2-methoxyacetamide;
N-[3,5-dicyano-4-(2,6-difluorophenyl)-6-methoxypyridin-2-yl]-2-methoxyacetamide;
N-[3,5-dicyano-4-(2,6-difluorophenyl)-6-methoxypyridin-2-yl]acetamide;
N-[3,5-dicyano-6-methoxy-4-(tetrahydropyran-2-yl)pyridin-2-yl]-2-methoxyacetamide; or
N-[3,5-dicyano-6-(2,2-difluoroethoxy)-4-(2-fluorophenyl)pyridin-2-yl]-2-methoxyacetamide; or
pharmaceutically acceptable salts thereof.

The compounds represented by the general formula (I) or (II) are further described as follows.

In the definition of the groups of the general formulae in the present specification, the term "lower" means, unless otherwise indicated, a straight or branched carbon chain of 1 to 6 carbon atoms.

Accordingly, the term "lower alkyl" means a C₁₋₆ alkyl, specifically including methyl, ethyl, propyl, butyl, pentyl, hexyl or isopropyl and a structural isomer thereof, preferably C₁₋₄ alkyl, more preferably methyl or ethyl.

The term "lower alkenyl" means a C₂₋₆ alkenyl, specifically including ethenyl, 1-propenyl, 1-butenyl, 1-pentenyl, 1-hexenyl or 2-propenyl, 1-methyl-2-propenyl, and a structural isomer thereof, preferably 2-propenyl.

The term "lower alkynyl" means a C₂₋₆ alkynyl, specifically including ethynyl, 1-propynyl, 1-butynyl, 1-pentynyl, 1-hexynyl or 2-propynyl, 2-butynyl, 1-methyl-2-propynyl, and a structural isomer thereof, preferably 2-propynyl or 2-butynyl.

The term "cycloalkyl" means a 3- to 8-membered cyclic hydrocarbon, specifically including cyclopropyl, cyclobutyl, cyclopentyl, cyclohexyl, cycloheptyl, and cyclooctyl.

The "halogen atom" includes fluorine atom, chlorine atom, bromine atom, and iodine atom.

The term "aryl" means an optionally substituted C₆₋₁₄ monocyclic to tricyclic aromatic ring, specifically including phenyl, naphthyl, anthranyl, phenanthryl, and the like, and preferably phenyl.

The term "heteroaryl" means an optionally substituted 5- to 8-membered monocyclic to tricyclic aromatic ring containing 1 to 4 heteroatoms selected from oxygen atom, sulfur atom and nitrogen atom, specifically including monocyclic heteroaryls such as furyl, thienyl, pyrrolyl, imidazolyl, thiazolyl, pyrazolyl, isothiazolyl, isoxazolyl, pyridyl, pyrimidyl, pyridazinyl, pyrazyl, triazolyl, tetrazolyl, and the like; and bicyclic heteroaryls such as indolyl, 2,3-dihydro-1H-indolyl, quinolyl, isoquinolyl, benzimidazolyl, naphthyridinyl, 1,3-benzodioxyl, 1,2,3,4-tetrahydroquinolyl, 3,4-dihydro-2H-benzo[1,4]oxazinyl, and the like.

The "5- or 6-membered saturated heterocycle" includes specifically pyrrolidine, piperidine, tetrahydrofuran, tetrahydropyran, morpholine, thiomorpholine, piperazine, and the like.

The "cyclic amino" includes specifically morpholino, piperidinyl, piperazinyl, methylpiperazinyl, pyrrolidinyl, and the like.

In this specification, as the substituent contained in "optionally substituted lower alkyl group", "optionally substituted lower alkenyl group", "optionally substituted lower alkynyl group", "optionally substituted cycloalkyl group", "optionally substituted aryl group", "optionally substituted heteroaryl" or "optionally substituted 5- or 6-membered saturated heterocyclic group", any kind of the conventionally used substituents may be used, and the respective groups may contain 1 to 3 substituents.

The preferred substituent of the "optionally substituted lower alkyl group" as R¹ includes halogen atom; cycloalkyl; optionally substituted aryl; optionally substituted 5- or 6- membered saturated heterocyclic group; optionally substituted heteroaryl; -O-aryl-; -O-heteroaryl; -NH₂; -NH-lower alkyl; -N-di-lower alkyl; cyclic alkyl; -OH; -O-lower alkyl; and -S-lower alkyl. These substituents of 2 or more may be attached to an alkyl group.

The preferred substituent of the "optionally substituted aryl group", "optionally substituted heteroaryl", "optionally substituted 5- or 6-membered saturated heterocycle" or "optionally substituted cycloalkyl" as R¹ includes halogen atom; lower alkyl; -OH; -O-lower alkyl; nitro; -NH₂; -NH-lower alkyl; -N-di-lower alkyl; cyclic amino; -CO₂H; -lower alkyl-CO₂H; -CO-lower alkyl; -lower alkyl-aryl; -lower alkyl-CO₂-lower alkyl; -CO₂-lower alkyl; -S-lower alkyl; -SO-lower alkyl; -SO₂-lower alkyl; -NHCO-lower alkyl; -NHSO₂-lower alkyl; -NHCO-cyclic amino; or-O-lower alkyl-O- group; and heteroaryl. The lower alkyl in these groups may be substituted by -OH; -NH₂; -NH-lower alkyl; or -N-di-lower alkyl; or -COOH, and it may form a new ring with the endocyclic atom present in the original ring to form a condensed ring.

The preferred substituent of the "optionally substituted lower alkyl group", or "optionally substituted alkenyl group" as R⁴, R⁵, R⁹ and R¹⁰ includes halogen atom; -OH; -O-lower alkyl; -O-aralkyl; -OCO-lower alkyl; lower alkyl-NH₂ optionally substituted by -OCO-; -COOH; -COO-lower alkyl; -NH₂; -NH-lower alkyl; -N-di-lower alkyl; lower alkyl-NH₂ optionally substituted by -NHCO-; -SO-lower alkyl; -SO₂-lower alkyl; optionally substituted aryl; optionally substituted heteroaryl; and optionally substituted 5- or 6-membered saturated heterocycle. In these substituents, the -NH₂ group may further be substituted by a -COO-lower alkyl or -COO-lower alkyl-aryl, or they may form a 1,3-dioxoisoindolin-2-yl group.

The preferred substituent of the "optionally substituted aryl group", "optionally substituted heteroaryl", or "optionally substituted 5- or 6-membered saturated heterocycle" as R⁴, R⁵, R⁹ and R¹⁰ includes halogen atom; -NH₂; -NH-lower alkyl; -N-di-lower alkyl; cyclic amino; lower alkyl; -COOH; -COO-lower alkyl; and lower alkyl-NH₂. The lower alkyl in these groups may be substituted by halogen atom; -OH; -NH₂; -NH-lower alkyl; or -N-di-lower alkyl group, and it may form a new ring with the endocyclic atom present in the original ring to form a condensed ring.

The compounds of the invention in some cases exist in the form of geometrical isomer or tautomer based on the double bond or amide bond depending on the kind of the substituent. These isomers including their isolated form and mixtures are also included in the invention. In addition, the compounds of the invention in some cases contain an asymmetric carbon or carbons and in such cases exist in the form of isomers based on the asymmetric carbon. The invention accordingly includes those optical isomers as a mixture or in an isolated form. Moreover, the invention also includes labeled compounds derived from the compounds of the invention by labeling with a radioisotope.

In addition, pharmaceutically acceptable pro-drugs are included in the compounds of the invention. The pharmaceutically acceptable pro-drugs mean the compounds of the invention in which a certain group can be converted into a functional group such as -NH2, -OH, -COOH, and so on by solvolysis or in a physiological condition. As for the groups used in the formation of the pro-drugs, those described in Prog. Med. 5, 2157-2161, 1985 or "Iyakuhin no Kaihatsu (Drug Development)" (Hirokawa Publishing Company, 1990) Vol. 7, Molecular Design, 163-196, are exemplified.

In addition, the compounds of the invention in some cases may form acid addition salts or salts with bases depending on the kind of the substituents. Such salts are also included in the invention as far as they are pharmaceutically acceptable. Specifically, such acid addition salts include those with an inorganic acid such as hydrochloric acid, hydrobromic acid, hydroiodic acid, sulfuric acid, nitric acid, phosphoric acid, and the like, and an organic acid such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, glutamic acid, and the like. Also included are salts with an inorganic base such as sodium, potassium, magnesium, calcium, aluminum, and the like, or an organic base such as methylamine, ethylamine, meglumine, ethanolamine, and the like, or a basic amino acid such as lysine, arginine, ornithine, and the like, or ammonium slats. Moreover, the invention includes a variety of the hydrates or solvates and polymorphic crystals of the compounds of the invention or their pharmaceutically acceptable salts.

### Processes for Production

The compounds of the invention and pharmaceutically acceptable salts thereof may be produced according to a variety of well-known synthetic methods utilizing the characteristics based on their basic structure or the kind of the substituents. In some functional groups, during syntheses, it is appropriate in view of the production technique to replace the functional group with a suitable protective group (one readily convertible into the original functional group) at the stage of the starting or intermediate compounds. Such a functional group is exemplified by an amino group, hydroxyl group, carboxyl group, and so on. As for the protecting groups, those described in for example Greene and Wuts, "Protective Groups in Organic Synthesis (3rd edition)" are exemplified. These protective groups may properly be selected according to the reaction condition. In such a process, the introduced protective group is removed if necessary after the main reaction, to yield the desired compound.

### (First Process)

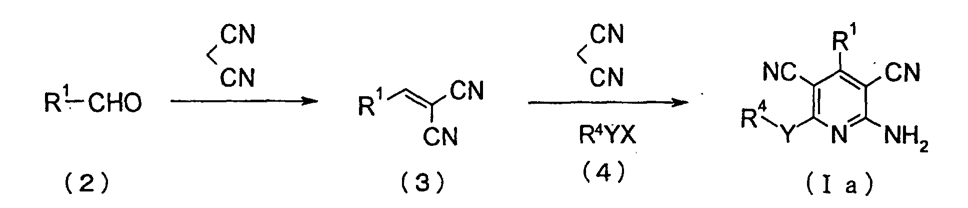

(Wherein R¹ and R⁴ have the meanings as defined above; X represents a Na, K or Li atom; Y is O or S)

Among the compounds of the invention, the compounds represented by the general formula (Ia) may be produced from the aldehydes of the general formula (2) through the dicyanoethylenes of the general formula (3).

The reaction of a reasonable amount of the aldehyde (2) with an equimolar or excess amount of malononitrile may be carried out without any solvent or in an inert solvent such as water, dimethylformamide (DMF), dimethylsulfoxide (DMSO), ether, tetrahydrofuran (THF), dioxane, acetone, methyl ethyl ketone (MEK), methanol (MeOH), ethanol (EtOH), methylene chloride, dichloroethane, chloroform, and the like, to give the dicyanoethylene (3). As the reaction solvent, a mixture of an alcohol and water is particularly preferred. It is also appropriate to use a corresponding amount of an amino acid such as glycine, a salt such as ammonium acetate, an organic base such as piperidine or its acetate, as a catalyst, with glycine being particularly preferred. The reaction is conducted at room temperature or elevated temperature, preferably at room temperature (W.S. Emerson, T.M. Patrick Jr, J. Org. Chem., 14, 790, 1949; J.B. Bastus, Tetrahedron Lett., 955, 1963, and so on).

The resulting dicyanoethylene (3) and malononitrile are then allowed to react with an equimolar or excess amount of the alkoxide or thioalkoxide of the general formula (4) without any solvent or in an inert solvent such as water, DMF, DMSO, ether, THF, dioxane, acetone, MEK, MeOH, EtOH, methylene chloride, dichloroethane, and the like, or in an alcohol corresponding to the alkoxide or thioalkoxide to give the compound (Ia). As the solvent, an alcohol is particularly preferred. The reaction is conducted at room temperature or elevated temperature, preferably at room temperature (W.J. Middleton, V.A. Engelhardt et al., J. Am. Chem. Soc., 80, 2832, 1958; Fuentes L., Soto J.L. et al., Heterocycles, 23 (1), 93, 1985, and so on).

### (Second Process)

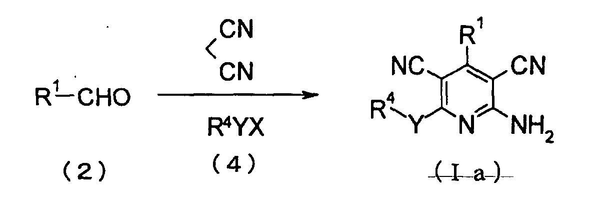

(Wherein R¹ and R⁴ have the meanings as defined above; X represents a Na, K or Li atom; Y is O or S)

Among the compounds of the invention, the compounds represented by the general formula (Ia) may also be produced directly from the aldehydes of the general formula (2).

The aldehyde (2) is allowed to react with 2 equimolar or more amounts of malononitrile and 3 equimolar or more amounts of the alkoxide or thioalkoxide of the general formula (4) without any solvent or in an inert solvent such as water, DMF, DMSO, ether, THF, dioxane, acetone, MEK, MeOH, EtOH, methylene chloride, dichloroethane, and the like or in an alcohol corresponding to the alkoxide or thioalkoxide to give the compound (Ia). As the solvent, an alcohol is particularly preferred. The reaction is conducted at room temperature or elevated temperature, preferably at room temperature (A.S. Alverez-Insua, M. Lora-Tamayo, J.L. Soto, J. Heterocycl. Chem., 7, 1305, 1970, and so on).

### (Third Process)

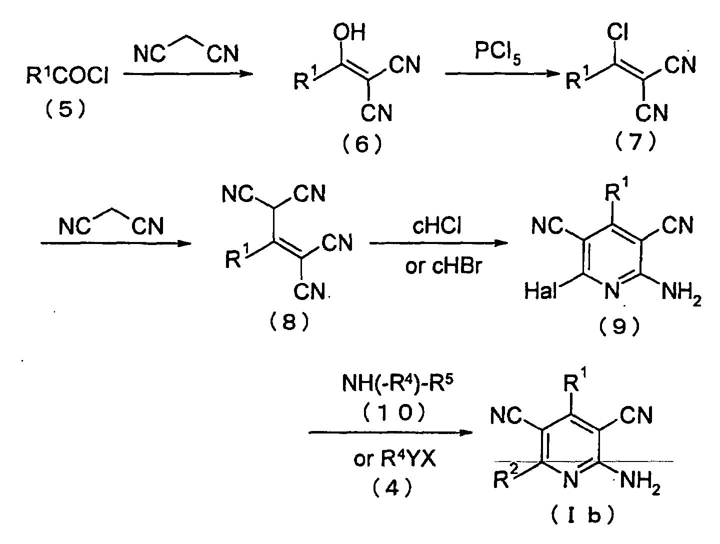

(Wherein R¹, R², R⁴ and R⁵ have the meanings as defined above; Hal represents a Br or Cl atom; X represents a Na, K or Li atom; Y is an O or S atom)

Among the compounds of the invention, the compounds represented by the general formula (Ib) may be produced according to the following process.

The acid chloride represented by the general formula (5) is allowed to react with malononitrile in an inert solvent such as dichloromethane in the presence of a base such as an aqueous sodium hydroxide solution and an organic ammonium salt such as benzyl triethylammonium chloride to give the hydroxydicyanoethylene of the general formula (6). The hydroxydicyanoethylene (6) is then allowed to react with a chlorinating agent such as phosphorus pentachloride without any solvent or in an inert solvent such as chloroform to give the chlorinated derivative of the general formula (7). The chlorinated derivative (7) is allowed to react with malononitrile in an inert solvent such as alcohol using an alkoxide such as sodium alkoxide to give the tetracyano derivative of the general formula (8), which is then allowed to react with concentrated HCl or concentrated HBr in an inert solvent such as acetone to give the halo-pyridine of the general formula (9). The halo-pyridine (9) is allowed to react with an equimolar or excess amount of an amine of the general formula (10) or alkoxide or thioalkoxide of the general formula (4) without any solvent or in an inert solvent such as DMF, DMSO, ether, THF, dioxane, acetone, MEK, MeOH, EtOH, methylene chloride, dichloroethane, and the like, if required in the presence of a base such as potassium carbonate, triethylamine, and the like.

Particularly, when R¹ is hydrogen, it is possible to carry out the same reaction using ethyl orthoformate in place of the acid chloride (5)(J. Am. Chem. Soc., 2832, 1958, ibid., 2815, 1958; J. Org. Chem., 5379, 1988, Synthesis, 8, 679, 1984, and so on).

### (Fourth Process)

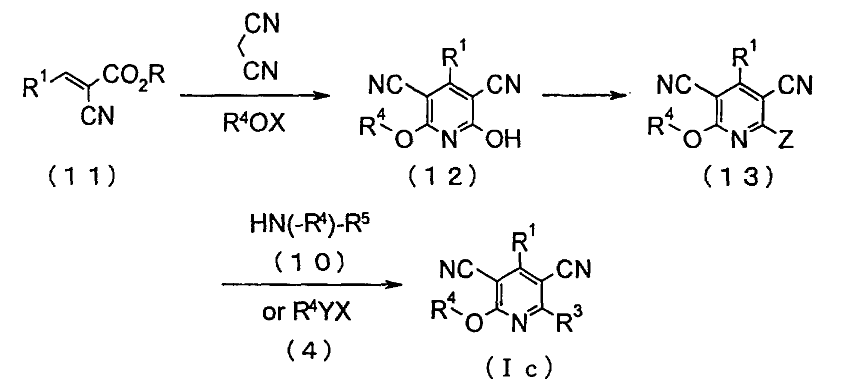

(Wherein R¹, R³, R⁴ and R⁵ have the meanings as defined above; R represents a lower alkyl group, preferably methyl or ethyl; X represents a Na, K or Li atom; Y is an O or S atom; Z is a halogen atom, p-toluenesulfonyloxy, or methane sulfonyloxy)

Among the compounds of the invention, the compounds represented by the general formula (Ic) may be produced according to the following process.

The hydroxypyridine derivatives represented by the general formula (12) can be produced according to the method described in Synthesis, p. 681, 1978. That is, the cyanoacetic acid ester derivative represented by the general formula (11) is allowed to react with malononitrile and an alkoxide in an alcohol at room temperature or under heating to give the hydroxypyridine derivative (12). The hydroxypyridine derivative (12) is subjected to halogenation with phosphorus oxychloride or sulfonylation with methanesulfonyl chloride or p-toluenesulfonyl chloride without any solvent or in an inert solvent such as methylene chloride to give the compound of the general formula (13). The compound (13) is allowed to react with an equimolar or excess amount of an amine of the general formula (10) or an alkoxide thioalkoxide of the general formula (4) without any solvent or in an inert solvent such as DMF, DMSO, ether, THF, dioxane, acetone, MEK, MeOH, EtOH, methylene chloride, dichloroethane, and the like, if required in the presence of a base such as potassium carbonate, triethylamine, and the like.

### (Fifth Process)

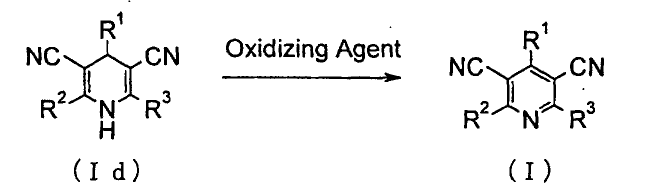

(Wherein R¹, R² and R³ have the same meanings as defined above)

The compounds (I) of the invention may also be produced from the dihydropyridines of the general formula (Id).

When the dihydropyridine (Id) is produced as a major product or by-product in the first or fourth process, it may be oxidized with an oxidizing agent such as manganese dioxide in an inert solvent such as DMF, ether, THF, dioxane, acetone, MEK, methylene chloride, dichloroethane, and the like to give the compound (I)(Alvarez, C., et al., Synth. Commun., 21(5), 619, 1991, and so on).

Alternatively, the compounds of the invention may be produced from the compounds produced in the above-described first to fifth processes by suitable conversion of the functional groups in a conventional way.

The conventional suitable conversion of the functional groups may be carried out according to the methods as described in the above-mentioned "Protective Groups in Organic Synthesis (3rd edition)", in which are described protection and deprotection of a carboxyl group, hydroxyl group, amino group, mercapto group, etc.; acylation; sulfonylation; as well as alkylation using an alkylating agent having a halogen or sulfonyloxy group with a base such as potassium carbonate or sodium hydride; oxidation of a sulfur atom with an oxidizing agent such as metachloroperbenzoic acid; conversion of an amino group into a halogeno or hydoroxyl group by the Sandmeyer reaction; removal of the lower alkyl in a lower alkyl-O- group attached at the 2 and/or 6 position of pyridine with acetic acid or concentrated hydrochloric acid; halogenation of the hydroxyl group attached at the 2 and/or 6 position of pyridine with phosphorus oxychloride; substitution of the halogen, lower alkyl-O-, lower alkyl-SO- or lower alkyl-SO₂- attached at the 2 and/or 6 position of pyridine for which can be properly applied a base such as potassium carbonate, alkali metal lower alkoxide or sodium hydride, with an alcohol, thioalcohol or amine; reduction of a nitro group, etc., with palladium-carbon, etc.; fluorination of a halogen other than fluorine with potassium fluoride; conversion of a carboxyl group into an amino group by the Curtius reaction; and the like. These reactions may be achieved according to the methods described in "Jikken Kagaku Kohza (Handbook of Experimental Chemistry) 4th edition" (Maruzen Co.; 1990-1992).

Thus resulting compounds of the invention may be isolated and purified as free products or salts thereof. The isolation and purification may be conducted in a conventional chemical procedure such as extraction, condensation, distillation, crystallization, filtration, recrystallization, a variety of chromatography, and the like.

A variety of isomers may be separated in a conventional manner utilizing the physical properties between the isomers. For example, the racemates can be converted into the sterochemically pure isomers by means of optical resolution (for example, conversion into the diastereomeric salt with a usual optically active acid (e.g., tartaric acid), followed by optical resolution). A mixture of diastereomers may be separated in a conventional manner, for example, fractional crystallization or chromatography.

In addition, the optically active compounds may also be produced from the suitable optically active starting compounds.

### Industrial Applicability

The compounds of the invention are useful as drugs for treatment of pollakiuria or urinary incontinence since they exhibit a high conductance-type calcium activated K channel (maxi-K channel) opening effect to show a smooth muscle relaxant effect in the urinary bladder. Additionally, the compounds of the invention are also useful in prophylaxis and/or treatment of hypertension, asthma, premature birth, irritable bowel syndrome, chronic heart failure, angina pectoris, myocardial infarction, cerebral infarction, subarachnoid hemorrhage, cerebrovascular spasm, cerebral hypoxia, peripheral vascular diseases, anxiety, male bald head, erectile insufficiency, diabetes mellitus, diabetic peripheral neuropathy, other diabetic complication, infertility, urinary calculus and its accompanying pain (relief).

The compounds of the invention inhibit spontaneous construction of the rat's removed bladder specimen. Since the inhibitory action is blocked by a known maxi-K channel blocker charybdotoxin or iberiotoxin, it is confirmed that the effect of the compounds of the invention is based on the maxi-K channel opening effect. Thus, the pharmacological effect of the compounds of the invention was confirmed according to the following method.

### <Inhibitory effect in construction of the rat's removed bladder specimen>

In this experiment, SD-family male rats (9-13 weeks of age) were used. The rats were killed by bleeding under ether anesthesia, and the bladders were removed. The removed bladders were immediately washed in a Klebs-Henseleit solution (NaCl 118.4, KCl 4.7, KH₂PO₄ 1.2, MgSO₄ 1.2, CaCl₂ 2.5, NaHCO₃ 25.0, glucose 11.1 [mM], aeration with 95% O₂/5% CO₂ mixed gas) kept at 37°C, and prepared into rectangular specimens of about 10 mm long and about 2 mm width on a Petri dish filled with the Klebs-Henseleit solution. The respective specimens were ligated at the both ends with a cotton string via a wire hook, and the one end was fixed to an FD pick-up and the other hung down vertically in an organ bath filled with the Klebs-Henseleit solution. After completion of the operation, 1.0 g of static tensile stress was given to the respective slices, which were then allowed to stand for 1.5-2 hours to stabilize. Then, a KCl solution was added to the organ bath so that the final K⁺ ion concentration become 15 mM to induce the contraction. Thereafter, the specimens were further allowed to stand for about 1-2 hours to stabilize, and the test was started. The contraction of the smooth muscle was measured isometrically through the FD pick-up, and the output signal was amplified through a strain stress amplifier to continuously record a chart on a pen recorder. At the same time, the respective contraction wave forms to be analyzed were recorded on a personal computer as magnetic data through an analogue/digital signal converter, and the under-area of the contraction was calculated by analytical software. The contraction 5 minutes immediately after the start of the test was regarded as the value before administration of the drug to be tested (100% reference value). The drug to be tested was added into the bath at intervals of 30 minutes, and the contraction for 5 minutes, respectively 25 minutes after the administration, was analyzed. The drug to be tested was administered at a common ratio of 3 or 10 accumulatively. The effect of the drug to be tested was represented by the dose by which 50% inhibition was attained to the value before the administration (100% reference value). Additionally, the wave form of contraction at the highest dose of the drug was recorded, and then a maxi-K channel selective blocker, charybdotoxin or iberiotoxin, was administered so that the final concentration in the organ bath became 100 nM. Thus, the effect of the drug was observed whether it was blocked or not.

| Example | Inhibition of the contraction of rat's removed bladder specimen IC₅₀/µM |
|---|---|
| 1 | 0.15 |
| 3 | 0.23 |
| 6 | 1.3 |
| 11 | 0.41 |
| 12 | 0.41 |
| 15 | 2.8 |
| 20 | 0.11 |
| 58 | 1.4 |
| 150 | 1.3 |
| 151 | 1.0 |
| 263 | 0.042 |
| NS-8 (Reference) | 1.1 |

As described above, the compounds of the invention exhibit an inhibitory effect to the contraction of the rat's removed bladder specimen. In addition, the inhibition of the contraction of the bladder smooth muscle by the compounds of the invention was confirmed to be through the effect of the maxi-K channel opening because the inhibition was blocked by administration of charybdotoxin or iberiotoxin.

### <Effect on the efflux of 86-rubidium in the cultured cells derived from human bladder>

This experiment was carried out according to the slightly modified method described in Daniel et al., Journal of Pharmacological Methods, 25, 185-193, 1991. In this experiment, the cultured cells (HTB-9) derived from human bladder were used. It has been confirmed by Monen et al. that the said cells are abundant in the maxi-K channel (J. Membrane Biol., 161, 247-256, 1998). The cells were incubated on a 96-well culture plate'in which an RPMI-1640 medium containing 10% calf serum was placed, so that the cells became confluent. The medium was then removed under suction, and further RPMI-1640 medium containing 1 µCi/ml of 86-rubidium (⁸⁶Rb) belonging to the same group as K was added so as to be 100µl/well. After lapse of 18-24 hours, the cells were washed well with an incubation solution (HEPES-buffered salt solution: comprising HBS, HEPES 20, NaCl 137, KCl 4.7, CaCl₂ 1.8, MgCl₂ 0.6, glucose 7.7 [mM]). Then, an incubation solution containing 0.3µM calcimycin (A23187) and DMSO was added at 200µl/well in the presence or absence of the test material. After lapse of 30 minute, the incubated solution was recovered with a pipette, and further a fresh incubation solution was added at 150µl/well. This was admixed with the washings to completely recover ⁸⁶Rb fluxed from the cells into the supernatant (Solution 1). Then, ⁸⁶Rb remaining in the cells was recovered. That is, 0.1M aqueous solution of NaOH was added at 0.175µl/well and agitated well for 15 minutes in a mixer to destroy the cells. This was neutralized with addition of 0.175µl/well of 0.1M HCl aqueous solution, and recovered completely with a pipette (Solution 2). In recovering the respective solutions, 96-well culture plates (white) were used as counting vessels. The ⁸⁶Rb amount contained in the counting vessels was determined by means of a liquid scintillation counter. The increase of ⁸⁶Rb eluted from the cells was calculated from [Radioactivity cpm in Solution 1]/([ Radioactivity cpm in Solution 1]+[ Radioactivity cpm in Solution 2])×100(%). The dose was calculated from the above-described efflux amount of ⁸⁶Rb which was increased by the drug to be tested and reached 60%. This was regarded as the activity of the drug.

As a result, it was found that the compounds of the invention greatly increased the efflux of ⁸⁶Rb from the cultured cells derived from human bladder. From the above results, it was demonstrated that the compounds of the invention exhibit the effect of opening the maxi-K channel of human bladder cells.

### <Effect on the rhythmic contraction of the bladder in rats under urethane anesthesia>

SD-Family female rats (about 300 g) were used. A catheter was inserted into the bladder through the external urethral orifice under urethane anesthesia and spontaneous breathing. The other end was connected to a pressure transducer and an infusion pump through a three-way cock. On the other hand, another catheter for measuring blood pressure was inserted into the right common carotid artery. Physiological saline warmed at about 38°C was injected in the bladder at a rate of 4.2 ml/hr until rhythmical bladder contraction was induced. Change of the internal pressure in the bladder was continuously recorded on a recorder. After the rhythmical bladder contraction was stabilized, a test compound suspended in 0.5% methylcellulose aqueous solution was administered through a catheter which had been attached to the duodenum. Thus, frequency of the bladder contraction (every 10 minutes), force of bladder contraction and average blood pressure were observed as evaluation items up to 2 hours after administration of the test compound.

| Example | Inhibition of frequency of the bladder contraction max% inhibition/% | Frequency of Bladder Contraction Retention time of 50% Inhibition/min (10 mg/kg, i.d.) |
|---|---|---|
| 3 | 97 | 39 |
| 6 | 65 | 39 |
| 11 | 87 | 27 |
| 12 | 77 | 31 |
| 15 | 89 | 36 |
| 20 | 86 | 40 |
| 58 | 93 | 46 |
| 150 | 81 | 30 |
| 151 | 71 | 26 |
| 252 | 78 | 32 |
| 263 | 69 | 23 |

The compounds of the invention, as mentioned above, exhibited the effect of inhibiting the frequency of bladder contraction without altering the average blood pressure and the force of bladder contraction in urethane-anesthetic rats.

From these results, it can be said that the compounds of the invention are useful as drugs for treatment of pollakiuria and/or urinary incontinence.

From the above results, it was demonstrated that the compounds of the invention exhibit the effect of opening the maxi-K channel in the bladder smooth muscle and are useful as drugs for treatment of pollakiuria and urinary incontinence.

The pharmaceutical preparations containing one or more species of the compounds of the invention or their salts may be produced with carriers or excipients conventionally used in pharmaceutical formulation as well as additives. As for the carriers or excipients for pharmaceutical preparations, solid or liquid ones, for example, lactose, magnesium stearate, starch, talc, gelatin, agar, pectin, gum arabic, olive oil, sesame oil, cacao butter, ethylene glycol, and other conventional ones are included.

Administration may be achieved by oral administration in a form of tablets, pills, capsules, granules, powder, liquid preparations, and the like, or by perenteral administration in a form of intravenous or intramuscular injections, suppositories, percutaneous preparations, and the like. The dose may be determined corresponding to individual cases taken the condition, age and sex of the subject into consideration. Usually, it may be administered orally in a single or divided dose of 1-1000 mg/day, preferably 50-200 mg/day for an adult, or intravenously at a single or divided dose of 1-500 mg/day for an adult, or continuously administered intravenously within a period of 1 to 24 hours a day. As described above, needless to say, the dose is altered depending on various conditions, and in some cases it is -sufficient in a smaller amount than that as mentioned above.

According to the invention, as an orally administrable solid composition, tablets, powder, granules, and the like are used. In such a solid composition, one or more of active materials are admixed with at least one inert diluent, for example, lactose, mannitol, glucose, hydroxypropyl cellulose, fine crystalline cellulose, starch, polyvinylpyrrolidone, metasilicic acid, or magnesium aluminate. The compositions may contain additives other than the inert diluents, for example, a lubricant such as magnesium stearate, or a disintegrator such as cellulose calcium gluconate, a stabilizer such as lactose, and a solubilizing agent such as glutamic acid or aspartic acid, according to a conventional manner. The tablets or pills, if required, may be coated with a gastric or enteric coating film such as sucrose, gelatin, hydroxypropylcellulose, hydroxypropylmethylcellulose phthalate, and the like.

In the liquid compositions for oral administration, a pharmaceutically acceptable emulsifying agent, solubilizing agent, suspending agent, syrup, elixir, and the like may be contained, and a generally used inert diluent, for example, purified water, ethanol, and the like may be contained. In addition to such inert diluents, a wetting agent, auxiliary agent such as suspending agent, sweetener, flavor, aromatic agent, antiseptic, and the like may be contained.

The injection preparations for perenteral administration include sterile aqueous or nonaqueous solutions, suspensions, and emulsions. As the aqueous solutions or suspensions, for example, distilled water or physiological saline is included. The nonaqueous solution or suspension includes, for example, propylene glycol, polyethylene glycol, a vegetable oil such as olive oil, an alcohol such as ethanol, polysorbate 80, and the like. Such compositions may further contain an antiseptic, wetting agent, emulsifying agent, dispersant, stabilizer (e.g., lactose), and solubilizing agent (e.g., glutamic acid, aspartic acid). These compositions are sterilized by filtration through a bacterial filter or by addition of a sterilizer or by irradiation. These may be prepared as a sterile solid composition and dissolved in sterile water or sterile solvent for injection before using.

### Best Mode for Carrying Out the Invention

The invention will be explained in more details by the following examples which are not intended as a limitation thereof.

### Reference Example 1

To a solution of 10 ml of benzaldehyde in 100 ml of EtOH-water (7:3) was added 6.5 g of malononitrile and 0.1 g of glycine, and the mixture was stirred at room temperature for 6 hours. The precipitated crystals were collected by filtration, washed with EtOH-water (7:3), and dried under reduced pressure to give 13.1 g of benzylidenemalononitrile.

In the same manner as in Reference Example 1, the compounds of Reference Examples 2 to 7 were produced.

### Reference Example 8

To a solution of 5.0 g of 4-aminomethylbenzoic acid in 40 ml of dioxane-water (1:1) was added 6.0 g of NaHCO₃ and a solution of 7.6 g of di-tertiary butyl dicarbonate in 20 ml of dioxane in order at room temperature, and the mixture was stirred at room temperature for 4 days. The solvent was distilled off under reduced pressure, and the residue was neutralized with aqueous hydrochloric acid. The precipitated solid was collected by filtration, and dried under reduced pressure to give 8.0 g of a carboxylic acid. The carboxylic acid (0.85 g) was dissolved in 10 ml of THF, to which was added 0.60 g of 1,1'-carbonylbis-1H-imidazole under ice-cooling, and the mixture was stirred at 50°C for 40 minutes. To the resulting solution was added 0.43 g of N,O-dimethylhydroxylamine hydrochloride and 0.7 ml of triethylamine (Et₃N) in order under ice-cooling, and the mixture was stirred overnight at room temperature. Water was added to the mixture, and the mixture was extracted with ethyl acetate (EtOAc). The resulting organic layer was dried over anhydrous sodium sulfate, filtered, and evaporated under reduced pressure. The resulting residue was purified by silica gel column chromatography to give 0.98 g of an amide. The amide (0.98 g) was dissolved in 10 ml of THF, to which was added 0.12 g of lithium aluminum hydride at -78°C, and the mixture was stirred at the same temperature for 40 minutes. There was added 2.0 g of Na₂SO₄·10H₂O and the mixture was warmed up to room temperature and stirred for 3 hours. The reaction mixture was filtered and evaporated under reduced pressure to give 0.76 g of tertiary (t-)butyl 4-formylbenzylcarbamate.

### Reference Example 9

To a solution of 2.0 g of 3-bromobenzylamine hydrochloride in 20 ml of dioxane-water (1:1) was added 1.5 g of NaHCO₃ and a solution of 2.2 g of di-t-butyl dicarbonate in 10 ml of dioxane in order at room temperature, and the mixture was stirred at room temperature for 1 day. Water was added to the reaction mixture and the reaction mixture was extracted with EtOAc. The organic layer was dried over anhydrous sodium sulfate and evaporated under reduced pressure to give 3.0 g of a bromo-derivative. The bromo-derivative (3.0 g) was dissolved in 30 ml of THF, to which was added 14 ml of 1.5M butyllithium/hexane solution at -78°C, and the mixture was stirred at the same temperature for 30 minutes. To the resulting solution was added a solution of 1.7 ml of DMF in 10 ml of THF at -78°C, and the mixture was warmed up to -15°C over 1.5 hours. An aqueous ammonium chloride solution was added to the reaction mixture and extracted with EtOAc. The resulting organic layer was dried over anhydrous sodium sulfate, filtered, and evaporated under reduced pressure. The resulting residue was purified by silica gel column chromatography to give 0.89 g of t-butyl 3-formylbenzylcarbamate.

### Reference Example 10

To 9.0 g of malononitrile was added 13.5 ml of ethyl orthoformate and 5.6 ml of pyridine (Py) at room temperature, and the mixture was stirred at 120°C for 30 minutes. The mixture was allowed to cool to room temperature, and to the mixture EtOH was added to yield crystals as precipitate, which was collected by filtration to give 10.2 g of 1,1,3,3-tetracyanopropene pyridine salt. This (6.2 g) was dissolved in 50 ml of acetone, to the solution was added 20 ml of concentrated hydrochloric acid (c-HCl) under ice cooling, and the mixture was stirred at 50°C overnight. The precipitated crystals were collected by filtration, washed with EtOH, and dried under reduced pressure to give 4.47 g of 2-amino-6-chloropyridine-3,5-dicarbo-nitrile.

### Reference Example 11

To a solution of 2.0 g of 4-hydroxybenzonitrile in 20 ml of DMF was added 2.8 g of potassium carbonate and 2.2 ml of benzyl bromide in order under ice cooling, and the mixture was stirred at room temperature for 1 day. The reaction mixture was concentrated under reduced pressure, and water was added and extracted with EtOAc. The resulting organic layer was dried over anhydrous sodium sulfate, filtered, and evaporated under reduced pressure to give 4-benzyloxy-benzonitrile. This compound (3.7 g) was dissolved in 40 ml of THF, to the solution was added 20 ml of 1M-BH₃-THF in 20 ml of THF under ice cooling, and the mixture was heated under reflux with stirring for 1 hour. The reaction mixture was cooled in an ice bath, 10 ml of MeOH was added to the mixture, and the mixture was heated under reflux with stirring for 30 minutes. The reaction mixture was again cooled in an ice bath, added 2.0 ml of c-HCl to the mixture, and the mixture was heated under reflux with stirring for 30 minutes. The reaction mixture was then allowed to cool to room temperature, and the precipitated solid was collected by filtration to give 4-benzyloxybenzylamine hydrochloride. The 4-benzyloxybenzylamine hydrochloride (1.28 g) was dissolved in 30 ml of dioxane-water (1:1), to the solution was added 0.65 g of NaHCO₃ and a solution of 1.3 g of di-t-butyl dicarbonate in 5.0 ml of dioxane in order at room temperature, and the mixture was stirred at room temperature for 4.5 hours. The solvent was distilled off under reduced pressure, water was then added to the residue, and the mixture was extracted with ethyl acetate. The resulting organic layer was dried over anhydrous sodium sulfate, filtered, and evaporated under reduced pressure to give t-butyl 4-benzyloxybenzylcarbamate. This compound (1.96 g) was dissolved in 20 ml of ethyl acetate, to the solution was added 0.20 g of 10% palladium-carbon (Pd/C), and the mixture was stirred in hydrogen under atmospheric pressure at room temperature overnight. The reaction mixture was filtered and evaporated under reduced pressure, and the resulting residue was purified by silica gel column chromatography to give 1.21 g of t-butyl 4-hydroxybenzylcarbamate.

In the same manner as in Reference Example 11, the compounds of Reference Examples 12 and 13 were produced.

### Reference Example 14

To a solution of 6.0 g of 3-carboxybenzaldehyde in 40 ml of 29% ammonia water was added 11.6 g of 40% glyoxal aqueous solution at 0°C. The mixture was warmed up to room temperature and stirred for 16 hours. The reaction mixture was concentrated under reduced pressure and neutralized with c-HCl at pH 7.0, and the precipitated crude crystals were collected by filtration, and washed with water and EtOH to give 5.3 g of 3-(1H-imidazol-2-yl)benzoic acid [¹H-NMR (DMSO-*d*₆): δ 3.31 (1H, brs), 7.17 (2H, s), 7.57 (1H, t), 7.89 (1H, dt), 8.17 (1H, td), 8.55 (1H, d)].

This compound (500 mg) was dissolved in 10 ml of DMF, to the solution was added 646 mg of 1,1'-carbonyldiimidazole at room temperature. At room temperature, to the mixture was added 520 mg of N,O-dimethylhydroxylamine hydrochloride and 1.0 ml of Et₃N, and stirred. To the mixture was added 10 ml of water and the mixture was extracted with EtOAc. The organic layer was washed with a saturated sodium chloride aqueous solution (brine), dried over magnesium sulfate (MgSO₄), and evaporated to give a crude product. This was purified by silica gel column chromatography to give 600 mg of 3-(1H-imidazol-2-yl)-N-methoxy-N-methylbenzamide [¹H-NMR (DMSO-*d*₆): δ 3.31 (3H, s), 3.56 (3H, s), 7.04 (1H, s), 7.27 (1H, s), 7.48-7.52 (3H, m), 8.16 (1H, m), 8.31 (1H, s)].

This compound (3.4 mg) was dissolved in 20 ml of THF, to the solution was added 29 ml of diisobutylaluminum hydride (DIBAL; 1M toluene solution) at 0°C. The mixture was stirred for 2 hours, then added 8 ml of DIBAL (1M toluene solution) to the mixture, and it was further stirred for 2 hours. Then, 5 ml of 1M-HCl aqueous solution(aq.) was added to the mixture, and the mixture was extracted with EtOAc. The organic layer was washed with brine, dried over MgSO₄, and evaporated to give a crude product. This was separated and purified by silica gel column chromatography to give 2.2 g of 3-(1H-imidazol-2-yl)benzaldehyde.

### Reference Example 15

To a solution of 2.0 g of 3-hydroxybenzaldehyde in 15 ml of DMF was added 2.5 g of K₂CO₃ and 7.2 g of ethylene carbonate at room temperature, and the mixture was heated up to 100°C and stirred for 3 hours. The solvent was distilled off, and the residue was purified by silica gel column chromatography to give 2.7 g of 3-(2-hydroxyethoxy)benzaldehyde. This compound (2.7 g) was dissolved in 50 ml of EtOH-water (7:3), to the solution was added 1.08 g of malononitrile and 50 mg of glycine, and the mixture was stirred at room temperature overnight. The mixture was then extracted with EtOAc, washed with brine, dried over MgSO₄ and evaporated to give a crude product. This was separated and purified by silica gel column chromatography to give 2-[3-(2-hydroxyethoxy)benzylidene]malononitrile in quantitative yield.

### Example 1

To 20 ml of MeOH was added 0.70 g of Na under ice cooling, and the mixture was stirred at room temperature until Na was dissolved. To the mixture were added 0.85 g of malononitrile and 2.0 g of the compound prepared in Reference Example 3, and the mixture was heated under reflux with stirring for 3 hours. The reaction mixture was poured into ice water, and the precipitated crystals were collected by filtration, recrystallized from ethyl acetate, and dried under reduced pressure to give 0.25 g of 2-amino-6-methoxy-4-(2-thienyl)pyridine-3,5-dicarbo-nitrile.

In the same manner as in Example 1, the compound of Example 2 was synthesized.

### Example 3

To 50 ml of MeOH was added 4.00 g of sodium methoxide, 3.19 g of malononitrile, and 3.00 g of 2-fluorobenzaldehyde under ice cooling, and the mixture was stirred at room temperature overnight. The precipitated crystals were collected by filtration, washed with methanol, and dried under reduced pressure to give 1.05 g of 2-amino-6-methoxy-4-(2-fluorophenyl)pyridine-3,5-dicarbonitril.

In the same manner as in Example 3, the compounds of Examples 4 and 5 were synthesized.

### Example 6

In argon atmosphere, 3.4 g of DMSO in 5 ml of methylene chloride was dropwise added to a solution of 2.8 g of oxalyl dichloride in 75 ml of methylene chloride at -78°C and stirred for 10 minutes. To the solution was dropwise added a solution of 2.3 g of tetrahydropyran-2-methanol in 15 ml methylene chloride at the same temperature. The mixture was then warmed up to room temperature over 1 hour, and then to the mixture was added 10 g of Et₃N. The reaction mixture was poured into water and extracted with chloroform. The organic layer was dried over anhydrous sodium sulfate and evaporated under reduced pressure to give tetrahydropyran-2-carbaldehyde. This was dissolved in 30 ml of MeOH, to the solution was added 2.6 g of malononitrile and 3.2 g of sodium methoxide in order under ice cooling. The reaction mixture was stirred at room temperature for 5 days, poured into a saturated ammonium chloride aqueous solution, and the mixture was extracted with EtOAc. The organic layer was dried over anhydrous sodium sulfate, and concentrated under reduced pressure. The residue was purified by silica gel column chromatography to give 0.73 g of 2-amino-4-(2-tetrahydropyranyl)-6-methoxypyridine-3,5-dicarbonitrile.

### Example 7

To a solution of 2.0 g of the compound prepared in Reference Example 4 in 10 ml of EtOH was added 0.85 g of malononitrile and 0.90 g of sodium thiomethoxide under ice cooling, and the mixture was stirred at room temperature overnight. The precipitated crystals were collected by filtration, washed with EtOH, and dried under reduced pressure to give 1.44 g of 2-amino-6-methylsulfanyl-4-(3-thienyl)pyridine-3,5-dicarbonitrile.

In the same manner as in Example 7, the compound of Example 8 was synthesized.

### Example 9

To a solution of 12 g of malononitrile in 300 ml of methylene chloride was added 20 ml of benzoyl chloride, 3.0 g of bentyl triethylammonium chloride and 40 ml of 10M NaOH aq. under ice cooling, and the mixture was stirred at room temperature overnight. The resulting solid material was collected by filtration, dissolved in water, the solution was neutralized with c-HCl, and extracted with chloroform. The organic layer was dried over anhydrous sodium sulfate, filtered, and concentrated under reduced pressure to give 22.6 g of benzoylmalononitrile. This compound (22.6g) was dissolved in 200 ml of methylene chloride, to the solution was added 50 g of phosphorus pentachloride, and the mixture was heated under reflux with stirring overnight. The mixture was then concentrated under reduced pressure and purified by silica gel column chromatography to give 15.2 g of a chloro-derivative. This chloro-derivative (7.2 g) was allowed to react with an EtOH solution of malononitrile sodium salt prepared from 70 ml of EtOH, 1.8 g of Na and 2.6 g of malononitrile, under ice cooling for a day to give 7.44 g of a tetracyano-derivative. This tetracyano-derivative (1.0 g) was dissolved in 20 ml of acetone, to the solution was added 5.0 ml of c-HCl, and the mixture was stirred at 50°C for 4.5 hours. The precipitated crystals were collected by filtration, washed with EtOH, and dried under reduced pressure to give 0.95 g of 2-amino-6-chloro-4-phenylpyridine-3,5-dicarbonitrile.

In the same manner as in Example 9, the compound of Example 10 was synthesized.

### Example 11

To a solution of 202 mg of propargyl alcohol in 5 ml of DMF was added 145 mg of 60% sodium hydride (NaH) under ice cooling, and the mixture was stirred at room temperature for 10 minutes. To the mixture was added 500 mg of the compound prepared in Example 10, and the mixture was stirred at room temperature for 2 hours. Further, to the mixture was added 404 mg of propargyl alcohol and 290 mg of 60% NaH, and the mixture was stirred for 1 hour, and ice was added to it. The reaction mixture was acidified with hydrochloric acid aqueous solution, and the precipitated solid material was collected by filtration and washed with water and hexane. The resulting solid material was recrystallized from EtOH to give 308 mg of 2-amino-4-(2-fluorophenyl)-6-(prop-2-yn-1-yloxy)pyridine-3,5-dicarbonitrile.

### Example 12

To a solution of 500 mg of the compound prepared in Example 10 in 5 ml of DMF was added 444 mg of 2,2-difluoroethanol and 278 mg of 60% NaH under ice cooling. The reaction mixture was stirred at room temperature for 3 hours, and then ice was added to it. The precipitated solid material was collected by filtration and washed with water and hexane. The resulting solid material was recrystallized from EtOH to give 306 mg of 2-amino-6-(2,2-difluoroethoxy)-4-(2-fluorophenyl)pyridine-3,5-dicarbonitrile.

### Example 13

To 10 ml of ethylene glycol was added 0.10 g Na at room temperature, and the mixture was stirred at 60°C until Na was dissolved. To the mixture was added 0.30 g of the compound prepared in Example 9, and the mixture was stirred at room temperature for 1 day. Water was added to the mixture, and the precipitated crystals were collected by filtration. The resulting solid material was recrystallized from EtOH to give 0.28 g of 2-amino-6-(2-hydroxyethoxy)-4-phenylpyridine-3,5-dicarbonitrile.

### Example 14

A mixture of 310 mg of the compound prepared in Example 13, 380 mg of N-carbobenzoxy-L-valine, 350 mg of 1-ethyl-3-(3-dimethylaminopropyl)carbodiimide hydrochloride (WSC HCl), 50 mg of 4-dimethylaminopyridine (DMAP) and 6 ml of DMF was stirred at room temperature overnight. The mixture was concentrated under reduced pressure, and the residue was dissolved in ethyl acetate and washed with water. The organic layer was dried over anhydrous MgSO₄, concentrated under reduced pressure, and the resulting residue was purified by silica gel column chromatography (ethyl acetate:chloroform = 1:4) to give 461 mg of 2-{(2-amino-3,5-dicyano-4-phenylpyridin-6-yl)oxy} ethyl (S)-2-benzyloxycarbonylamino-3-methylbutanoate [¹H-NMR (DMSO-*d*₆): δ 0.87 (6H, d), 1.97-2.08 (1H, m), 3.91-3.97 (1H, m), 4.34-4.64 (4H, m), 5.03 (2H, brm), 7.28-7.71 (11H, m), 8.02 (2H, brs)].

A mixture of 406 mg of this compound, 50 ml of THF, 400 mg of 10% palladium-carbon/50% water, 30 ml of MeOH and 1 ml of 1M HCl aq. was stirred under hydrogen pressure of 3 kg/cm² for 1 hour in a Parr apparatus. The reaction mixture was filtered through celite and concentrated under reduced pressure. The residue was dissolved in chloroform and washed with a sodium bicarbonate aqueous solution. The organic layer was dried over MgSO₄, concentrated under reduced pressure, and the residue was purified by silica gel column chromatography (MeOH:chloroform = 3:97) to give 113 mg of 2-[(6-amino-3,5-dicyano-4-phenylpyridin-2-yl)oxy]ethyl (S)-2-amino-3-methylbutanoate. This compound was dissolved in MeOH together with 26 mg of oxalic acid, and the solvent was distilled off. The precipitated crystals were washed with ethyl acetate to give 122 mg of 2-[(6-amino-3,5-dicyano-4-phenylpyridin-2-yl)oxy]ethyl (S)-2-amino-3-methylbutanoate monooxalate.

### Example 15

A mixture of 348 mg of the compound prepared in Example 13, 440 mg of N-(t-butoxycarbonyl)-L-phenylalanine, 390 mg of WSC HCl, 50 mg of DMAP and 6 ml of DMF was stirred at room temperature for 2 hours. The mixture was concentrated under reduced pressure, and the residue was dissolved in ethyl acetate and the organic layer was washed with water. The organic layer was dried over anhydrous MgSO₄, concentrated under reduced pressure, and the resulting residue was purified by silica gel column chromatography (EtOAc:chloroform = 3:17) to give 635 mg of 2-[(2-amino-3,5-dicyano-4-phenylpyridin-6-yl)oxy]ethyl (S)-2-(t-butoxycarbonylamino)-3-phenylpropanoate [¹H-NMR (DMSO-*d*₆): δ 1.31 (9H, s), 2.80-3.03 (2H, m), 4.13-4.62 (5H, m), 7.15-7.28 (5H, m), 7.32 (1H, d), 7.46-7.60 (5H, m), 8.02 (2H, brs)].

A mixture of 575 mg of this compound, 30 ml of MeOH and 6 ml of 4M HCl-EtOAc solution was stirred at room temperature or under refluxing for 20 minutes. The reaction mixture was concentrated under reduced pressure, and the precipitated crystals were washed with EtOAc to give 401 mg of 2-[(2-amino-3,5-dicyano-4-phenylpyridin-6-yl)oxy]ethyl (S)-2-amino-3-phenylpropanoate monohydrochloride monohydrate.

### Example 16

To a solution of 3.00 g of the compound prepared in Example 4 in 10 ml of MeOH was added 2 ml of concentrated sulfuric acid, and the mixture was heated under reflux with stirring for 5 hours. The reaction mixture was cooled to room temperature, and the precipitated crystals were collected by filtration to give 2.60 g of methyl 4-(2-amino-3,5-dicyano-6-methoxypyridin-4-yl)benzoate.

### Example 17

To a suspension of 1.3 g of the compound of Example 77 in 40 ml of dichloromethane was added 1.1 g of metachloroperbenzoic acid under ice cooling, and the mixture was stirred for 1 hour. The reaction mixture was washed with a saturated sodium hydrogencarbonate aqueous solution and extracted with chloroform. The organic layer was dried over anhydrous sodium sulfate, concentrated under reduced pressure, and the residue was recrystallized from EtOH to give 0.57 g of 2-amino-6-methanesulfinyl-4-(2-fluorophenyl)pyridine-3,5-dicarbonitrile.

### Example 18

To a solution of 400 mg of the compound prepared in Example 17 in 6 ml of propan-2-ol was added 60 mg of 60% NaH, and the mixture was stirred for 1 hour. The reaction mixture was poured into water, and the precipitate was collected by filtration and recrystallized from EtOH to give 140 mg of 2-amino-4-(2-fluorophenyl)-6-isopropoxypyridine-3,5-dicarbonitrile.

### Example 19

To a solution of 500 mg of the compound prepared in Example 1 in 10 ml of Py was added 5 ml of acetic anhydride and 25 mg of DMPA, and the mixture was stirred at room temperature for 20 hours. The reaction mixture was concentrated, and the residue was recrystallized from EtOH to give 370 mg of N-(3,5-dicyano-6-methoxy-4-thiophen-2-ylpyridin-2-yl)acetamide.

### Example 20

To a solution of 7.00 g of the compound prepared in Example 4 in t-butyl alcohol was added 8.47 g of diphenylphosphoryl azide and 3.12 g of Et₃N, and the mixture was stirred under refluxing for 5 hours. Water was added to the reaction mixture and extracted with EtOAc. The organic layer was washed with brine, dried over anhydrous MgSO₄, filtered, and concentrated under reduced pressure. The residue was purified by silica gel column chromatography to give 5.75 g of t-butyl [4-(2-amino-3,5-dicyano-6-methoxypyridin-4-yl)phenyl]carbamate. This compound was dissolved in dioxane, to the solution was added 10 ml of 4M HCl-acetic acid solution, and the mixture was stirred under heating at 50°C for 2 hours. Further, 10 ml of 4M HCl-acetic acid solution was added, and the mixture was stirred under heating at 50°C for 3 hours. The reaction mixture was allowed to cool to room temperature, and the precipitated crystals were collected by filtration. The crystals were suspended in MeOH, adjusted to pH 10 with addition of 1M NaOH aq., then stirred at room temperature for 2 hours, and collected by filtration. The crystals were added to MeOH, to the mixture was further added 6.0 ml of 4M HCl-EtOAc solution. The reaction mixture was concentrated, and the residual crystals were washed with MeOH to give 1240 mg of 2-amino-(4-aminophenyl)-6-methoxypyridine-3,5-dicarbonitrile monohydrochloride monohydrate.

### Example 21

To 1.00 g of the compound prepared in Example 5 was added 10 ml of acetic acid and 2 ml of c-HCl, and the mixture was stirred under heating at 100°C for 2 hours, and then allowed to cool to room temperature. The precipitated crystals were collected by filtration and recrystallized from acetone-water to give 416 mg of 2-amino-(4-aminophenyl)-6-hydroxypyridine-3,5-dicarbonitrile.

### Example 22

To 500 mg of the compound prepared in Example 5 was added 2.2 ml of 1.5M KOH/MeOH-aqueous solution and 20 ml of MeOH, and the mixture was stirred under heating at 60°C for 3 hours. The mixture was then neutralized with 1M HCl aq., and the precipitated crystals were collected by filtration and recrystallized from acetone-water to give 218 mg of 4'-(2-amino-3,5-dicyano-6-hydroxypyridin-4-yl)acetanilide.

### Examples 23 and 24

To a suspension of 1.0 g of the compound prepared in Example 8 in 30 ml of dibromomethane was added 6 ml of isoamyl nitrite, and the mixture was stirred for 3 days. The precipitate was collected from the reaction mixture by filtration and recrystallized from EtOH to give 0.087 g of 2-hydroxy-6-methylsulfanyl-4-thiophen-2-ylpyridine-3,5-dicarbonitrile (Example 23). In addition, the mother liquid was evaporated under reduced pressure and the residue was purified by silica gel column chromatography to give 1.9 g of 2-bromo-6-methylsulfanyl-4-thiophen-2-ylpyridine-3,5-dicarbonitrile (Example 24).

### Examples 25, 26 and 27

To a suspension of 16 g of the compound prepared in Example 2 in 500 g of dibromomethane was added 47 ml of isoamyl nitrite, and the mixture was stirred for 10 days. The precipitate was collected from the reaction mixture by filtration and recrystallized from EtOH to give 0.27 g of 2-hydroxy-6-methoxy-4-phenylpyridine-3,5-dicarbonitrile (Example 25). In addition, the mother liquid was evaporated under reduced pressure and the residue was purified by silica gel column chromatography to give 1.9 g of 2-methoxy-4-phenylylpyridine-3,5-dicarbonitrile (Example 26) and 3.5 g of 2-bromo-6-methoxy-4-phenylylpyridine-3,5-dicarbonitrile (Example 27).

### Example 28

To a solution of 0.88 g of 2-amino-6-methoxy-4-(4-t-butoxycarbonylaminomethyl)phenylpyridine-3,5-di carbonitrile (which was synthesized starting from 0.95 g of the compound prepared in Reference Example 5 in the same manner as in Example 1) in 10 ml of EtOAc was added 4.0 ml of 4M HCl-EtOAc solution under ice cooling, and the mixture was stirred at room temperature for a day. The precipitated solid material was collected by filtration, dissolved in water, and neutralized with a sodium carbonate aqueous solution. The resulting solid material was collected by filtration, washed with EtOH, and dried under reduced pressure to give 0.37 g of 2-amino-4-(4-aminomethylphenyl)-6-methoxypyridine-3,5-dicarbonitrile. This compound (0.37 g) was added to 15 ml of EtOH, to the solution was added 1.0 ml of 4M HCl-EtOAc solution, and the mixture was heated under reflux for dissolution. The mixture was filtered while hot and evaporated under reduced pressure. The resulting crystals were washed with EtOH and dried under reduced pressure to give 0.27 g of 2-amino-4-(4-aminomethylphenyl)-6-methoxypyridine-3,5-dicarbonitrile monohydrochloride.

### Example 29

To a solution of 1.0 g of the compound of Example 79 in a mixture of 40 ml of EtOH and 10 ml of water was added 1.0 g of NaOH, and the mixture was stirred for 3 hours. The reaction mixture was acidified with c-HCl, and the precipitate was collected by filtration and recrystallized from EtOH to give 251 mg of 4-(2-amino-3,5-dicyano-6-methylsulfanylpyridin-4-yl)benzoic acid.

### Example 30

To a solution of 500 mg of the compound of Example 83 in 20 ml of dioxane was added 8 ml of 6M HCl aq., and the mixture was heated at 70°C with stirring for 3 hours. The reaction mixture was concentrated under reduced pressure, and the residue was washed with EtOH, and the washings were concentrated. The residual crystals were washed with ether to give 125 mg of 3-[2-(2-amino-3,5-dicyano-6-methylsulfanylpyridin-4-yl)-1H-pyrrol-1-yl]propio nic acid.

### Example 31

To 0.33 g of the compound of Example 199 was added 5 ml of 25% hydrobromic acid/acetic acid solution at room temperature, and the mixture was stirred at 110°C for a day. The reaction mixture was allowed to cool to room temperature, and the resulting crystals were collected by filtration and dried under reduced pressure to give 0.08 g of 3-[(6-amino-3,5-dicyano-4-phenylpyridin-2-yl)oxy]benzoic acid.

### Example 32

To 2 ml of trifluoroacetic acid was added 300 mg of t-butyl 3-(2-amino-3,5-dicyano-6-methoxypyridin-4-yl)piperidine-1-carboxylate [¹H-NMR (DMSO-*d*₆): δ 1.42 (9H, s), 1.73-1.92 (2H, m), 2.20-2.40 (1H, m), 2.55-2.65 (1H, m), 2.92-3.02 (1H, m), 3.25-3.30 (1H, m), 3.92-4.06 (5H, m), 7.90 (2H, brs)] (synthesized starting from t-butyl 3-hydroxymethylpiperidine-1-carboxylate in the same manner as in Example 6) under ice cooling, and the mixture was stirred for 30 minutes. To the mixture was added 20 ml of 1M NaOH aq., and the resulting precipitate was recrystallized from EtOH to give 100 mg of 2-amino-6-methoxy-4-piperidine-3,5-dicarbonitrile.

### Example 33

To 0.20 g of 2-(2-methylpropan-2-yloxyethoxy)-6-methoxy-4-phenylpyridine-3,5-dicarbonit rile [¹H-NMR (DMSO-*d*₆): δ 1.24 (9H, s), 3.74-3.81 (2H, m), 4.13 (3H, s), 4.55-4.64 (2H, m), 7.51-7.57 (5H, m)](synthesized starting from the compound of Example 357 in the same manner as in Example 11) was added 5 ml of trifluoroacetic acid under ice cooling, and the mixture was stirred at room temperature for 15 minutes. The reaction mixture was poured into water and extracted with EtOAc. The organic layer was dried over anhydrous sodium sulfate and concentrated under reduced pressure. The residue was purified by silica gel column chromatography to give 0.13 g of 2-(2-hydroxyethoxy)-6-methoxy-4-phenylpyridine-3,5-dicarbonitrile.

### Example 34

To a solution of 2.80 g of 3-(2-amino-3,5-dicyano-6-methoxypyridin-4-yl)benzoic acid [¹H-NMR (DMSO-*d*₆): δ 3.92 (3H, s), 7.68-7.82 (2H, dm), 8.02-8.36 (4H, m), 13.3 (1H, brs)](synthesized starting from 3-carboxybenzaldehyde in the same manner as in Example 3) in 60 ml of MeOH was added 1 ml of concentrated sulfuric acid, and the mixture was heated under reflux with stirring overnight. The reaction mixture was allowed to cool to room temperature, and the precipitated crystals were collected by filtration to give 2.41 g of methyl 3-(2-amino-3,5-dicyano-6-methoxypyridin-4-yl)benzoate. This compound (40 g) was dissolved in 60 ml of THF, to the solution was dropwise added 25 ml of DIBAL (1M toluene solution) at 0 to -5°C, and the mixture was stirred at the same temperature for 1 hour. To the mixture was added 1M HCl aq., and the mixture was extracted with EtOAc. The organic layer was washed with brine, dried over MgSO₄, filtered, and concentrated under reduced pressure. The residual crystals were washed with MeOH to give 1.40 g of 2-amino-4-[3-(hydroxymethyl)phenyl]-6-methoxypyridine-3,5-dicarbonitrile.

### Example 35

To a solution of 500 mg of 2-amino-6-methylsulfanyl-4-(3-nitrophenyl)pyridine-3,5-dicarbonitrile [¹H-NMR (DMSO-*d*₆): δ 2.67 (3H, s), 7.89 (1H, t), 8.04 (1H, d), 8.15 (2H, brs), 8.44 (1H, m), 8.49 (1H, m)](synthesized starting from 3-nitrobenzaldehyde in the same manner as in Example 7) in THF was added 50 mg of 10% Pd/C, and the mixture was hydrogenated in hydrogen gas with stirring at room temperature for 3 hours. After filtration, additional 200 mg of 10% Pd/C was added to the filtrate, and the mixture was hydrogenated in hydrogen gas with stirring at room temperature for 1 hour. After filtration, additional 200 mg of 10% Pd/C was added to the filtrate, and the mixture was hydrogenated in hydrogen gas with stirring at room temperature for 5 hours. After filtration, the filtrate was concentrated and the residue was purified by silica gel column chromatography. The resulting aniline derivative was added to chloroform/MeOH, to the solution was further added 0.55 ml of 4M HCl-EtOAc solution. The reaction mixture was concentrated and the residual crystals were washed with ethanol to give 170 mg of 2-amino-4-(3-aminophenyl)-6-methylsulfanylpyridine-3,5-dicarbonitrile monohydrochloride.

### Example 36

To a solution of 170 mg of the compound prepared in Example 20 in 5 ml of Py was added 97 mg of morpholine-4-carbonyl chloride, and the mixture was stirred at room temperature for 6 days. The mixture was acidified with addition of 1M HCl aq., and the crystals were collected by filtration and washed with THF to give 101 mg of 4'-(2-amino-3,5-dicyano-6-methoxypyridin-4-yl)morpholine-4-carbonitrile.

### Example 37

To a solution of 500 mg of the compound of Example 100 in DMF was added 323 mg of 2-diethylamino-1-chloroethane hydrochloride and 516 mg of K₂CO₃, and the mixture was stirred at room temperature for 1 hour and then at 70°C for 30 minutes. There was further added 64 mg of 2-diethylamino-1-chloroethane hydrochloride and 103 mg of mg of K₂CO₃, and the mixture was stirred at 70°C for 1 hour. Water was added to the mixture, and the mixture was extracted with EtOAc. The organic layer was washed with brine, dried over MgSO₄, and filtered. To the filtrate was added 1 ml of 4M HCl aq., and the mixture was concentrated under reduced pressure. The residue was crystallized from ether-EtOH to give 480 mg of 2-amino-4-[4-(2-diethylaminoethoxy)phenyl]-6-methoxypyridine-3,5-dicarboni trile monohydrochloride.

### Example 38

To a solution of 500 mg of the compound prepared in Example 20 in Py was added 228 mg of methanesulfonyl chloride, and the mixture was stirred at room temperature overnight. The reaction mixture was acidified with addition of 1M HCl aq., and the crystals were collected by filtration and recrystallized from acetonitrile to give 286 mg of N-[4-(2-amino-3,5-dicyano-6-methoxypyridin-4-yl)phenyl]methanesulfonamid e.

### Example 39

To a solution of 2-amino-6-methoxy-4-(1-tritylpiperidin-4-yl)pyridine-3,5-dicarbonitrile (synthesized starting from 2.0 g of 1-tritylpiperidin-4-ylmethanol in the same manner as in Example 6) in 20 ml of acetone was added 5 ml of c-HCl, and the resulting precipitate was collected by filtration and recrystallized from EtOH to give 632 mg of 2-amino-6-methoxy-4-piperidin-4-ylpyridine-3,5-dicarbonitrile monohydrochloride dihydrate.

### Example 40

To a solution of 1.09 g of 2-amino-6-[(2,2-dimethyl-1,3-dioxolan-4-yl)methoxy]phenylpyridine-3,5-dicar bonitrile [[¹H-NMR (DMSO-*d*₆): δ 1.30 (3H, s), 1.36 (3H, s), 3.60-3.90 (1H, m), 3.95-4.25 (1H, m), 4.44 (3H, brs), 7.55 (5H, brs), 7.98 (2H, brs)](synthesized starting from 2,2-dimethyl-1,3-dioxolane-4-methanol and the compound prepared in Example 9 in the same manner as in Example 11) in 15 ml of EtOH was added 1.0 ml of c-HCl under ice cooling, and the mixture was stirred at room temperature for a day. The reaction mixture was concentrated under reduced pressure. To the residue was added water, and the mixture was extracted with EtOAc. The resulting organic layer was dried over anhydrous sodium sulfate, filtered, and evaporated under reduced pressure. The resulting residue was recrystallized from EtOAc to give 0.75 g of 2-amino-6-(2,3-dihydroxypropoxy)-4-phenylpyridine-3,5-dicarbonitrile.

### Example 41

To a solution of 5.0 g of malononitirle in 80 ml of diethyl ether was added 4.5 ml of EtOH and 19 ml of 4M HCl-EtOAc solution under ice cooling, and the mixture was stirred at 4°C overnight. The precipitated solid material was filtered to give 5.7 g of ethyl 2-cyanoacetimidate hydrochloride. This compound (3.5 g) was added together with 5.5 g of ammonium acetate to a solution of 1.0 ml of benzaldehyde in 15 ml of EtOH at room temperature, and the mixture was heated under reflux with stirring for a day. The reaction mixture was poured into ice water, and the precipitated crystals were collected by filtration, washed with EtOH, and dried under reduced pressure to give 0.67 g of 2,6-diamino-4-phenylpyridine-3,5-dicarbonitrile.

### Example 42

To a solution of 2.2 g of 2-amino-6-(1-benzylpiperidin-2-ylmethoxy)-4-(2-fluorophenyl)pyridine-3,5-dic arbonitrile (synthesized starting from (1-benzylpiperidin-2-yl)methanol and the compound prepared in Example 10 in the same manner as in Example 11) in 40 ml of MeOH was added 2.0 g of 10% Pd/C, and the mixture was stirred in hydrogen atmosphere at room temperature for 2 hours. The reaction mixture was filtered through celite, and the filtrate was concentrated and purified by silica gel column chromatography. Then, 2 ml of 4M HCl-EtOAc solution was added to the residue, and the resulting crude crystals were washed with EtOAc to give 224 mg of 2-amino-4-(2-fluorophenyl)-6-(piperidin-2-ylmethoxy)pyridine-3,5-dicarbonitri le monohydrochloride.

### Example 43

To a solution of 0.20 g of the compound of Example 357 in DMF was added 0.44 g of potassium fluoride at room temperature, and the mixture was stirred for 4 hours. The reaction mixture was poured into water and extracted with chloroform, and the organic layer was dried over anhydrous sodium sulfate and concentrated under reduced pressure. The residue was purified by silica gel column chromatography and recrystallized from EtOH to give 27 mg of 2-fluoro-6-methoxy-4-phenylpyridine-3,5-dicarbonitrile.

### Example 44

To a suspension of 0.50 g of the compound prepared in Example 8 in 100 ml dichloromethane was added 1.0 g of metachloroperbenzoic acid, and the mixture was stirred for 18 hours. The reaction mixture was washed with a saturated sodium hydrogencarbonate aqueous solution and extracted with chloroform. The organic layer was dried over anhydrous sodium sulfate, concentrated under reduced pressure, and the residue was recrystallized from EtOH to give 0.18 g of 2-amino-6-methanesulfonyl-4-thiophen-2-ylpyridine-3,5-dicarbonitrile.

### Example 45

To a solution of 700 mg of the compound prepared in Example 7 in DMF was added 102 mg of 60% NaH and then 353 mg of methanesulfonyl chloride, and the mixture was stirred at room temperature for 1 hour. Additional 102 mg of 60% NaH was added, and the mixture was stirred at room temperature for 1 hour. Water and 1M HCl aq. were then addedto the mixture, and the precipitated crystals were collected by filtration and purified by silica gel column chromatography to give 40 mg of N-(3,5-dicyano-6-methylsulfanyl-4-thiophen-3-ylpyridin-2-yl)methanesulfona mide.

### Example 46

To a solution of 600 mg of 2-amino-6-methoxy-4-thiophen-3-ylpyridine-3,5-dicarbonitrile [¹H-NMR (DMSO-*d*₆): δ 3.97 (3H, s), 7.37 (1H, dd), 7.77 (1H, dd), 8.02 (1H, dd)](synthesized starting from the compound prepared in Reference Example 4 in the same manner as in Example 1) in 4 g of ethylene glycol was added 128 mg of 60% NaH, and the mixture was stirred at 110°C for 20 minutes. The reaction mixture was poured into water, 4 ml of 1M HCl and chloroform, and the resulting precipitate was collected by filtration and recrystallized from EtOH to give 218 mg of 2-amino-6-(2-hydroxyethoxy)-4-thiophen-3-ylpyridine-3,5-dicarbonitrile.

### Example 47

The compound (0.20 g) of Example 357 was added to 3 ml of ethylenediamine and stirred at room temperature for 20 minutes. The reaction mixture was poured into water, and the precipitate was collected by filtration. This was dissolved in 3 ml of EtOH, to the solution was added 3 ml of 4M HCl-EtOAc solution, and the precipitated crystals were collected by filteration to give 75 mg of 2,6-bis(2-aminoethylamino)-4-phenylpyridine-3,5-dicarbonitrile dihydrochloride.

### Example 48

A solution of 500 mg of the compound of Example 144 and 551 mg of 1,1'-carbonyldiimidazole in 5 ml of DMF was stirred at 50°C for 1 hour. There was added a solution of 487 mg of guanidine hydrochloride and 196 mg of 60% NaH in 5 ml of DMF separately prepared, and the mixture was stirred at room temperature overnight. Water was added to the mixture, and the precipitated crystals were collected by filtration. The crystals were dissolved in EtOH, to the solution was added 2 ml of 4M HCl-EtOAc solution, and the precipitated crystals were collected by filtration and washed with EtOH to give 400 mg of N-[3'(2-amino-3,5-dicyano-6-methoxypyridin-4-yl)benzoyl]guanidine monohydrochloride.

### Example 49

To 10 ml of phosphorus oxychloride was added 250 mg of the compound of Example 145, and the mixture was stirred at 90°C for 2 hours. The reaction mixture was poured into ice water and the precipitated crystals were collected by filtration and washed with water and EtOH to give 210 mg of 2-amino-4-(3-cyanophenyl)-6-methoxypyridine-3,5-dicarbonitrile.

### Example 50

To a solution of 300 mg of the compound of Example 102 dissolved in 5 ml of acetic acid was added 180 mg of 2,5-dimethoxytetrahydrofuran, and the mixture was stirred at 60°C for 2 hours. After evaporation under reduced pressure, the residue was purified by silica gel column chromatography to give 89 mg of 2-amino-6-methoxy-4-[3-(1H-pyrrol-1-yl)phenyl]pyridine-3,5-dicarbonitrile.

### Example 51

To a solution of 500 mg of the compound of Example 334 dissolved in 15 ml of toluene was added 160 mg of hexane-2,5-dione and 20 mg of p-toluenesulfonic acid, and the mixture was heated under reflux for 2 hours. After cooling to room temperature, 1M NaOH was added to the mixture and extracted with EtOAc. The solvent was distilled off under reduced pressure and the residue was purified by silica gel column chromatography and recrystallization to give 150 mg of 2-amino-6-benzylsulfanyl-4-[3-(2,5-dimethyl-1H-pyrrol-1-yl)phenyl]pyridine-3 ,5-dicarbonitrile.

### Example 52

To a solution of 2.3 g of 60% NaH in 40 ml of THF was added 1.66 g of Ni(OAc)₂ and 2.2 ml of 3-hydroxy-1-methylpiperidine in order, and the mixture was stirred at 65°C for 2 hours. After cooling to room temperature, 0.50 g of the compound of Example 76 was added to the mixture, and the mixture was stirred at 65°C overnight. The reaction mixture was poured into ice water and the mixture was extracted with EtOAc. The organic layer was washed with a saturated sodium chloride aqueous solution, dried over anhydrous sodium sulfate, and filtered. The solvent was distilled off under reduced pressure, and the resulting residue was purified on silica gel column chromatography and recrystallized from EtOH to give 0.26 g of 2-amino-6-[(1-methylpiperidin-3-yl)oxy]-4-phenylpyridine-3,5-dicarbonitrile.

### Example 53

To a solution of 5.00 g of the compound of Example 126 dissolved in 100 ml of acetic acid was added 10 ml of c-HCl, and the mixture was stirred at 100°C for 3 hours. The solvent was distilled off under reduced pressure, water added to the residue, and the precipitated crystals were collected by filtration and washed with water and EtOH to give 4.28 g of 2-amino-4-(2,5-difluorophenyl)-6-hydroxypyridine-3,5-dicarbonitrile.

### Example 54

A solution of 2.00 g of the compound prepared in Example 53 dissolved in 30 ml of phosphorus oxychloride was stirred at 80°C for 24 hours. After evaporation of the solvent, ice water was added to the residue, and the precipitated crystals were collected by filtration. The crystals were washed with water and EtOH to give 2.13 g of 2-amino-6-chloro-4-(2,5-difluorophenyl)pyridine-3,5-dicarbonitrile.

### Example 55

To a solution of 0.20 g of the compound of Example 352 dissolved in 5.0 ml of acetic acid was added 0.20 g of iron (reduced), and the mixture was stirred at 50°C for 3 hours and then at room temperature overnight. The insoluble material was filtered off and the solvent was distilled off under reduced pressure to give the residue, which was purified by silica gel column chromatography to give 0.12 g of a compound. This compound was suspended in EtOH and allowed to react with 4M HCl-EtOAc to give 0.11 g of 2-amino-4-(3-aminophenyl)-6-[(pyridin-3-yl)sulfanyl]pyridine-3,5-dicarbonitril e dihydrochloride.

### Example 56

To a solution of 1.0 g of the compound prepared in Example 55 dissolved in 20 ml of dichloroethane was added 262 mg of formalin and 0.37 ml of acetic acid, and the mixture was stirred for 30 minutes. To the mixture was added 1.4 g of sodium triacetoxyborohydride, and after stirring for 1 hour water was added to the mixture and it was extracted with EtOAc. The organic layer was distilled off and the residue was purified by silica gel column chromatography. Then, 4M HCl-EtOAc solution was added, and the mixture was stirred for 30 minutes and evaporated, The residue was purified by recrystallization to give 79 mg of 2-amino-4-(3-dimethylaminophenyl)-6-[(pyridin-3-ylmethyl)sulfanyl]pyridine-3,5-dicarbonitrile dihydrochloride.

### Example 57

To a solution of 400 mg of 1-t-butoxycarbonyl-2,3-dihydroindole-6-carboxylic acid in THF was added CDI at room temperature. The mixture was warmed up to 50°C and then stirred for 10 minutes. After cooling to 0°C, 1 ml of water and 168 mg of sodium borohydride were added to the mixture and the mixture was stirred for 1 hour. Water was added to the mixture, and the mixture was extracted with ethyl acetate. The organic layer was dried over magnesium sulfate, filtered and concentrated under reduced pressure. The resulting crude product was purified by silica gel column chromatography to give 400 mg of 1-t-butoxycarbonyl-6-hydroxymethyl-2,3-dihydroindole (FAB-MS m/z: 249 (M⁺)).

This compound (400 mg) was dissolved in dichloromethane, to the solution was added 1.6 ml of triethylamine and 5 ml of DMSO. After cooling to 0°C, there was dropwise added a solution of 2.5 g of SO₃·Py in 5 ml of DMSO. After stirring for 30 minutes, the mixture was warmed up to room temperature. Water was added to the mixture, and the mixture was extracted with EtOAc. The organic layer was dried over magnesium sulfate, filtered and concentrated under reduced pressure. The resulting crude product was purified by silica gel column chromatography to give 80 mg of 1-t-butoxycarbonyl-6-formyl-2,3-dihydroindole (FAB-MS m/z: 248 (M⁺+1)).

This compound (80 mg) was dissolved in 5 ml of MeOH, to the solution was added 43 mg of malononitrile and 52 mg of sodium methoxide at 0°C, and the mixture was warmed up to room temperature and stirred for 12 hours. The reaction mixture was concentrated under reduced pressure, and the resulting crude product was dissolved in 5 ml of EtOH, to the solution was added c-HCl, and stirred under heating at 80°C for 1 hour. After cooling to room temperature, the mixture was concentrated under reduced pressure, and the residue was neutralized with 1M sodium hydroxide aqueous solution. The resulting crystals were collected by filtration and dissolved in 5 ml of EtOH, to the solution was then added 1 ml of 4M HCl-EtOAc solution. The reaction mixture was concentrated under reduced pressure, and the resulting crude crystals were washed with EtOH to give 40 mg of 2-amino-4-(2,3-dihydro-1H-indol-6-yl)-6-methoxypyridine-3,5-dicarbonitrile monohydrochloride.

### Examples 58 and 59

To a solution of 500 mg of the compound prepared in Example 3 in 10 ml of Py was added 5 ml of acetic anhydride and 25 mg of DMAP, and the mixture was stirred at room temperature overnight. The solvent was distilled off under reduced pressure, and water was added to the residue, and the mixture was extracted with EtOAc. The organic layer was washed with water and 1M HCl aq., dried over anhydrous MgSO₄, and filtered. The filtrate was concentrated under reduced pressure, and the residue was purified by silica gel column chromatography to give 2 kinds of oily products of which the Rf values on a thin layer chromatogram were different from each other. The respective products were crystallized from ether-EtOH to give 110 mg of N-[3,5-dicyano-4-(2-fluorophenyl)-6-methoxypyridin-2-yl]acetamide (Example 58) and 25 mg of 2-diacetylamino-4-(2-fluorophenyl)-6-methoxypyridine-3,5-dicarbonitrile (Example 59).

### Example 60

To a solution of 700 mg of the compound prepared in Example 3 in THF was added 125 mg of 60% NaH and then after stirring 562 mg of 2-methoxyacetyl chloride under ice cooling, and the mixture was stirred at room temperature overnight. Ice was added to the reaction mixture and it was extracted with EtOAc. The organic layer was concentrated under reduced pressure and the residue was purified by silica gel column chromatography. The resulting oily material was crystallized from ether to give 473 mg of N-[3,5-dicyano-4-(2-fluorophenyl)-6-methoxypyridin-2-yl]-2-methoxyacetamid e.

### Example 61

To a solution of 600 mg of the compound prepared in Example 12 in 10 ml of THF was added 90 mg of 60% NaH and then after stirring 267 mg of 2-methoxyacetyl chloride under ice cooling, and the mixture was stirred at room temperature for 1 hour. Then, additional 90 mg of 60% NaH was added and the mixture was stirred at room temperature overnight. To the mixture was added ice and 1M NaOH aq., and the mixture was stirred for 2 hours and extracted with EtOAc. The organic layer was washed with water and brine, dried over anhydrous MgSO₄, and filtered. The filtrate was concentrated under reduced pressure, and the residue was recrystallized from EtOH to give 213 mg of N-[3,5-dicyano-6-(2,2-difluoroethoxy)-4-(2-fluorophenyl)pyridin-2-yl]-2-metho xyacetamide.

### Example 62

To a solution of 532 mg of the compound of Example 127 in 10 ml of THF was added 89 mg of 60% NaH and then after stirring 242 mg of 2-methoxyacetyl chloride under ice cooling, and the mixture was stirred at room temperature for 1 hour. To the mixture was further added 89 mg of 60% NaH and 242 mg of 2-methoxyacetyl chloride, and the mixture was stirred at room temperature overnight. To the mixture was added ice and 1M NaOH aq., and the mixture was stirred for 8 hours and then acidified with 1M HCl aq. The reaction mixture was extracted with EtOAc. The organic layer was washed with water and brine, dried over anhydrous MgSO₄, and filtered. The filtrate was concentrated under reduced pressure, and the residue was purified by silica gel column chromatography. The resulting crystals were recrystallized from MeOH to give 243 mg of N-[3,5-dicyano-4-(2,6-difluorophenyl)-6-methoxypyridin-2-yl]-2-methoxyaceta mide.

### Example 63

To a solution of 532 mg of the compound of Example 127 in 10 ml of THF was added 89 mg of 60% NaH and then after stirring 175 mg of acetyl chloride under ice cooling, and the mixture was stirred at room temperature for 1 hour. Then, additional 89 mg of 60% NaH was added, and the mixture was stirred at room temperature for 1 hour. Ice was added to the mixture and it was extracted with EtOAc. The organic layer was concentrated under reduced pressure, and the residue was purified by silica gel column chromatography. The resulting oily product was crystallized from EtOH to give 78 mg of N-[3,5-dicyano-4-(2,6-difluorophenyl)-6-methoxypyridin-2-yl]acetamide.

### Example 64

To a solution of 480 mg of the compound prepared in Example 6 in 10 ml of THF was added 89 mg of 60% NaH and then after stirring 242 mg of 2-methoxyacetyl chloride under ice cooling, and the mixture was stirred at room temperature for 1 hour. To the mixture was further added 89 mg of 60% NaH and 242 mg of 2-methoxyacetyl chloride, and the mixture was stirred at room temperature overnight. To the mixture was added ice, and the mixture was extracted with EtOAc. The organic layer was washed with water and brine, dried over anhydrous MgSO₄, and filtered. The filtrate was concentrated under reduced pressure, and the residue was recrystallized from EtOH to give 180 mg of N-[3,5-dicyano-6-methoxy-4-(tetrahydropyran-2-yl)pyridin-2-yl]-2-methoxyac etamide.

### Example 65

To a solution of 5.0 g of 2-(3-bromophenyl)-1,3-dioxolane in 50 ml of toluene was added 4.2 g of 2-chloropropylamine hydrochloride, 500 mg of Pd₂(dba)₃, 500 mg of BINAP and 9.4 g of sodium t-butoxide, and the mixture was stirred at 80°C for 1 hour. Water was added to the mixture and the mixture was extracted with EtOAc. The organic layer was dried over anhydrous MgSO₄ and concentrated under reduced pressure, and the resulting residue was purified by silica gel column chromatography to give 860 mg of 1-(3-[1,3]dioxolan-2-ylphenyl)azetidine (FAB-MS m/z: 206 (M⁺+1)).

This compound (860 mg) was dissolved in 10 ml of acetic acid, to the solution was added 330 mg of malonodinitrile and 0.5 ml of piperidine at room temperature, and the mixture was warmed up to 50°C and stirred for 12 hours. Water was added to the mixture, and the mixture was extracted with EtOAc. The organic layer was dried over anhydrous MgSO₄ and concentrated under reduced pressure, and the resulting crude product was purified by silica gel column chromatography to give 130 mg of 2-(3-azetidin-1-ylbenzylidene)malononitrile (FAB-MS m/z: 210 (M⁺+1)).

This compound (130 mg) was dissolved in 5 ml of MeOH, to the solution was added 41 mg of malononitrile and 67 mg of sodium methoxide, and the mixture was stirred for 12 hours. Water was added to the mixture, and the mixture was extracted with EtOAc. The organic layer was dried over anhydrous MgSO₄ and concentrated under reduced pressure, and the resulting crude product was purified by silica gel column chromatography. The crude crystals were dissolved in EtOH, to the solution was then added 1 ml of 4M HCl-EtOAc solution. The mixture was concentrated under reduced pressure, and the resulting crude crystals were recrystallized from EtOH to give 11 mg of 2-amino-4-[3-(3-chloropropylamino)phenyl]-6-methoxypyridine-3,5-dicarbonit rile monohydrochloride.

### Example 66

To a solution of 1.1 g of 1-triisopropylsilyl-1H-pyrrole-3-carbaldehyde in 20 ml of MeOH was added 580 mg of malononitrile and 700 mg of sodium methoxide at 0°C, and the mixture was warmed up to room temperature and stirred for 12 hours. Water was added to the mixture, and the mixture was extracted with EtOAc. The organic layer was dried over anhydrous MgSO₄ and concentrated under reduced pressure, and the resulting crude product was purified by silica gel column chromatography. The resulting crystals were recrystallized from EtOH to give 150 mg of 2-amino-6-methoxy-4-(1H-pyrrol-3-yl)pyridine-3,5-dicarbonitrile.

### Example 67

To a solution of 1.0 g of 2-aminothiazole-5-carbaldehyde in 20 ml of THF was added 2.5 g of DIBOC and 1.4 g of DMAP at room temperature, and the mixture was stirred for 12 hours. Water was added to the mixture, and the mixture was extracted with EtOAc. The organic layer was dried over anhydrous MgSO₄ and concentrated under reduced pressure, and the resulting crude product was purified by silica gel column chromatography to give 600 mg of t-butyl (5-formylthiazole-2-yl)carbamate (FAB-MS m/z: 229 (M⁺+1)).

This compound (600 mg) was dissolved in 15 ml of MeOH, to the solution was added 350 mg of malononitrile and 420 mg of sodium methoxide at 0°, and the mixture was warmed up to room temperature and stirred for 100 hours. Water was added to the mixture, and the mixture was extracted with EtOAc. The organic layer was dried over anhydrous MgSO₄ and concentrated under reduced pressure, and the resulting crude product was purified by silica gel column chromatography. The resulting crystals were recrystallized from EtOH to give 290 mg of t-butyl [5-(2-amino-3,5-dicyano-6-methoxypyridin-4-yl)thiazol-2-yl]carbamate (FAB-MS m/z: 373 (M⁺+1)).

This compound (290 mg) was dissolved in 5 ml of MeOH, to the solution was added 1 ml of 4M HCl-EtOAc solution at room temperature. The reaction mixture was concentrated under reduce pressure, and the resulting crude crystals were washed with EtOH to give 110 mg of 2-amino-4-(2-aminothiazol-5-yl)-6-methoxypyridine-3,5-dicarbonitrile monohydrochloride.

### Example 68

To a solution of 17.7 g of methyl 2-cyano-3-(2-fluorophenyl)acrylate in 20 ml of MeOH was added 11.1 g of sodium methoxide and 6.79 g of malononitrile, and the mixture was stirred at room temperature overnight and then under reflux for 3 hours. The solvent was distilled off under reduce pressure, 1M HCl aq. was added to the residue, and the precipitated crystals were collected by filtration and washed with water and EtOH to give 8.46 g of 4-(2-fluorophenyl)-2-hydroxy-6-methoxypyridine-3,5-dicarbonitrile (FAB-MS m/z: 270 (M⁺+1)).

This compound (500 mg) was dissolved in 10 ml of dichloroethane, to the solution was added 425 mg of tosyl chloride, 0.33 ml of triethylamine and 50 mg of DMAP, and the mixture was stirred at room temperature for 3 hours. To the mixture was added 340 mg of 2-aminoethanol, and the mixture was stirred for 1 hour. Then, 1M HCl aq. was added to the reaction mixture, and the precipitated crystals were collected by filtration. The crystals were purified by silica gel column chromatography. The resulting oily material was crystallized from ether to give 138 mg of 4-(2-fluorophenyl)-2-[(2-hydroxyethyl)amino]-6-methoxypyridine-3,5-dicarbon itrile.

### Example 69

To a solution of 268 mg of the compound prepared in Example 3 in 5 ml of THF was added 0.14 ml of triethylamine at room temperature. After addition of 0.14 ml of trifluoroacetic anhydride at 0°C, the mixture was warmed up to room temperature and stirred for 20 hours. Additional 0.07 ml of triethylamine and 0.07 ml trifluoroacetic anhuydride were added, and the mixture was warmed up to 50°C and stirred for 5 hours. After cooling to room temperature, water was added to the mixture, and the mixture was extracted with chloroform. The organic layer was washed with water and then with brine, dried over anhydrous sodium sulfate and evaporated under reduced pressure. The resulting residue was washed with hexane to give 300 mg of N-[3,5-dicyano-4-(2-fluorophenyl)-6-methoxypyridin-2-yl]-2,2,2-trifluoroaceta mide.

### Example 70

To a solution of 500 mg of the compound prepared in Example 3 in 10 ml of THF was added 86 mg of 60% NaH and then after stirring 234 mg of cyclopropanecarbonyl chloride under ice cooling, and the mixture was stirred at room temperature for 1 hour. To the mixture was added additional 86 mg of 60% NaH and the mixture was stirred at room temperature for 1 hour. Ice was added, and the mixture was extracted with EtOAc. The organic layer was washed with brine, dried over anhydrous MgSO₄, and filtered. The filtrate was concentrated under reduced pressure, and the residue was recrystallized from ethanol to give 260 mg of N-[3,5-dicyano-4-(2-fluorophenyl)-6-methoxypyridin-2-yl]cyclopropanecarboxa mide.

### Example 71

To a solution of 463 mg of the compound prepared in Example 74 in 6 ml of THF was added 98 mg of dimethylamine hydrochloride at room temperature. Triethylamine (0.50 ml) was added to the mixture at 0°C, then warmed up to room temperature and stirred for 12 hours. Additional 0.38 ml of triethylamine and 74 mg of dimethylamine hydrochloride were added, and the mixture was stirred at room temperature for 2.5 hours. The precipitated crystals were collected by filtration, and the filtrate was concentrated under reduce pressure and purified by silica gel column chromatography to give 139 mg of 3-[3,5-dicyano-4-(2-fluorophenyl)-6-methoxypyridin-2-yl]-1,1-dimethylurea.

### Example 72

To a solution of 500 mg of the compound prepared in Example 3 in 10 ml of THF was added 84 mg of 60% NaH at 0°C, and the mixture was stirred at the same temperature for 20 minutes. At 0°C, to the mixture was added 0.27 ml of methyl 3-chlorocarbonyl-propionate, and the mixture was warmed up to room temperature and stirred for 1 hour. Again, 84 mg of 60% NaH was added to the mixture at 0°C, and the mixture was warmed up to room temperature, and stirred for 30 minutes. Then, 0.27 ml of methyl 3-chlorocarbonyl-propionate was added and the mixture was stirred for 1 hour. Water was added to the mixture, and the mixture was extracted with EtOAc. The organic layer was washed with brine, dried over anhydrous MgSO₄ and concentrated under reduced pressure. The resulting crude product was purified by silica gel column chromatography (hexane-ethyl acetate) and recrystallized from diethyl ether to give 738 mg of 2-[bis(3-methoxycarbonylpropanoyl)amino]-4-(fluorophenyl)-6-methoxypyridi ne-3,5-dicarbonitrile.

### Example 73

To a solution of 700 mg of the compound prepared in Example 3 in 10 ml of THF was added 483 mg of propionyl chloride and 1.1 ml of triethylamine under ice cooling, and the mixture was stirred at room temperature overnight. To the mixture was added 483 mg of propionyl chloride and 1.1 ml of triethylamine, and the mixture was stirred at room temperature for 3 hours. Then, additional 242 mg of propionyl chloride and 0.55 ml of triethylamine, and the mixture was stirred for 2 hours. Ice was added to the mixture. The mixture was acidified with 1M HCl aq., and extracted with EtOAc. The organic layer was concentrated under reduced pressure, and the residue was purified by silica gel column chromatography. The resulting oily material was crystallized from ether to give 403 mg of 2-dipropanoylamino-4-(2-fluorophenyl)-6-methoxypyridine-3,5-dicarbonitrile.

### Example 74

To a solution of 5 g of the compound prepared in Example 3 in 93 ml of THF was added 5.16 ml of triethylamine. To the mixture was added 4.70 ml of phenyl chloroformate at 0°C, and the mixture was warmed up to room temperature and stirred at room temperature for 2.5 hours. The precipitated crystals were collected by filtration, and the filtrate was concentrated under reduce pressure and purified by silica gel column chromatography (hexane-ethyl acetate) to give 6.323 g of 2-[bis(phenoxycarbonyl)amino]-4-(2-fluorophenyl)-6-methoxypyridine-3,5-dica rbonitrile.

The structure and physical properties of the compounds prepared in Reference Examples and Working Examples are shown in Tables 1 to 10. The symbols in the tables have the following meanings.
Rf: Reference Example number
Ex: Example number
Data: Physical Data (MS: FAB-MS(M+H)⁺; MN: FAB-MS(M-H)⁻;
NMR: δ (ppm) of the peak in ¹H-NMR in DMSO-*d*₆ as a solvent for measurement unless otherwise indicated using (CH₃)₄Si as internal reference
mp: melting point)
Salt: HCl: hydrochloride; Ox: oxalate; H₂O: hydrate;
EtOH: ethanol solvate; no indication: free
Syn: Process for production (the numeral indicates the Example number corresponding to the production)
R, R¹, R², R³: the substituent in the general formulae
Me: methyl; Et: ethyl; iPr: isopropyl; cPr; cyclopropyl; tBu: tertiary butyl; cPen: cyclopentyl; cHex: cyclohexyl; cHep: cycloheptyl; Ph: phenyl; Bn: benzyl; Pn: pyridylmethyl; The: thienyl: Py: pyridyl; Mor: morpholin-4-yl; Ac: acetyl; Bz: benzoyl; Boc: t-butyloxycarbonyl. The numeral prefixed to the substituent indicates the position of the substituent; for example, 2,5-diF-Ph means 2,5-difluorophenyl; 3-BocNHCH₂-Bn, 3-(t-butyloxycarbonylaminomethyl) phenylmethyl; 2-Me-3-PnS, 2-methylpyridin-3-ylmethylsulfanyl; and 5-CO₂H-2-The, 5-carboxylthiophen-2-yl, respectively.

**Table 1**

| Rf | Compound Name | Data |
|---|---|---|
| 1 | 2-benylidenemalononitrile | NMR(CDCl₃):7.30-7.70(2H,m),7.78(1H,s), 7.70-8.10(3H,m). |
| 2 | 2-(2-fluorobenzylidene)malononi trile | NMR:7.51-7.73(2H,m),7.72-7.80(1H,m),8. 07(1H,t),8.60(1H,s). |
| 3 | 2-(thiophen-2-ylmethylidene)ma lononitrile | NMR(CDCl₃):7.27(1H,dd),7.70-8.00(3H,m ). |
| 4 | 2-(thiophen-3-ylmethylidene)ma lononitrile | NMR(CDCl₃):7.50(1H,dd),7.76(1H,s),7.81 (1H,d),8.18(1H,dd). |
| 5 | 2-[(4-(t-butoxycarbonylaminome thyl)benzylidene) malononitrile | NMR(CDCl₃):1.46(9H,s),4.39(2H,d),4.90(1H,brs),7.45(2H,d),7.79(2H,d),7.93(1H,s). |
| 6 | 2-[(3-(t-butoxycarbonylaminome thyl)benzylidene) malononitrile | NMR:1.39(9H,s),4.18(2H,d),7.48(1H,dd),7 .56(1H,s),7.59(1H,d),7.79(1H,s),7.89(1H,d ),8.55(1H,s). |
| 7 | 2-(4-dimethylaminobenzylidene) malononitrile | NMR:3.10(6H,s),6.85(2H,d),7.84(2H,d),8. 05(1H,s). |
| 8 | t-butyl 4-formylbenzylcarbamate | NMR(CDCl₃):1.47(9H,s),4.38(2H,d),4.80(1H,brs),7.45(2H,d),7.85(2H,d),10.0(1H,s). |
| 9 | t-butyl 3-formylbenzylcarbamate | NMR(CDCl₃):1.47(9H,s),4.38(2H,d),4.80(1H,brs),7.45-7.60(2H,m),7.65-7.85(2H,m) ,10.0(1H,s). |
| 10 | 2-amino-6-chloropyridine-3,5-di carbonitrile | NMR:8.36(2H,brs),8.55(1H,s). |
| 11 | t-butyl 4-hydroxybenzylcarbamate | NMR(CDCl₃):1.46(9H,s),4.21(2H,d),4.75(1H,brs),5.08(1H,s),6.77(2H,d),7.14(2H,d). |
| 12 | t-butyl 3-hydroxybenzylcarbamate 3-hydroxybenzylcarbamate | NMR(CDCl₃):1.46(9H,s),4.25(2H,d),4.75(1H,brs),5.05(1H,s),6.60-6-90(3H,m),7.05-7.25(1H,m). |
| 13 | t-butyl 2-hydroxybenzylcarbamate | NMR(CDCl₃):1.44(9H,s),4.14(2H,d),5.20(1H,s),6.65-7.40(4H,m),8.77(1H,s). |
| 14 | 3-(1H-imidazol-2-yl)benzaldehy de | NMR:7.01-8.47(7H,m),10.07(1H,s). |
| 15 | 2-[3-(2-hydroxyethoxy)benzylide ne]malononitrile | NMR(CDCl₃):1.97(1H,t),3.98-4.03(2H,m), 4.15-4.18(2H,m),7.20-7.61(4H,m),9.98(1H ,s). |

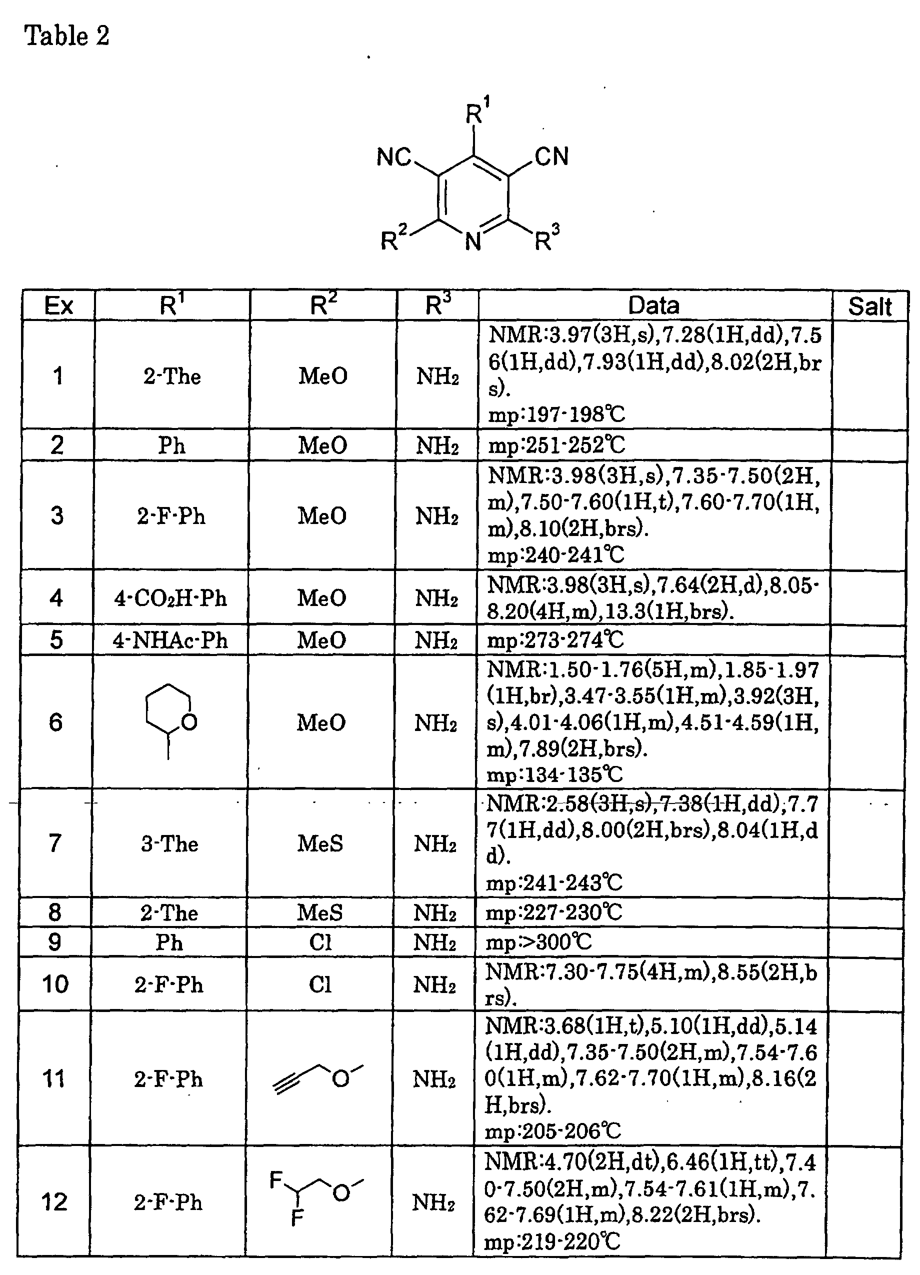

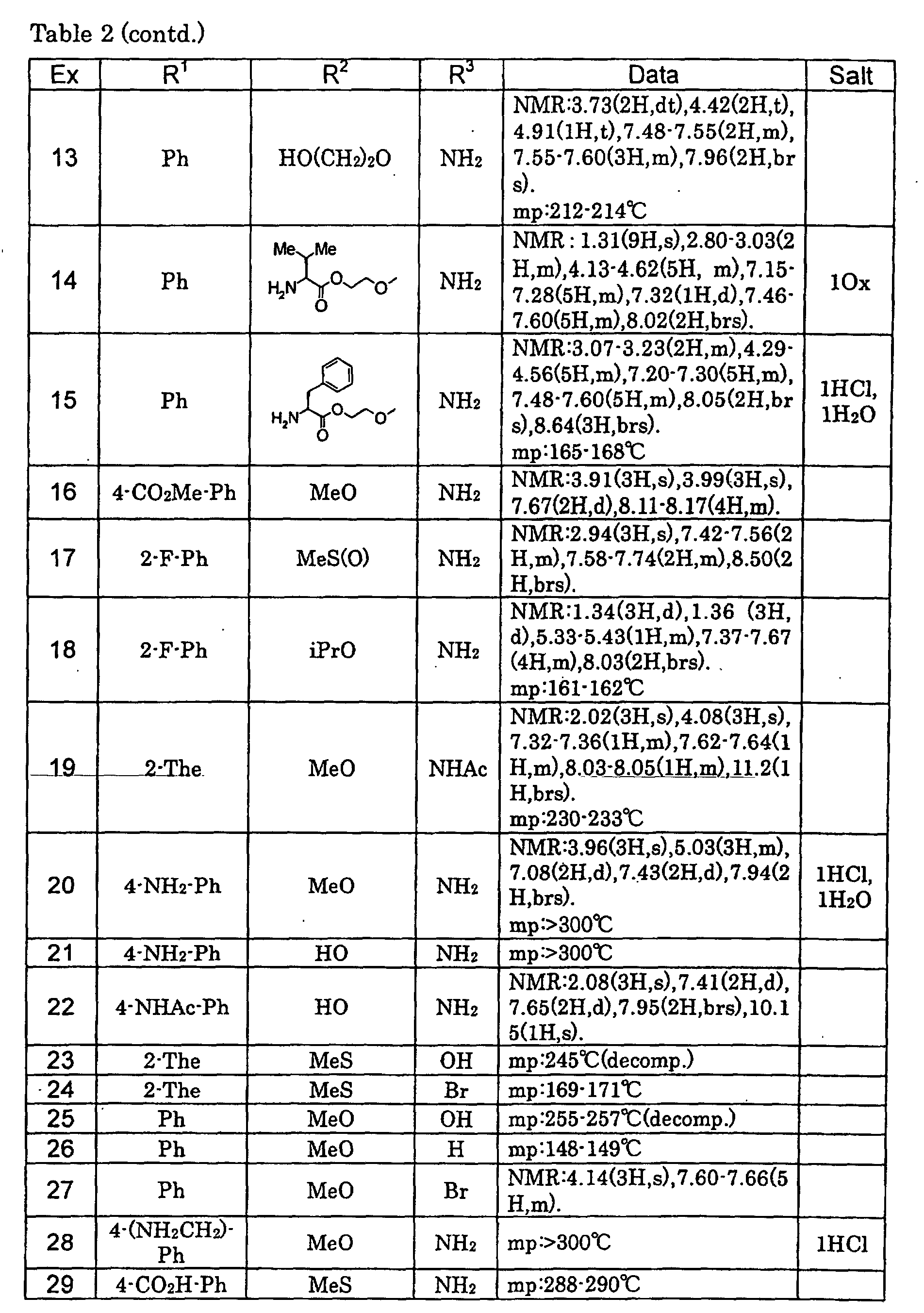

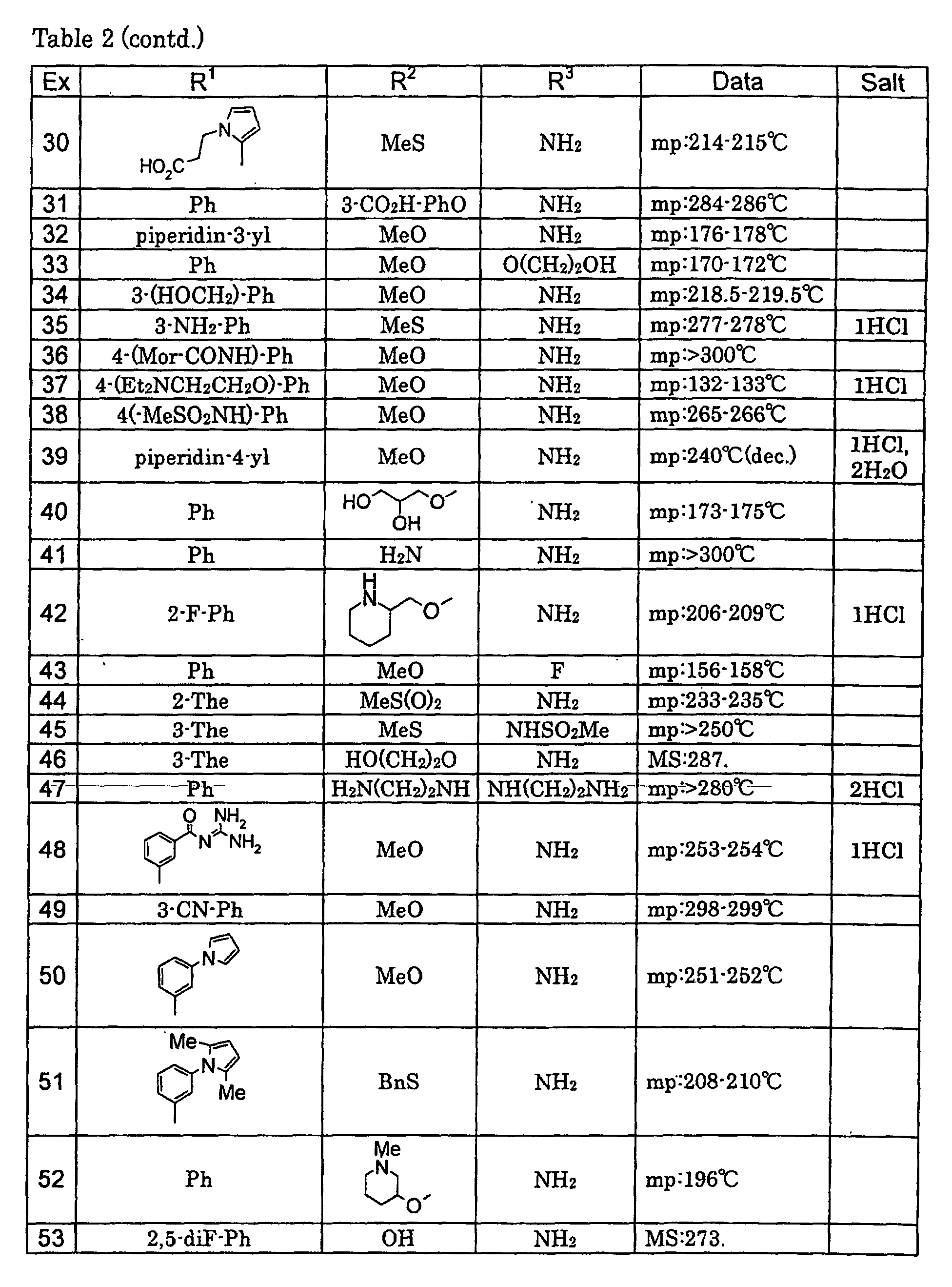

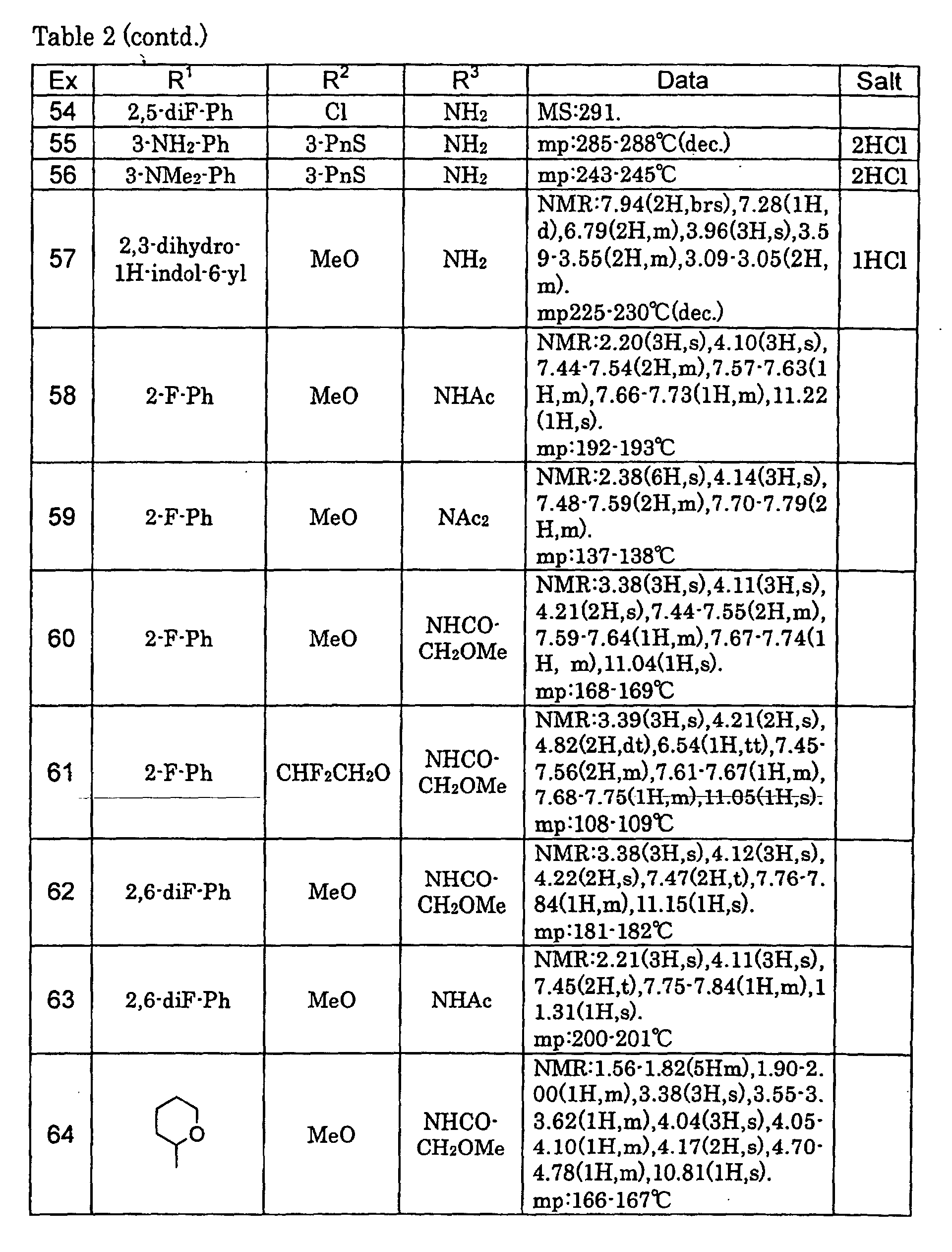

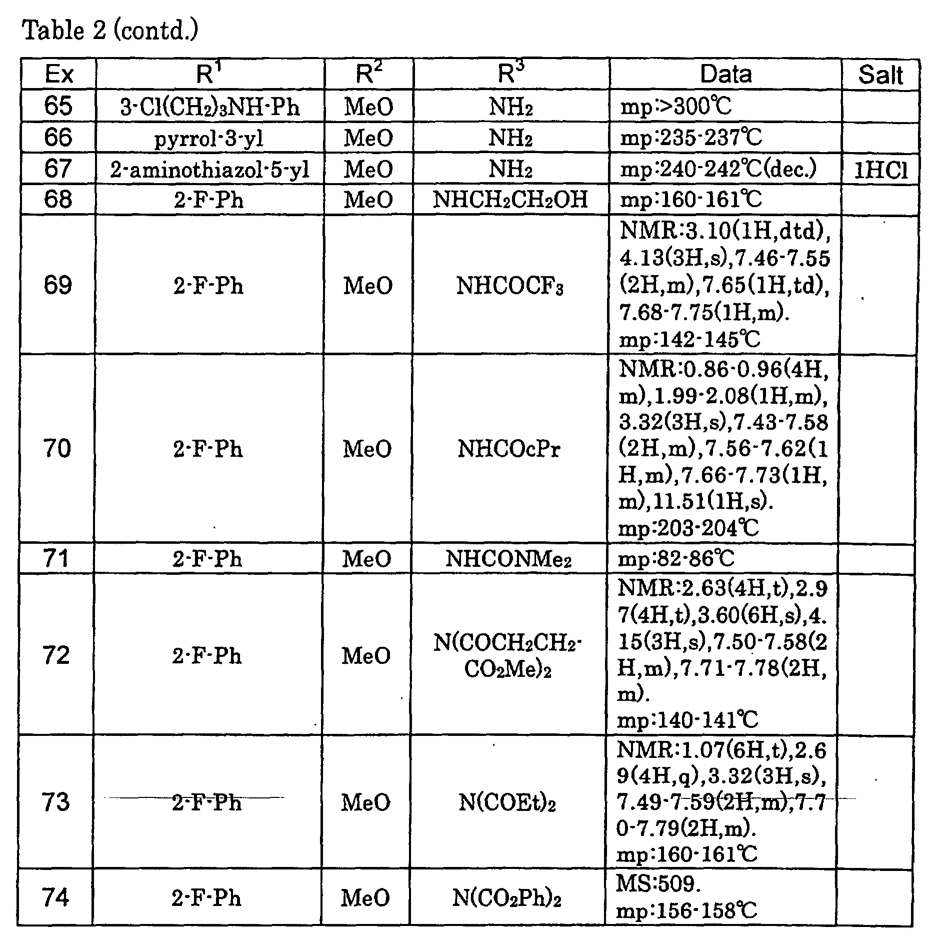

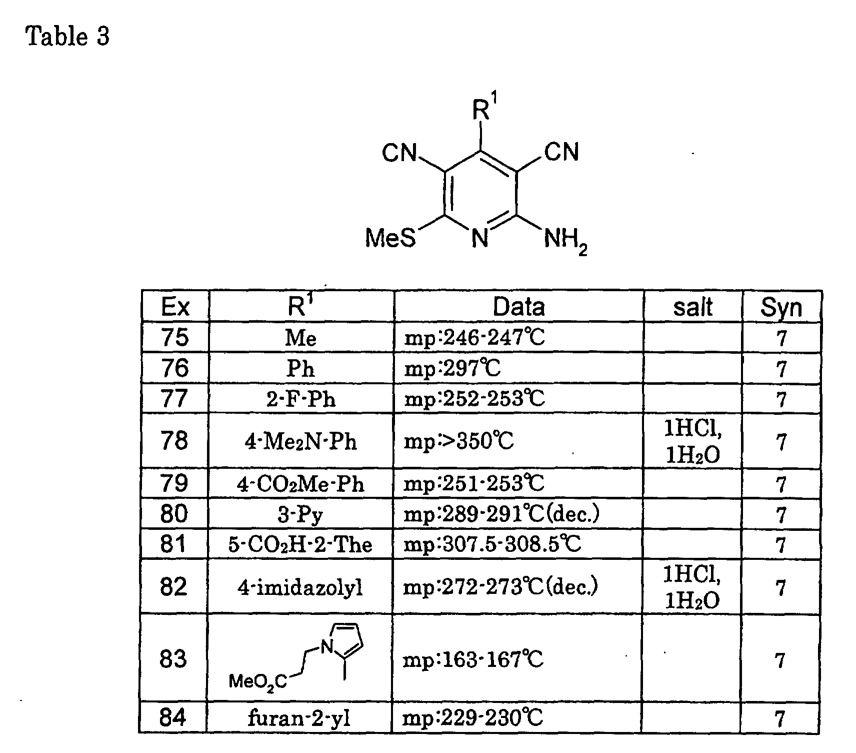

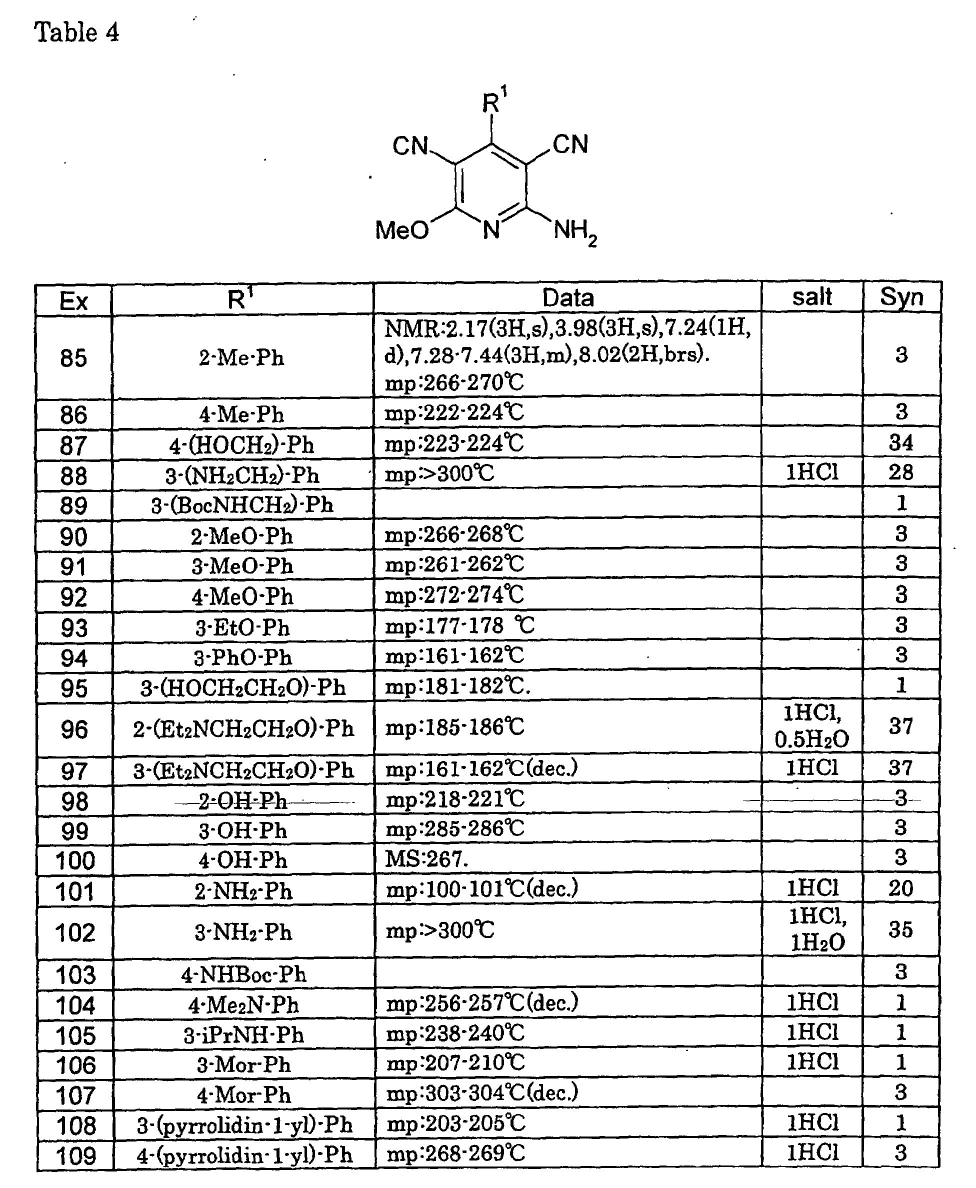

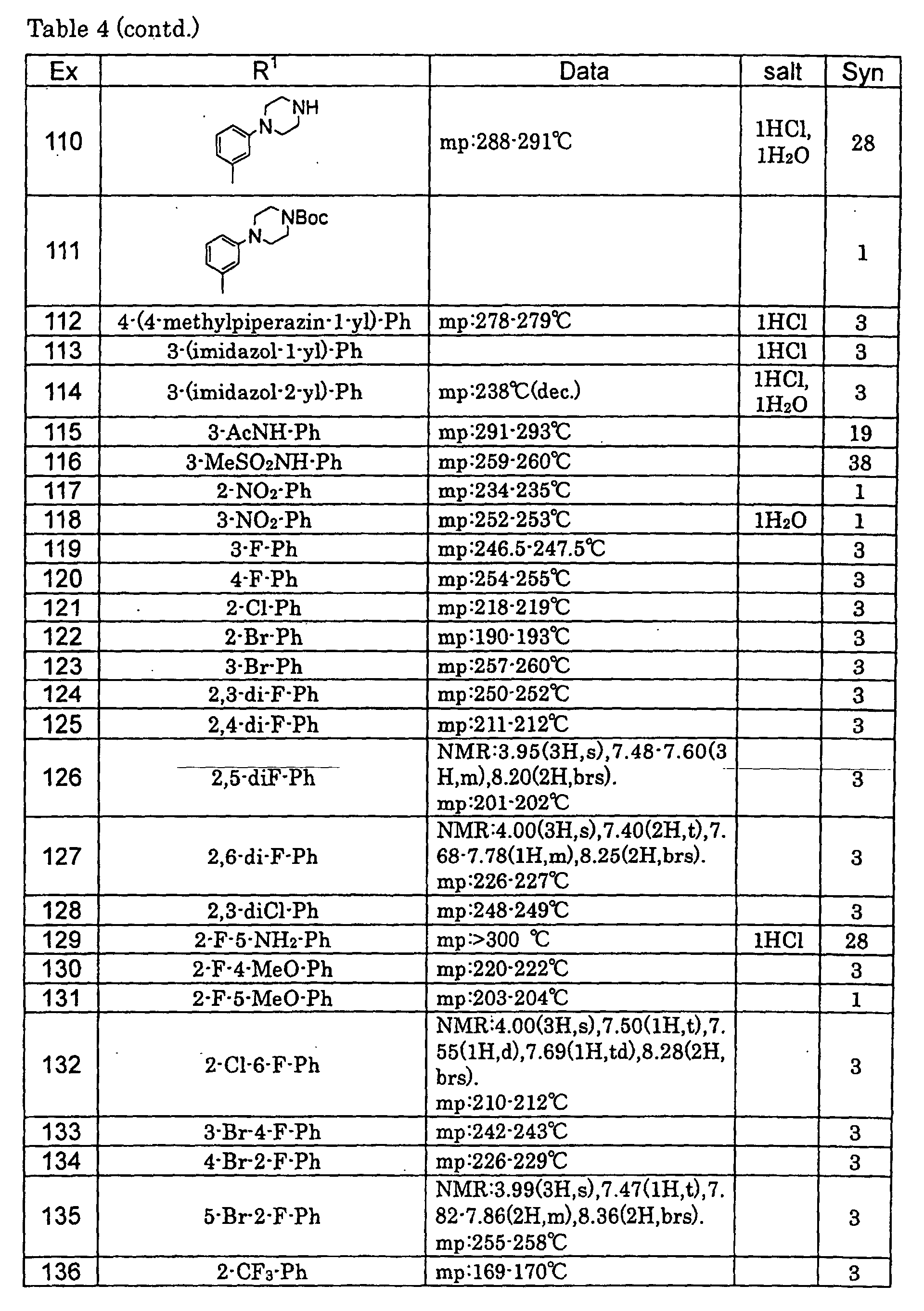

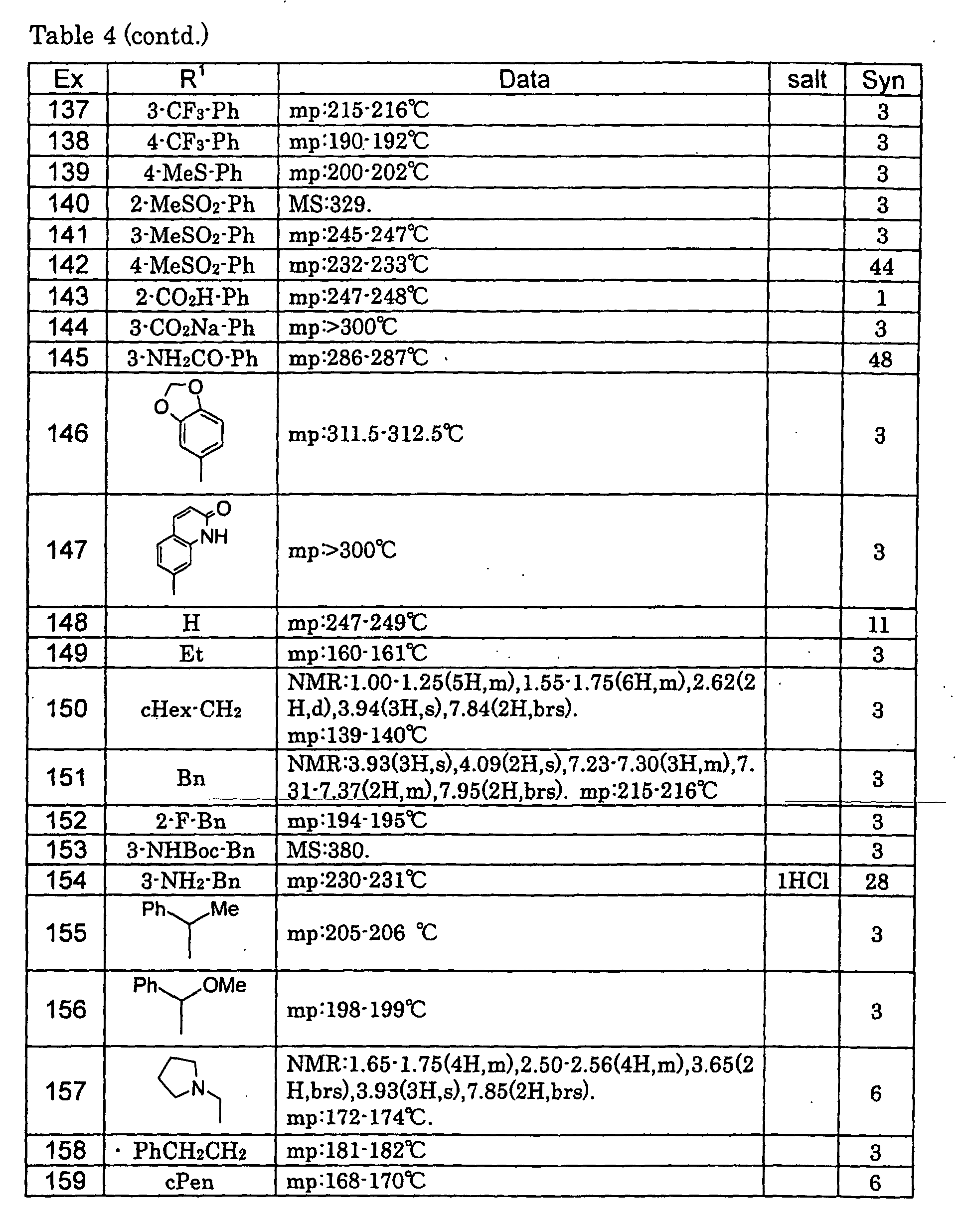

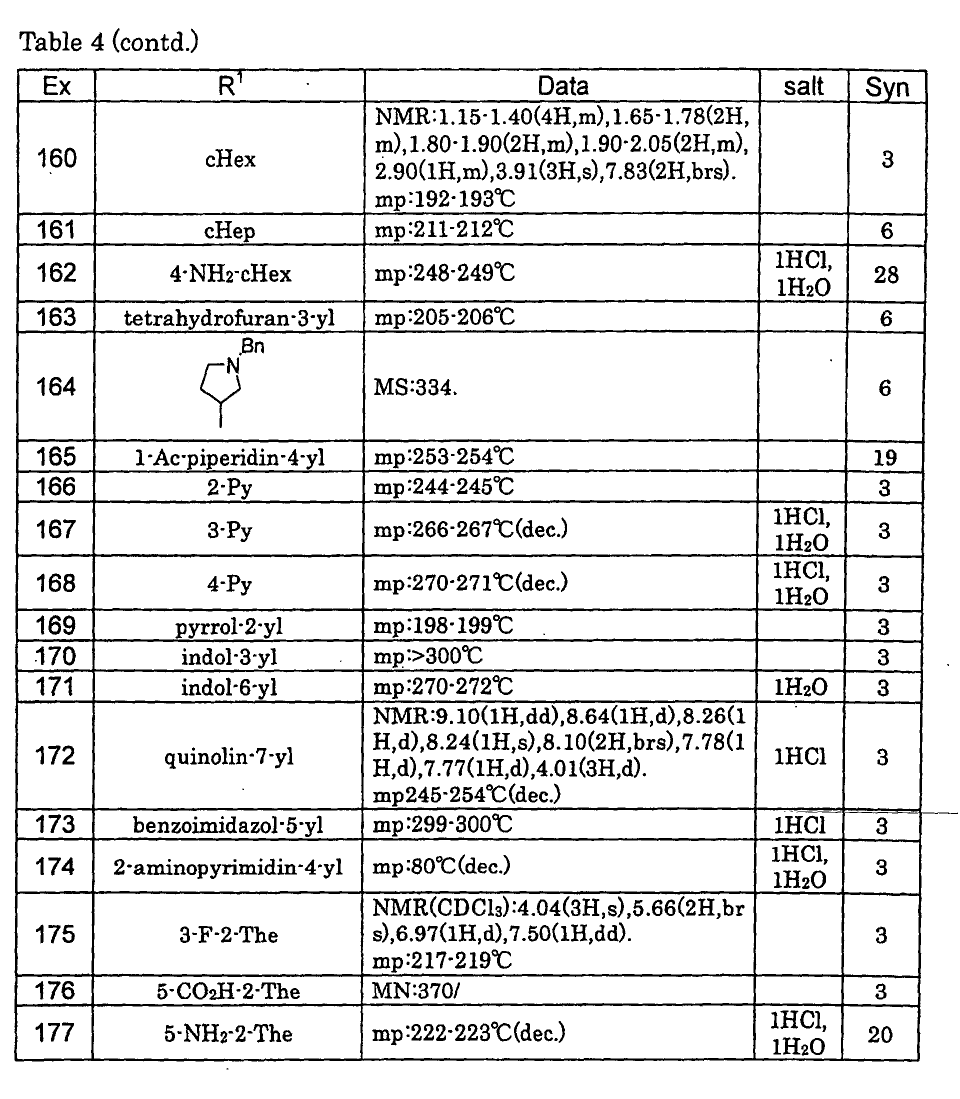

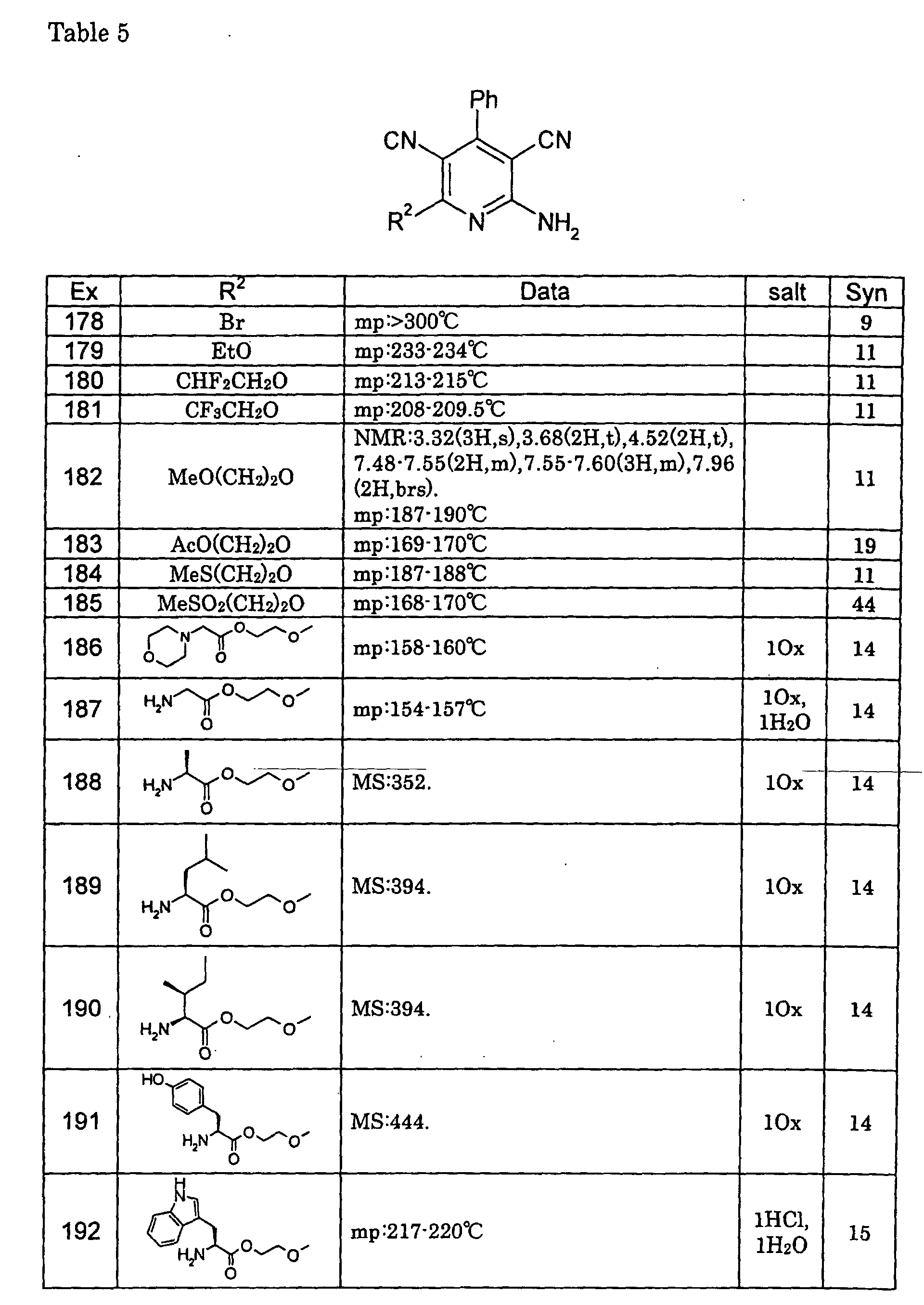

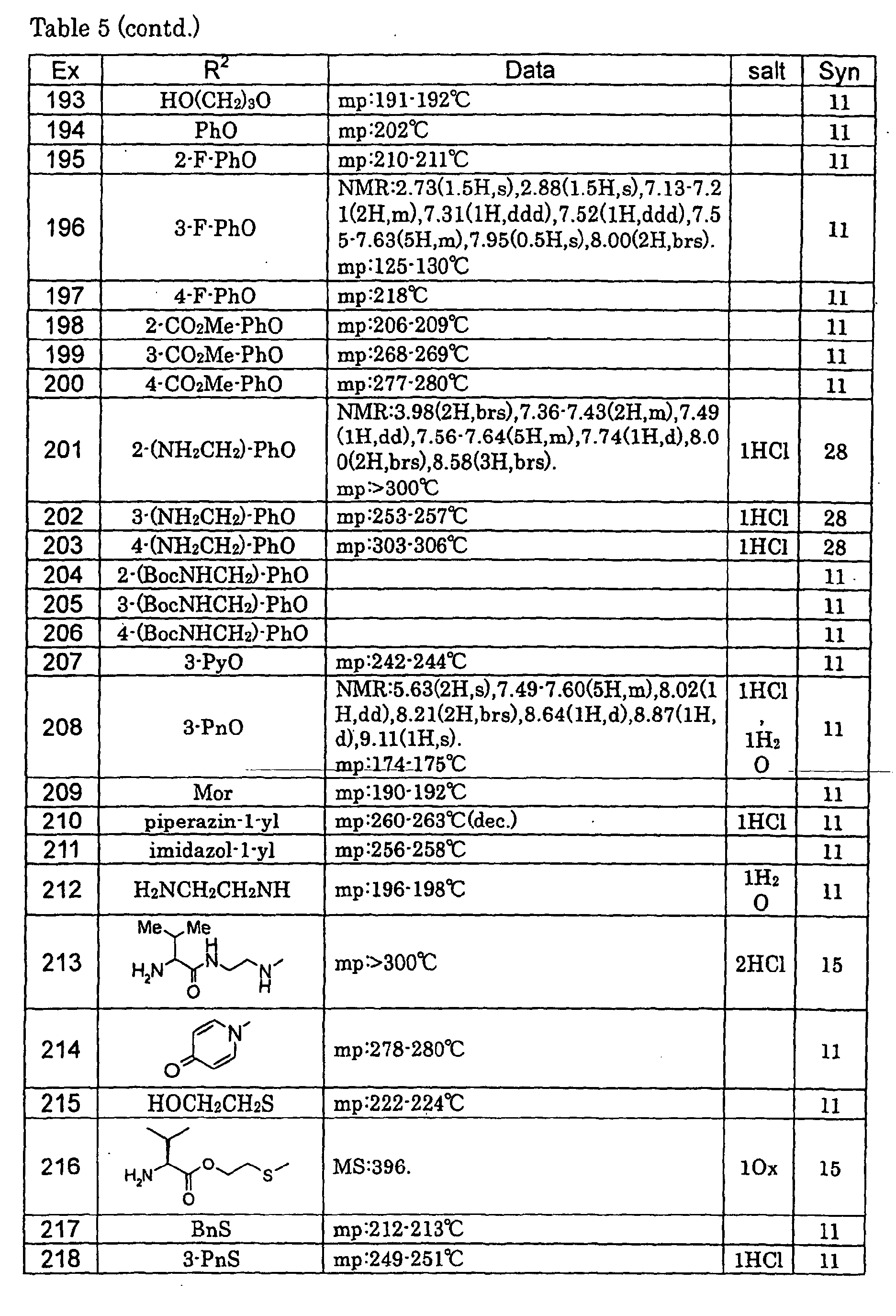

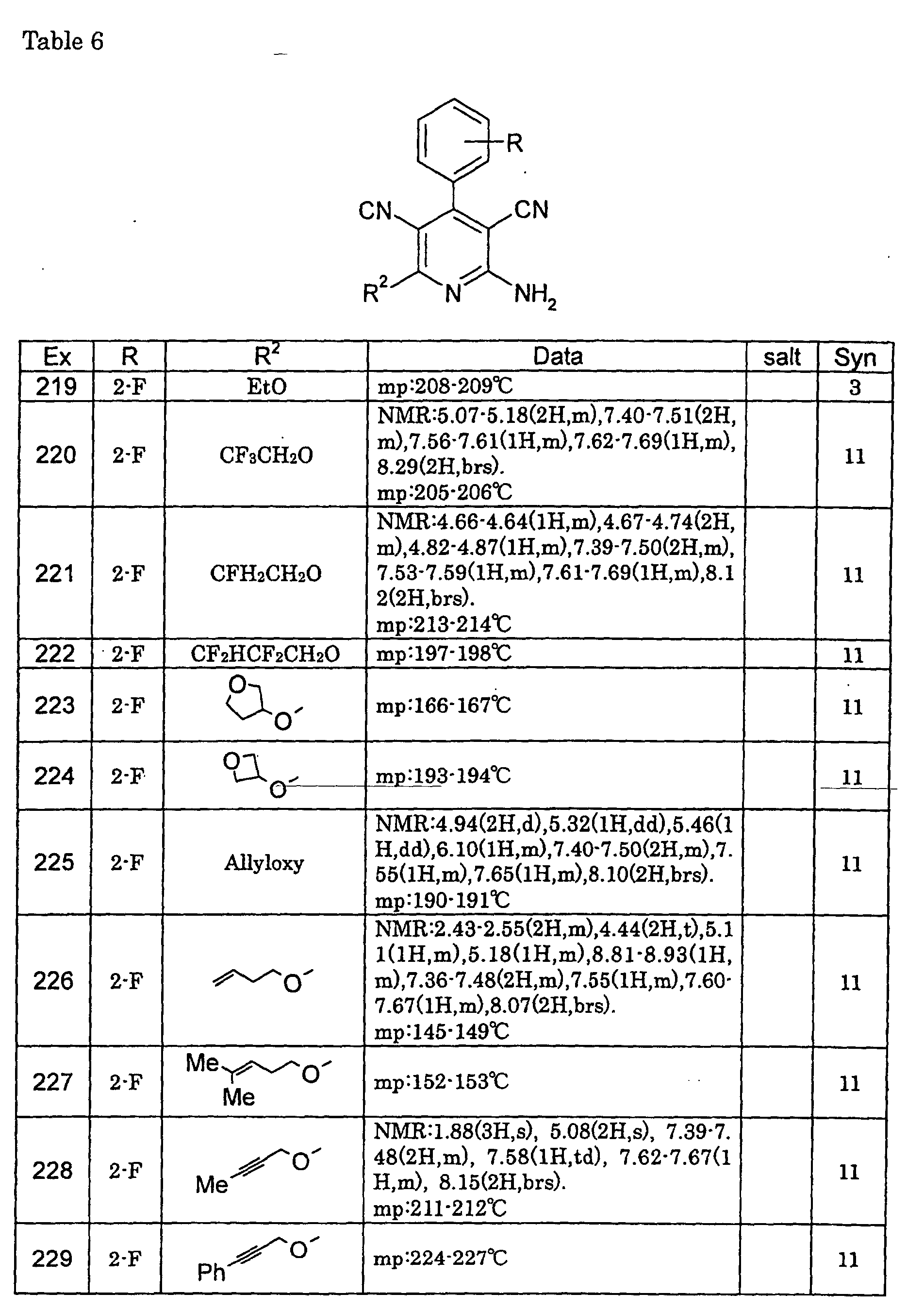

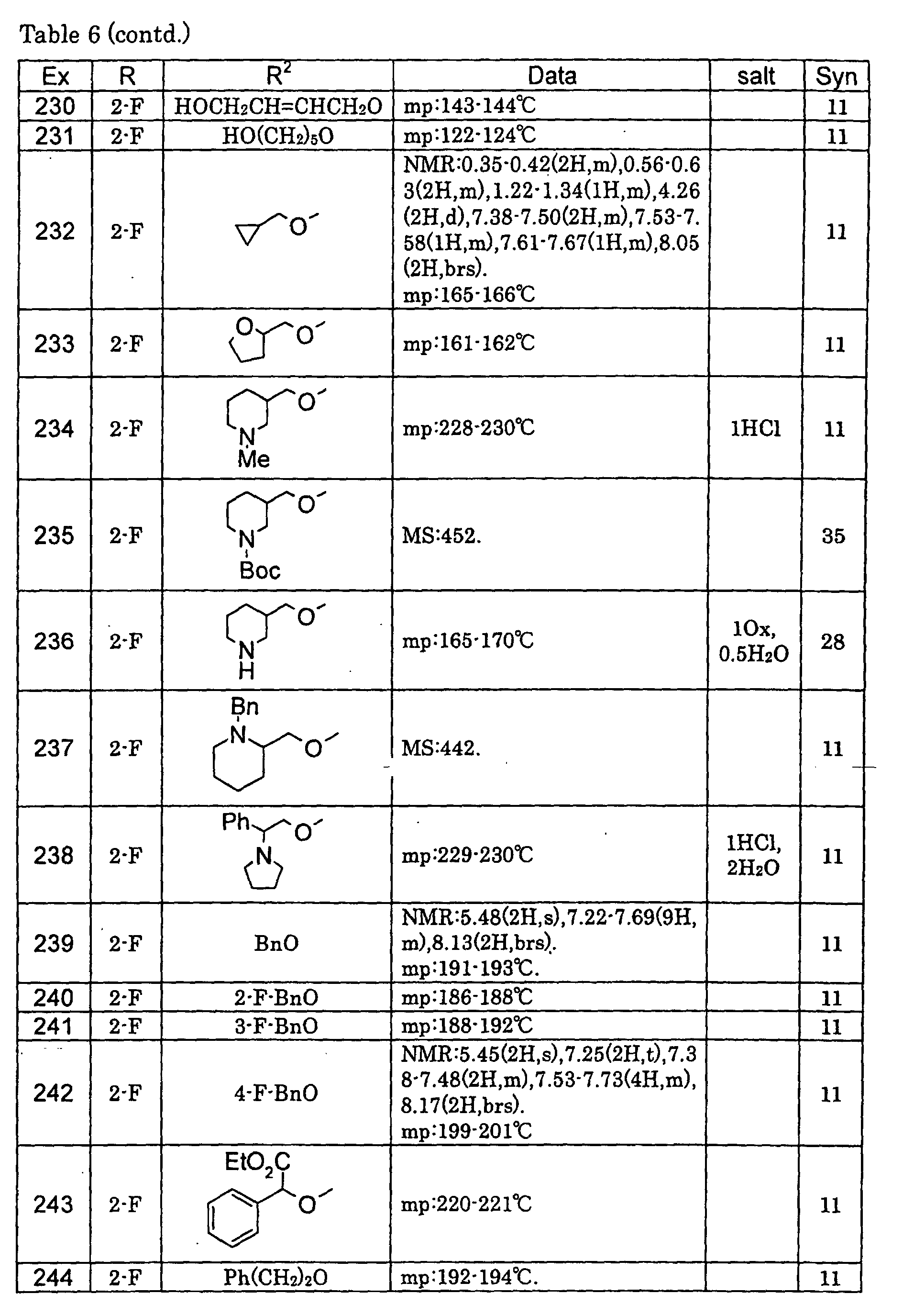

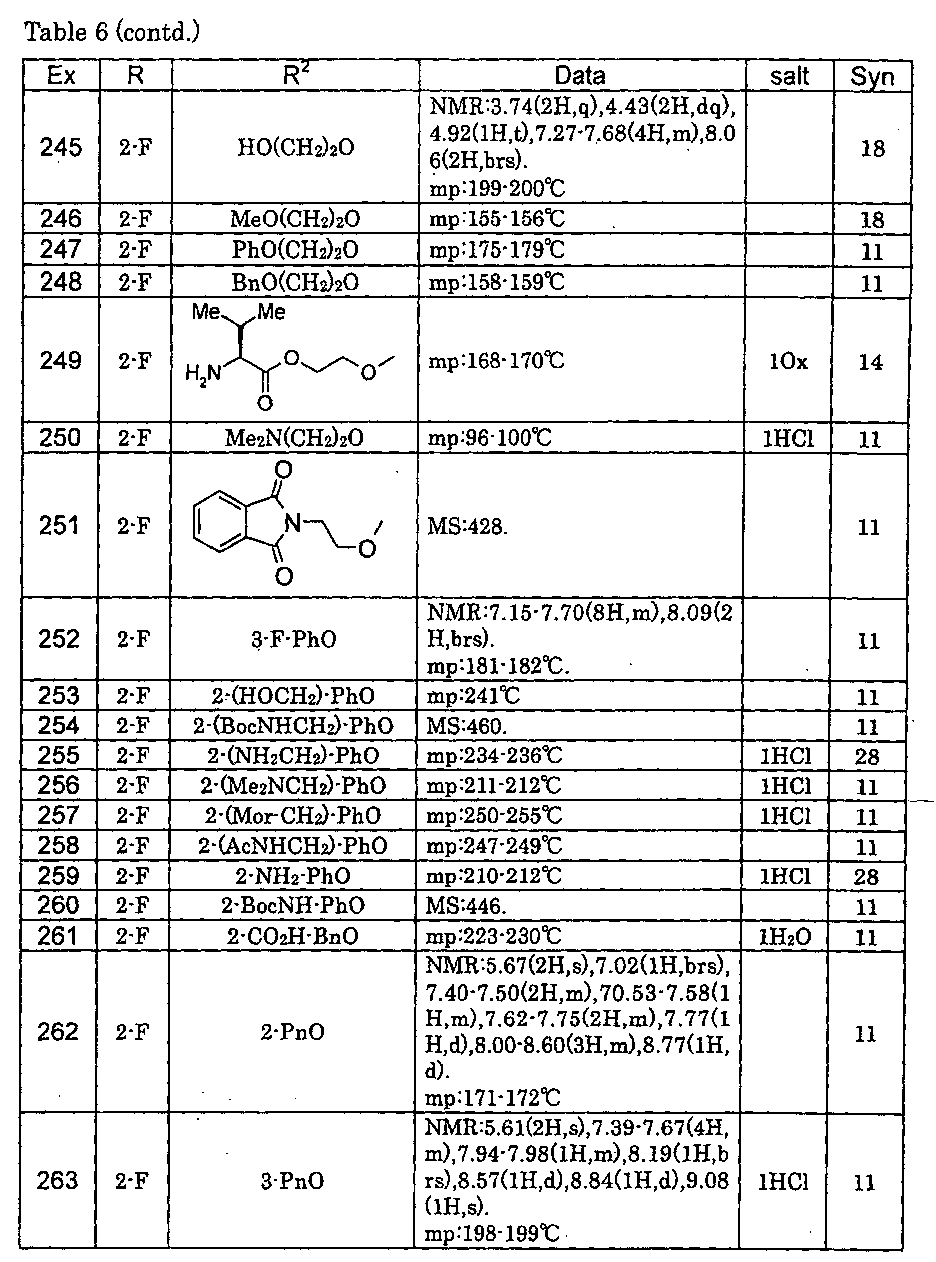

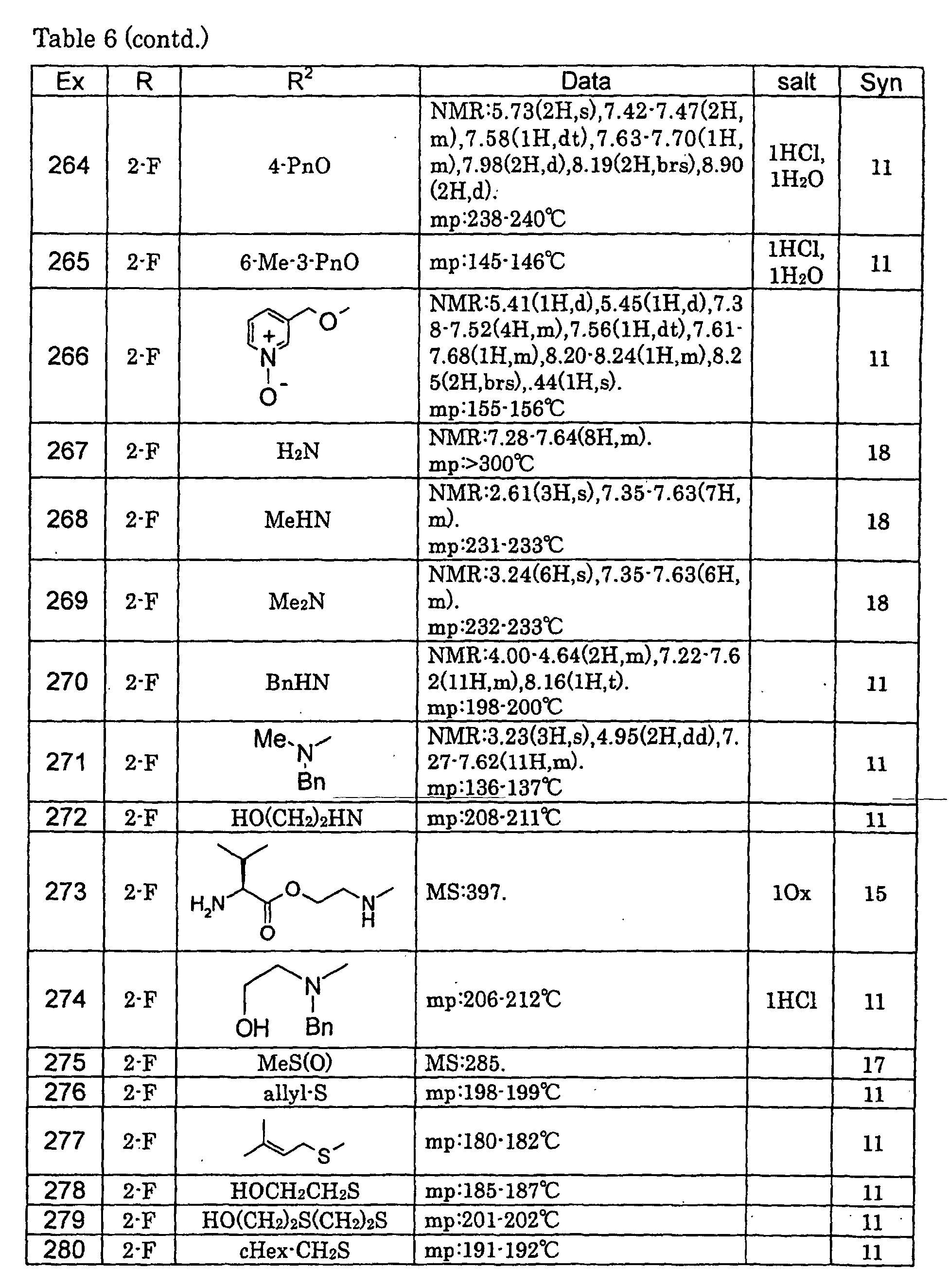

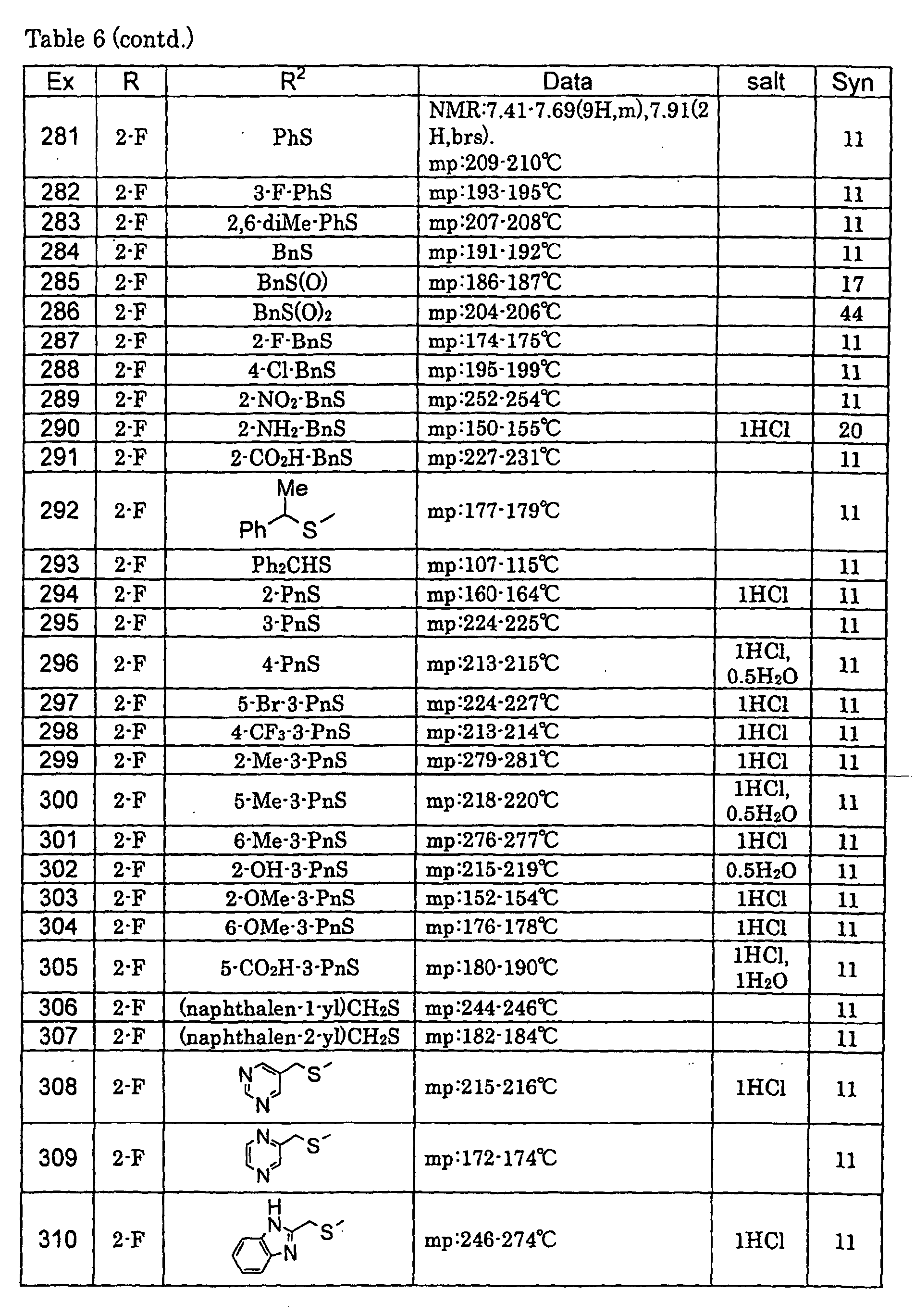

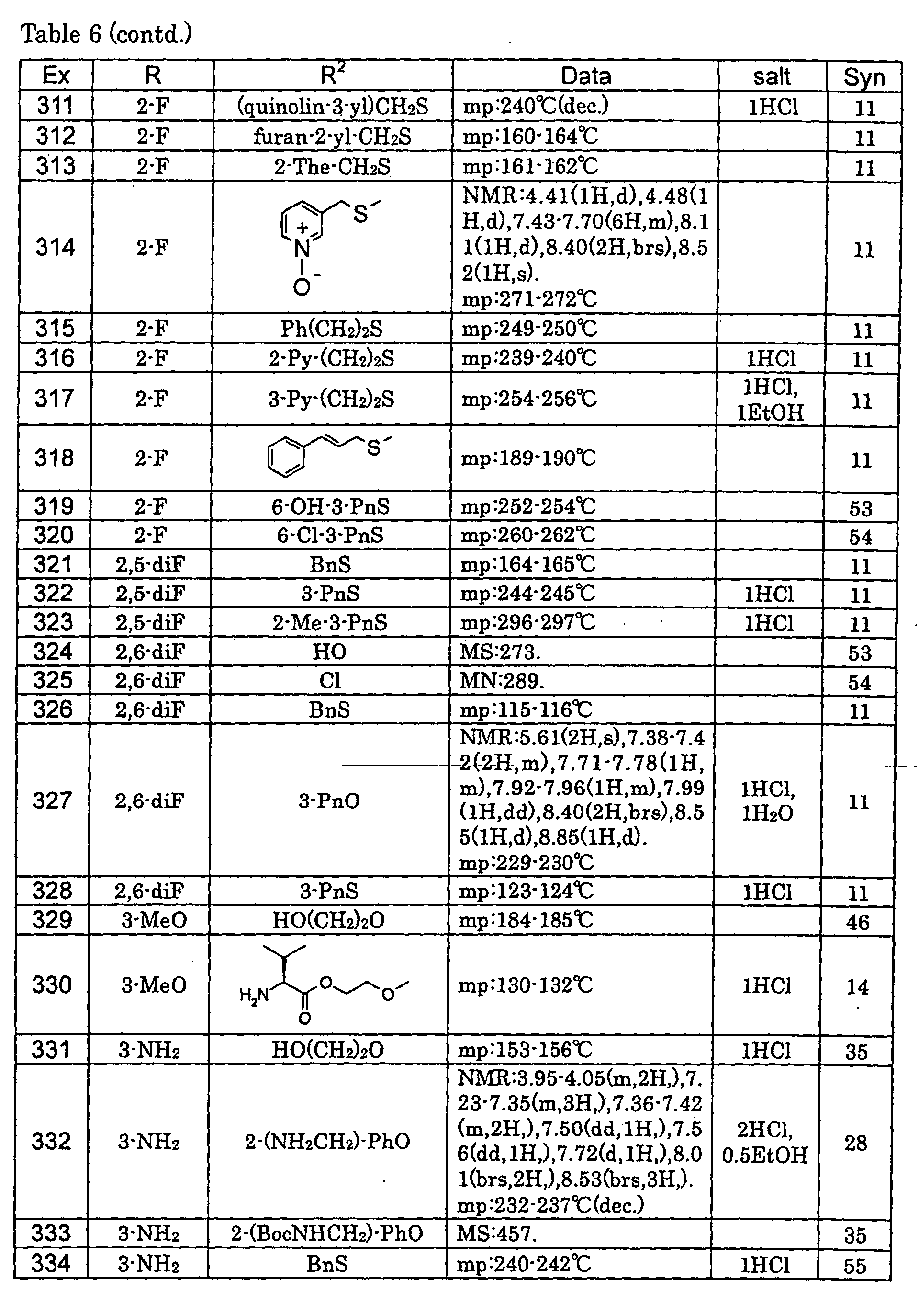

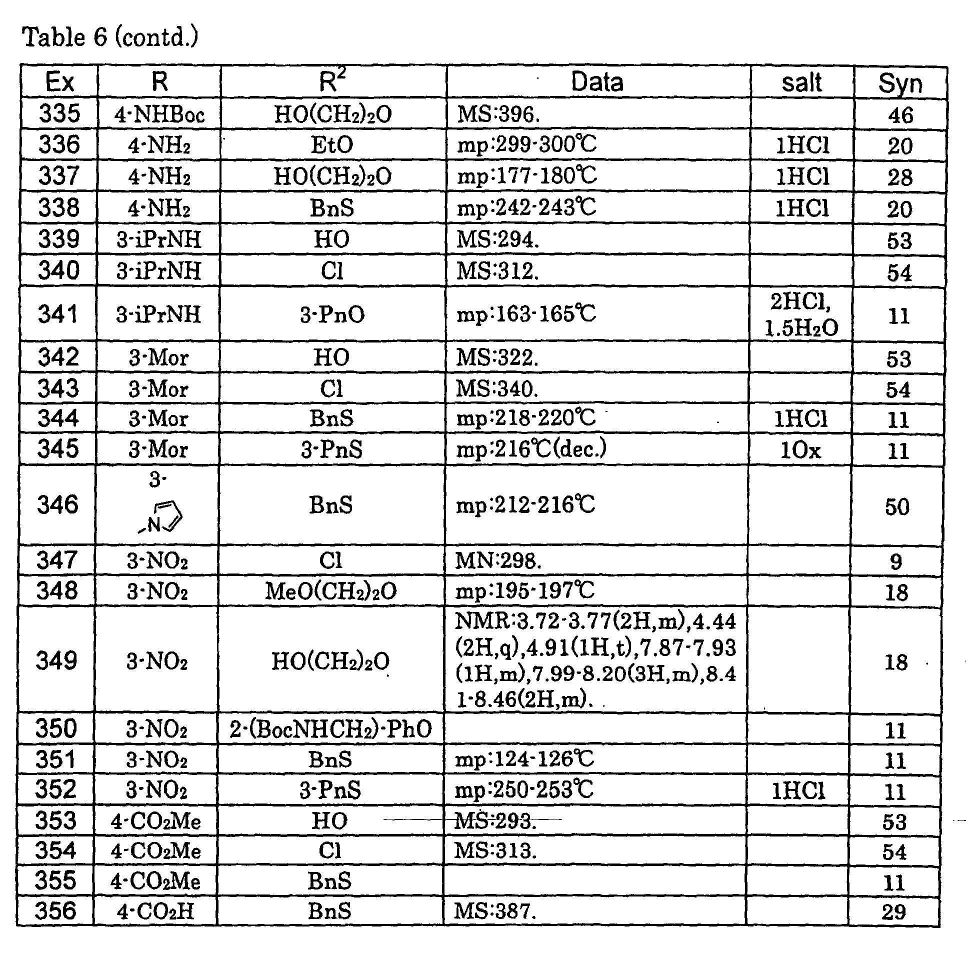

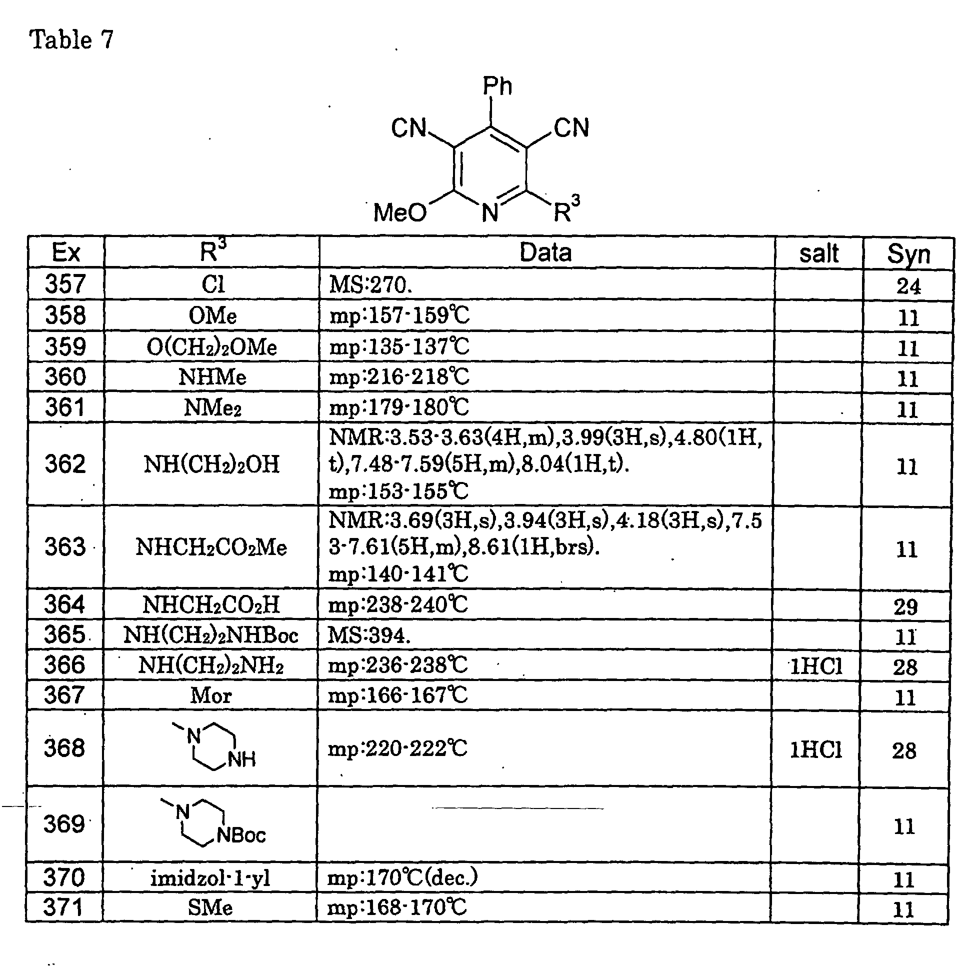

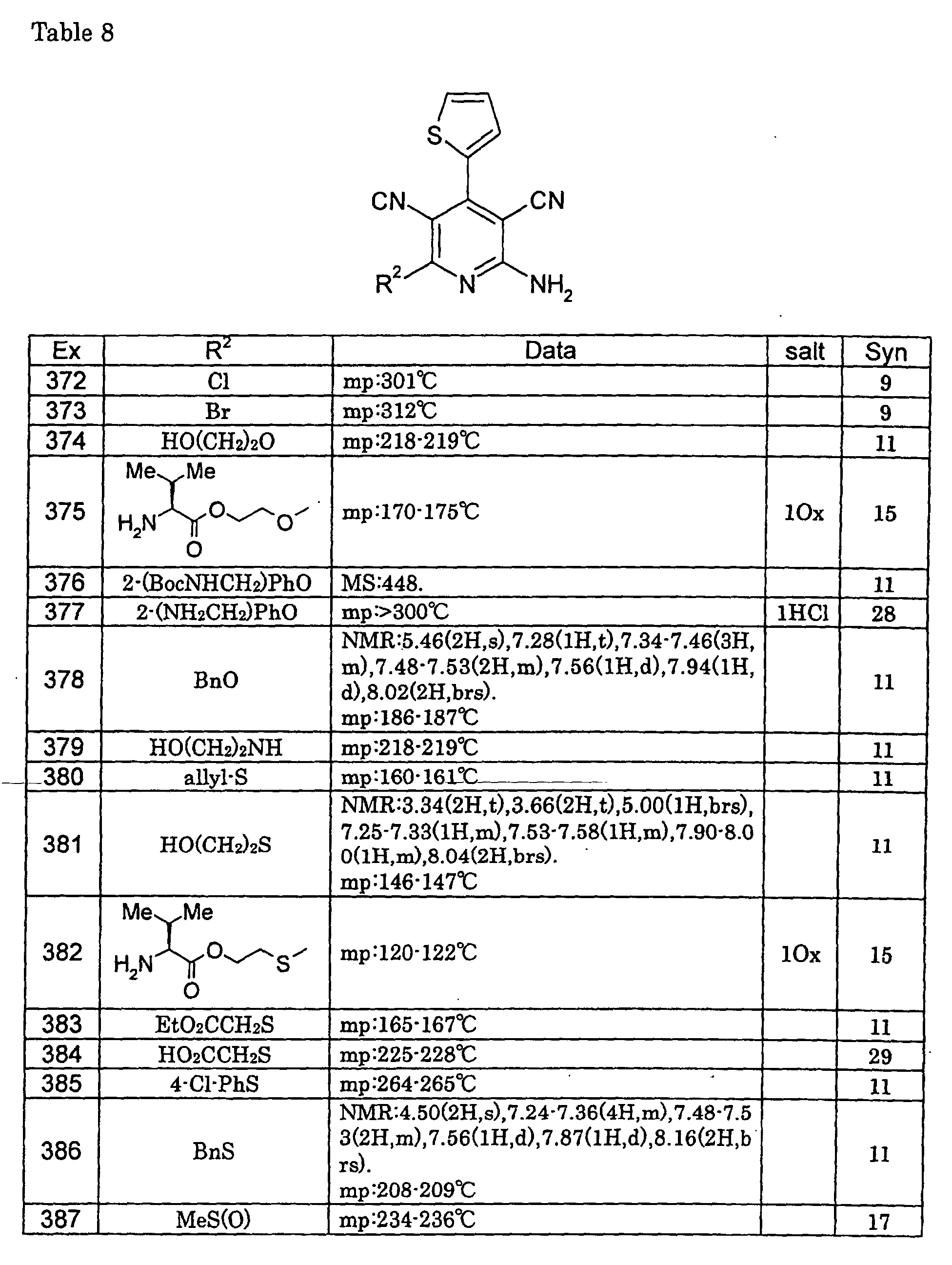

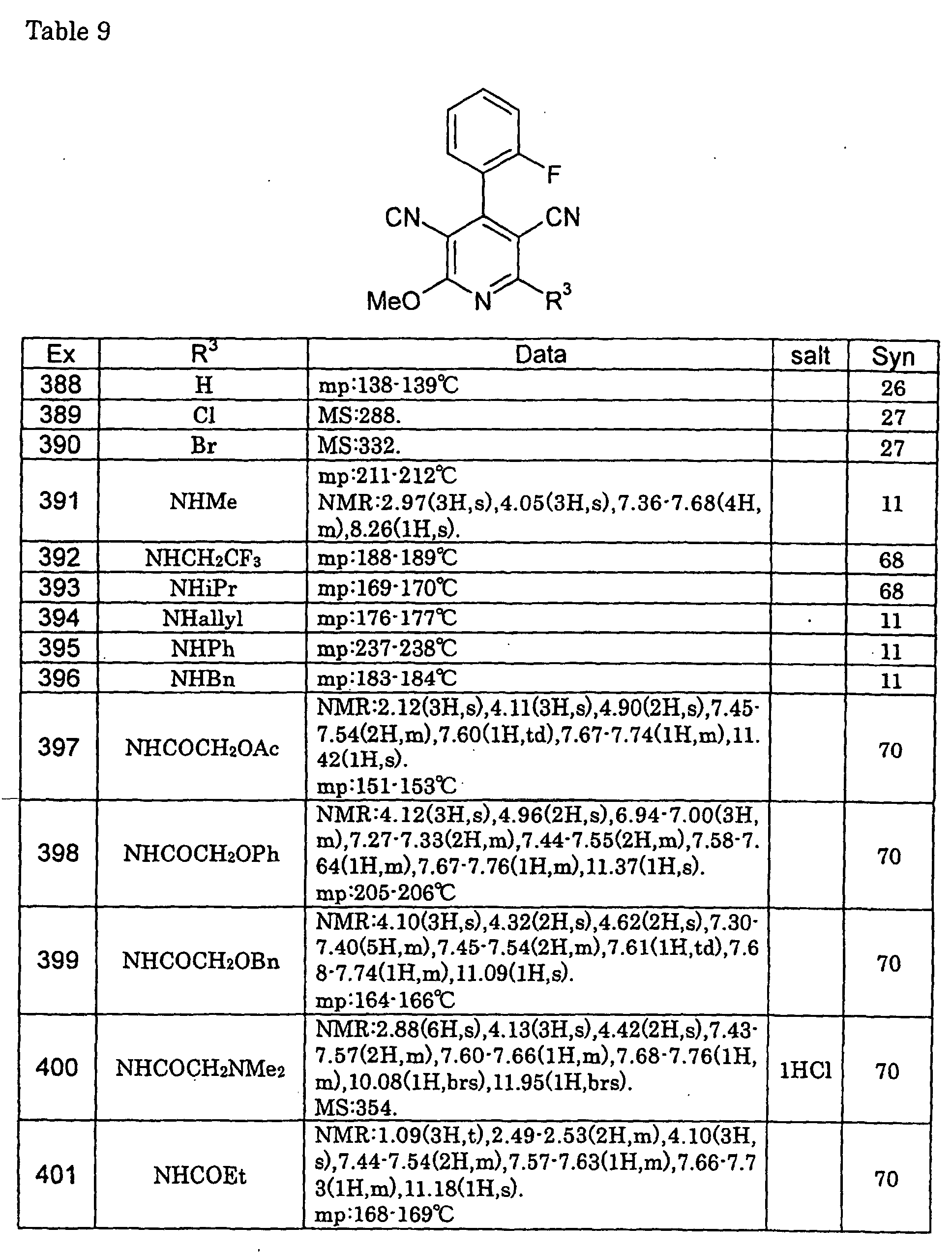

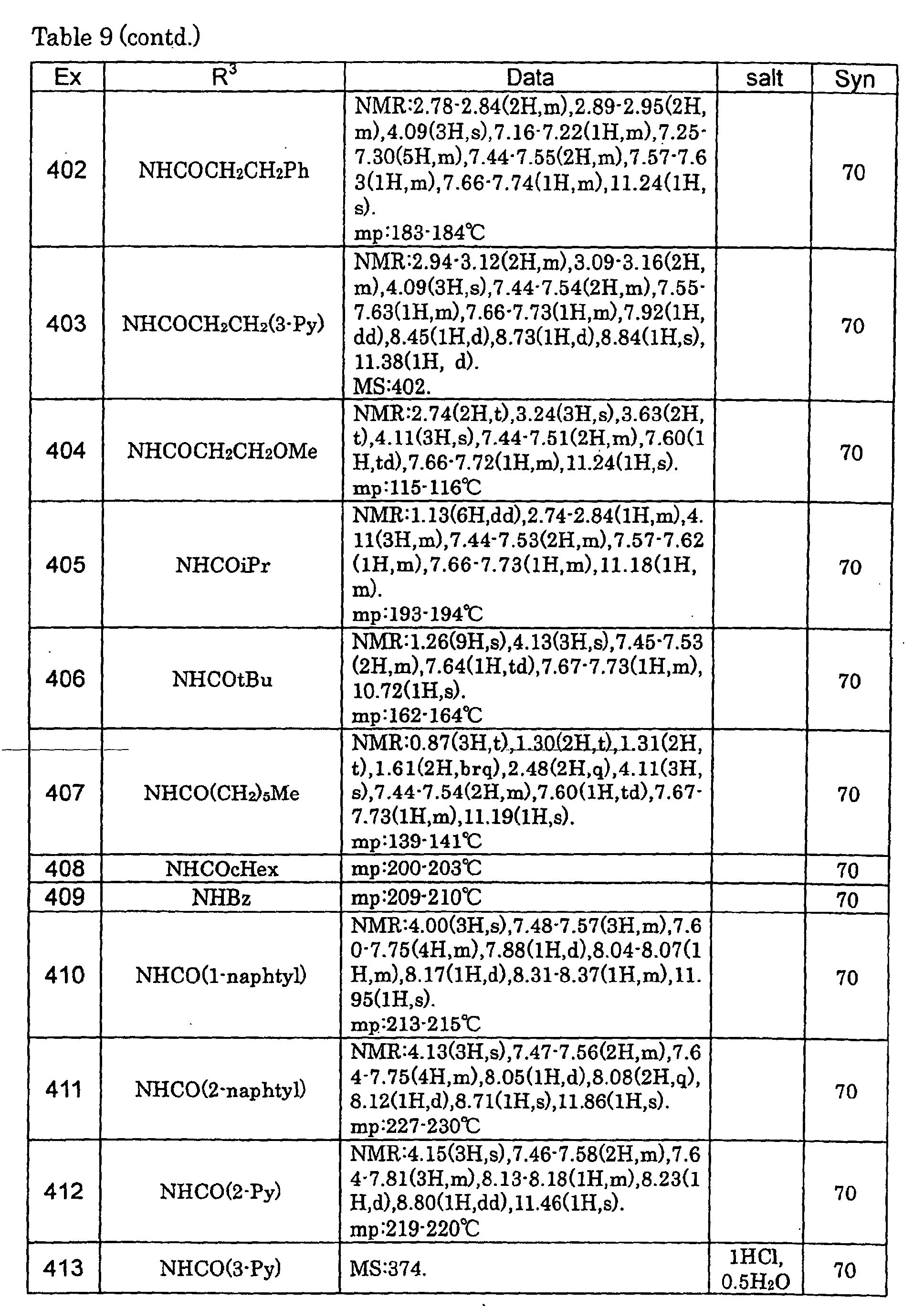

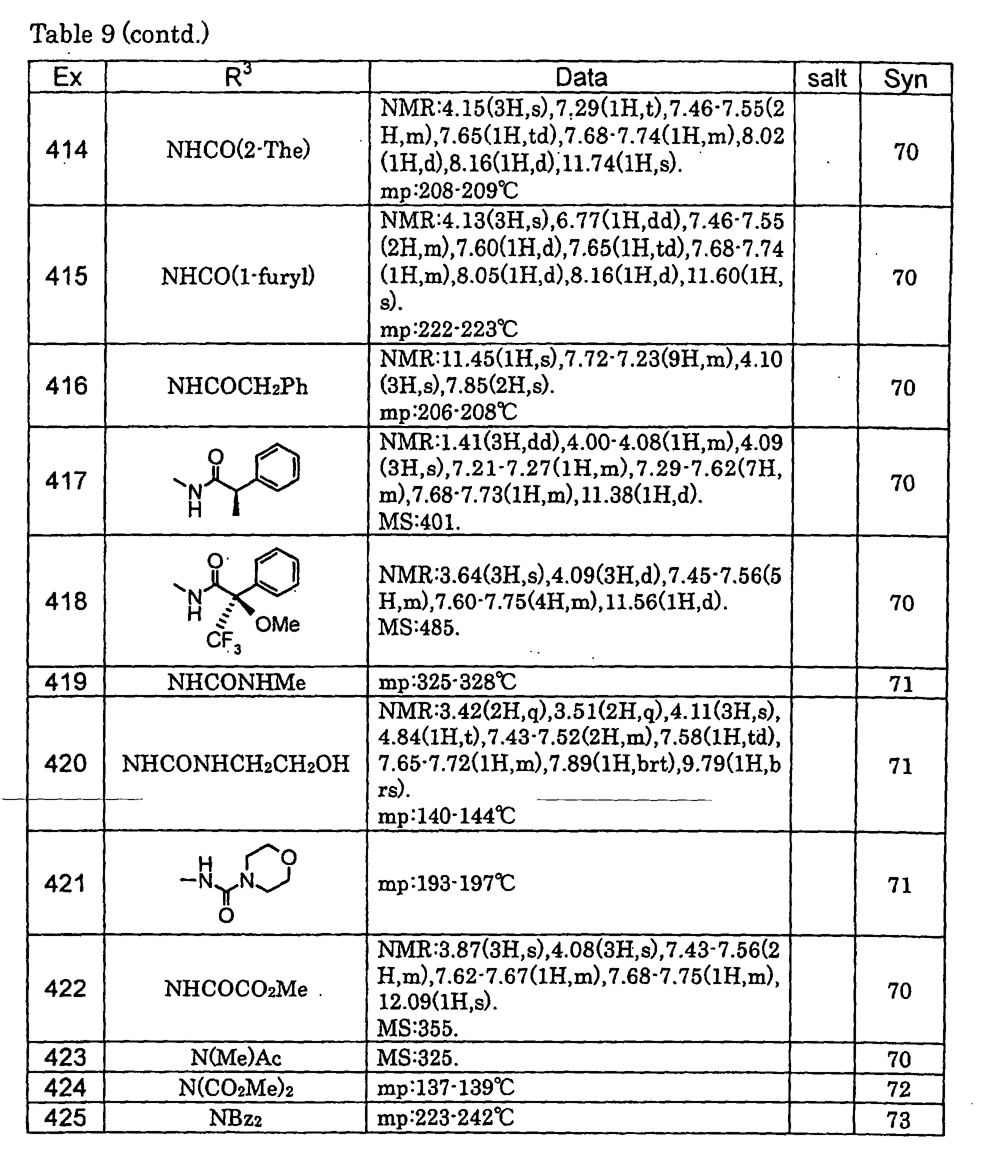

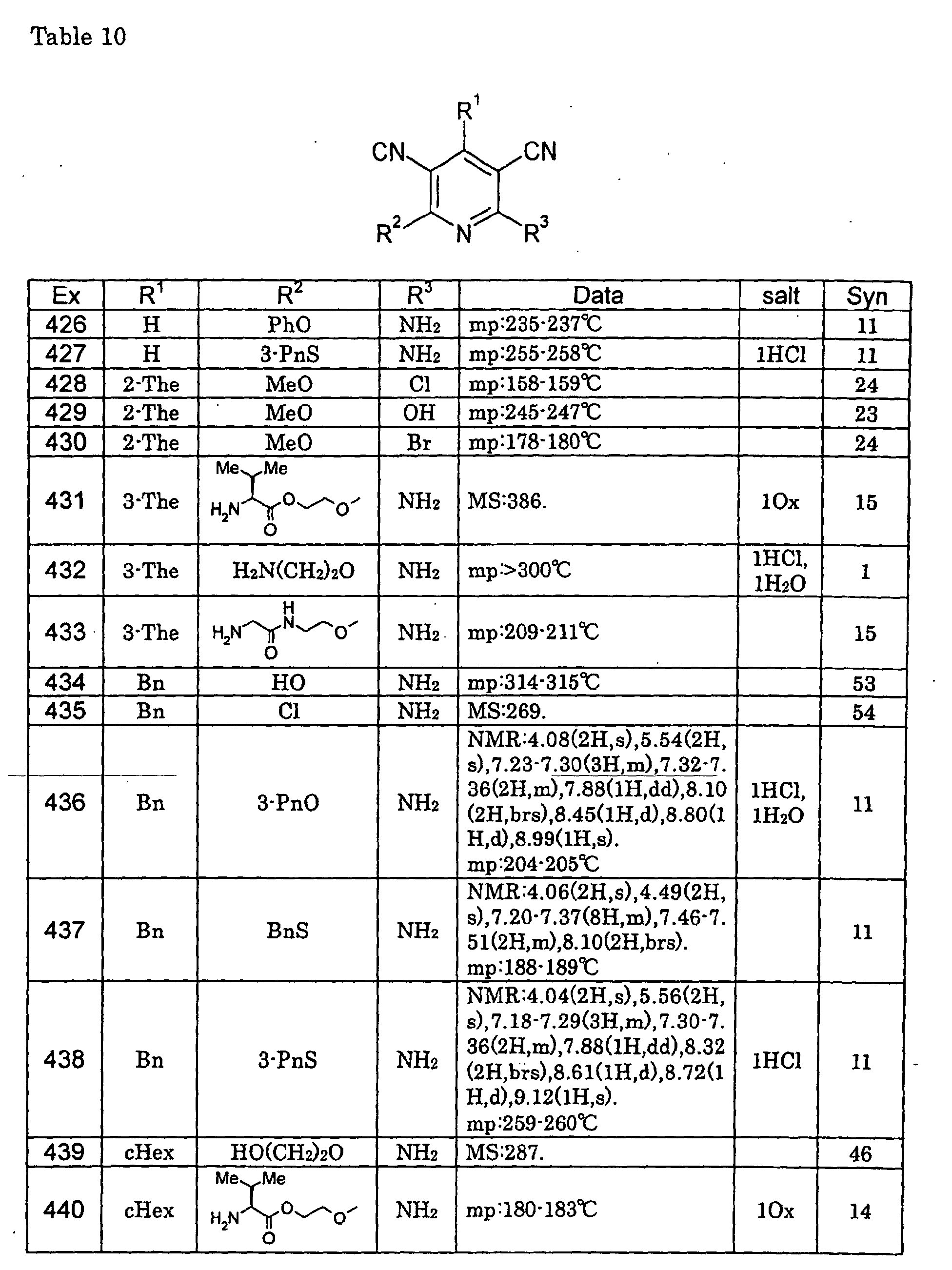

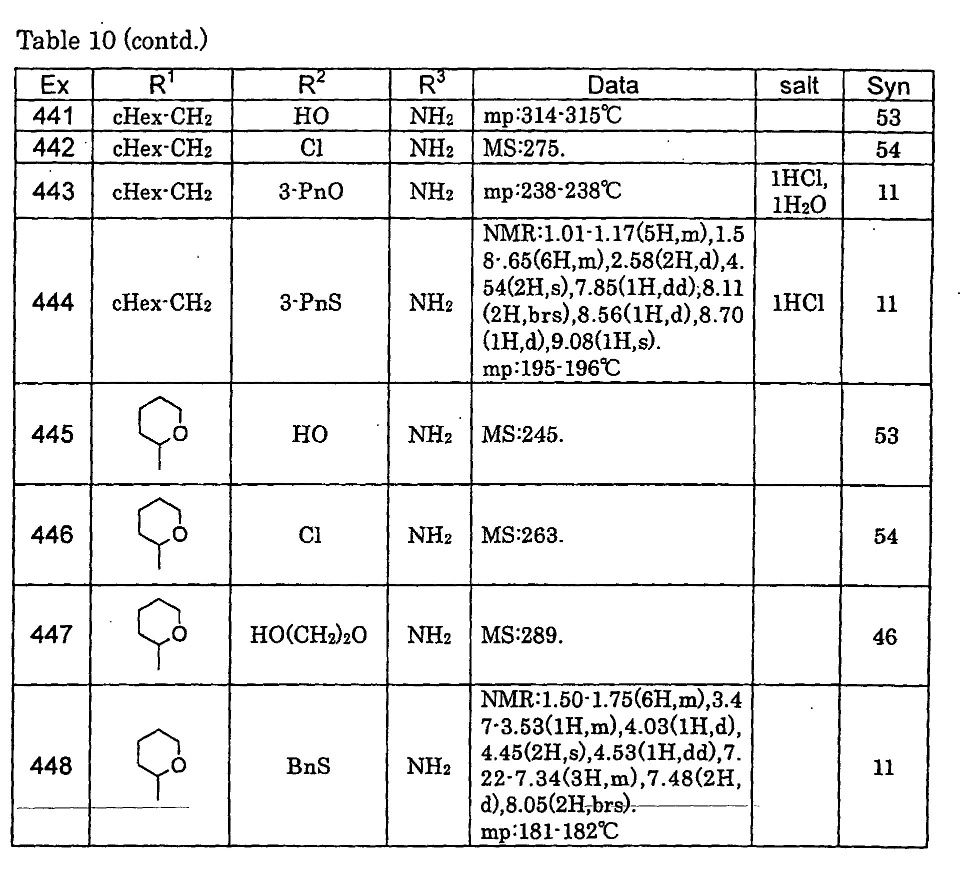

## Claims

1. A high conductance-type of calcium-activated K channel (maxi-K channel) opening agent, comprising any one of 3,5-dicyanopyridine derivatives of the general formula (I) or pharmaceutically acceptable salts thereof as an effective component: 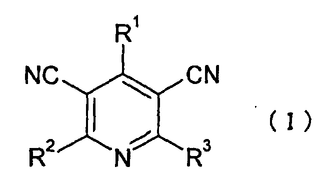 wherein
R¹ represents H, an optionally substituted lower alkyl, optionally substituted cycloalkyl, optionally substituted aryl, optionally substituted heteroaryl, or optionally substituted 5- or 6-membered saturated heterocycle;
R² and R³ are the same or different, each representing -O-R⁴, -S(O)ₙ-R⁴, -N(-R⁴)-R⁵, -NHCO-R⁵, -NHS(O)ₙ-R⁵, -NHCON(-R⁴)-R⁵, -N(CO-R⁵)₂, halogen atom or optionally substituted heteroaryl;
R⁴ represents H, an optionally substituted lower alkyl, optionally substituted lower alkenyl, optionally substituted alkynyl, optionally substituted aryl, optionally substituted heteroaryl, or optionally substituted 5- or 6-membered saturated heterocycle;
R⁵ represents H, an optionally substituted lower alkyl, cycloalkyl, -lower alkyl-O-lower alkyl, -lower alkyl-O-aryl, -lower alkyl-aryl, optionally substituted aryl, or optionally substituted heteroaryl;
or alternatively R⁴ and R⁵ taken with the adjacent N atom may form a 5- or 6-membered saturated heterocycle or a heteroaryl; and
n represents 0, 1 or 2.

2. A smooth muscle relaxant for bladder comprising any one of the compounds of the general formula (I) as claimed in Claim 1 or pharmaceutically acceptable salts thereof as an effective component.

3. An agent for treating pollakiuria and urinary incontinence comprising any one of the compounds of the general formula (I) as claimed in Claim 1 or pharmaceutically acceptable salts thereof as an effective component.

4. A 3,5-dicyanopyridine derivative of the general formula (II) or pharmaceutically acceptable salt thereof 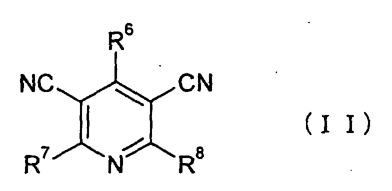 wherein
R⁶ represents phenyl, 2-fluorophenyl, 2,5-difluorophenyl, 2,6-difluorophenyl, 4-aminophenyl, 2,3-dihydro-1H-indol-6-yl, quinolin-7-yl, 3,4,5,6-tetrahydro-2H-pyran-2-yl, cyclohexylmethyl, benzyl, thiophen-2-yl or thiophen-3-yl.
R⁷ and R⁸ are the same or different, each representing -O-R⁹, -S(O)ₘ-R⁹, -N(-R⁹)-R¹⁰, -NHCO-R¹⁰, -NHS(O)ₘ-R¹⁰, -NHCON(-R⁹)-R¹⁰, -N(CO-R¹⁰)₂, halogen atom or optionally substituted heteroaryl;
R⁹ represents H, an optionally substituted lower alkyl, optionally substituted lower alkenyl, optionally substituted alkynyl, optionally substituted aryl, optionally substituted heteroaryl, or optionally substituted 5- or 6-membered saturated heterocycle;
R¹⁰ represents H, an optionally substituted lower alkyl, cycloalkyl, -lower alkyl-O-lower alkyl, -lower alkyl-O-aryl, -lower alkyl-aryl, optionally substituted aryl, or optionally substituted heteroaryl;
or alternatively R⁹ and R¹⁰ taken with the adjacent N atom may form a 5-or 6-membered saturated heterocycle or a heteroaryl; and
m represents 0, 1 or 2;
provided that
when R⁶ is phenyl, then
R⁷ is methoxy, 2-(2-amino-3-phenylpropionyloxy)ethoxy, 2-hydroxyethoxy, 2-aminomethylphenoxy or pyridin-3-ylmethyloxy; when R⁶ is phenyl and R⁷ is methoxy, then R⁸ is 2-hydroxyethylamino or methoxycarbonylmethylamino;
when R⁶ is phenyl, 2-fluorophenyl, 2,5-difluorophenyl, 2,6-difluorophenyl or 4-aminophenyl, R⁷ is -S-R⁹, and R⁹ is not N-oxidopyridinylmethyl, then
R⁸ excludes NH₂;
when R⁶ is benzyl, then
2-amino-4-benzyl-6-ethoxypyridine-3,5-dicarbonitrile is excluded;
when R⁶ is thiophen-2-yl, then
R⁷ is methoxy or 2-hydroxyethylsulfanyl; and
when R⁶ is thiophen-3-yl, then
2-amino-6-sulfanyl-4-(thiophen-2-yl)pyridine-3,5-dicarbonitrile is excluded.

5. A compound as claimed in Claim 4 or pharmaceutically acceptable salt thereof selected from:
2-amino-4-(2-fluorophenyl)-6-methoxypyridine-3,5-dicarbonitrile;
2-amino-6-methoxy-4-(tetrahydro-2H-pyran-2-yl)pyridine-3,5-dicarbonitrile;
2-[(6-amino-3,5-dicyano-4-phenylpyridin-2-yl)oxy]ethyl (S)-2-amino-3-phenylpropanoate;
2-amino-6-(2,2-difluoroethoxy)-4-(2-fluorophenyl)pyridine-3,5-dicarbonitrile;
2-amino-4-(2-fluorophenyl)-6-(prop-2-yn-1-yloxy)pyridine-3,5-dicarbonitrile;
N-[3,5-dicyano-4-(2-fluorophenyl)-6-methoxypyridin-2-yl]acetamide;
2-amino-4-(2,3-dihydro-1H-indol-6-yl)-6-methoxypyridine-3,5-dicarbonitrile;
N-[3,5-dicyano-4-(2-fluorophenyl)-6-methoxypyridin-2-yl]-2-methoxyacetamide;
N-[3,5-dicyano-4-(2,6-difluorophenyl)-6-methoxypyridin-2-yl]-2-methoxyacetamide;
N-[3,5-dicyano-4-(2,6-difluorophenyl)-6-methoxypyridin-2-yl]acetamide;
N-[3,5-dicyano-6-methoxy-4-(tetrahydropyran-2-yl)pyridin-2-yl]-2-methoxyacetamide; and
N-[3,5-dicyano-6-(2,2-difluoroethoxy)-4-(2-fluorophenyl)pyridin-2-yl]-2-methoxyacetamide; or
pharmaceutically acceptable salts thereof.

6. A pharmaceutical composition comprising as an effective component any one of the compounds as claimed in Claim 4 or 5 or pharmaceutically acceptable salts thereof.

7. A high conductance-type of calcium-activated K channel (maxi-K channel) opening agent, comprising as an effective component any one of the compounds as claimed in Claim 4 or 5 and pharmaceutically acceptable salts thereof.

8. A smooth muscle relaxant for bladder comprising as an effective component any one of the compounds as claimed in Claim 4 or 5 and pharmaceutically acceptable salts thereof.

9. A agent for treating pollakiuria and urinary incontinence, comprising as an effective component any one of the compounds as claimed in Claim 4 or 5 and pharmaceutically acceptable salts thereof.
